# EUROPEAN PATENT APPLICATION

(11) **EP 2 003 212 A1**
(43) Date of publication of application: **17.12.2008**
(21) Application number: 07105280.7
(22) Date of filing: 29.03.2007
(51) Int. Cl.: C12Q 1/68

(54) **In vitro method for diagnosing pathological conditions of male infertility**

(71) Applicant: Universitätsklinikum Hamburg-Eppendorf, 20246 Hamburg (DE)
(72) Inventor: Feig, Caroline, 20259, Hamburg (DE); Schulze, Wolfgang, 20249, Hamburg (DE); Kirchhoff, Christiane, 24582, Bordesholm (DE); Spiess, Andrej-Nicolai, 21271, Hamstedt (DE)
(74) Representative: UEXKÜLL & STOLBERG

(57) **Abstract**

The invention relates to an in vitro method for diagnosing and/or characterizing a pathological condition associated with male infertility. The invention also relates to an in vitro method for evaluating the prospect of success of a testicular sperm extraction procedure. In a further aspect, the invention provides a microarray for diagnosing and/or characterizing a pathological condition associated with male infertility and/or for evaluating the prospect of success of a testicular sperm extraction procedure. Kits for conducting the methods of the invention are provided. Finally, a method of screening an active agent for treating a pathological condition associated with male infertility is also provided.

SEQUENZPROTOKOLL

<110>  Universitätsklinikum Hamburg Eppendorf

<120>  In vitro method for diagnosing male infertility

<130>  P72135

<160>  92

<170>  PatentIn version 3.4

<210>  1
<211>  4456
<212>  DNA
<213>  Homo sapiens

<223>  tripartite motif-containing 36 (TRIM36), transcript variant 1, mRNA

<400>  1
gatggggctt ttttccagat cttcgcgccg agagccgggg acggccaacc tgccggcatg      60

cgataaaggc cggggaatgt ctagctgggg ccgcgctgag ccgagtgcgg acccgctttg     120

tgtgcgcgtt tgtttgtctc cgctgcggtc cgaaccgctg ccgcgacccc cgaggaggcg     180

gggcctccgc gcgcccggct ccgcctggtg agggtttcac ggcctctgct gccgccgccg     240

ccgccctcgt ttgttccgtt agatcgcgca gccccgaccg ctgcacccgg atcctagcaa     300

gccgggcgag gctgcccggg agccctcgat ggccttcatt tcacccaagc ccgcttcttg     360

ctttccccgg cgcttcccct cttttcctgg ttaacagctt atgggcgggg agctcggcaa     420

aactcagact aaaaacagaa aaagagaaaa gaaaggacaa attcgataca cccgcgtcgg     480

tcctccagag tttgtgaagg ggtgtaaaca tgtcggagtc tggggagatg agtgaatttg     540

gctacatcat ggaattgata gctaaaggca aggttaccat taagaatatc gaaagggagc     600

tcatttgccc agcatgcaag gagctgttta cccacccatt gattctccct tgccaacata     660

gtatctgtca taaatgtgta aaagaactcc tgctgactct cgatgattca ttcaacgatg     720

tgggatcaga caactccaat caaagcagtc ctcgacttcg gctcccctcc cctagtatgg     780

ataaaattga ccgaattaac agaccaggct ggaagcgcaa ttcattgacc ccgaggacaa     840

ctgttttccc ttgccctggc tgtgagcatg atgtggatct tggagaacga ggaatcaatg     900

gtctgtttcg aaacttcact ttggaaacta ttgtggaaag atatcgtcaa gcagctaggg     960

cagccacagc cattatgtgt gacctttgta aaccaccacc tcaagaatcc acaaaaagct    1020

gcatggactg tagtgcaagt tactgcaatg aatgcttcaa aattcatcac ccttggggta 1080

ctataaaagc tcaacatgag tatgttggtc caactactaa cttcagaccc aagattttaa 1140

tgtgcccaga acatgaaaca gagagaataa acatgtactg tgaattatgt aggaggccag 1200

tttgccatct gtgtaagttg ggtggtaatc atgccaacca ccgtgtaacc actatgagca 1260

gtgcctacaa aaccttaaag gaaaagcttt caaaggatat tgattacctt attggtaagg 1320

aaagccaggt gaagagtcaa atatctgaac taaacttgtt aatgaaagaa acagagtgta 1380

atggagagag ggctaaagaa gaagcaatta cacattttga aaagctcttt gaagttctgg 1440

aagagaggaa atcatctgtt ttgaaagcaa ttgactcctc taagaaacta agattagaca 1500

aatttcagac tcaaatggaa gagtaccagg gacttctaga gaacaatgga cttgtgggat 1560

atgctcaaga agtgctaaag gagacagatc agtcttgctt tgtgcagaca gcaaagcagc 1620

tccacctcag aatacagaaa gccacagaat ctttgaagag ctttagacct gcagctcaga 1680

cttcttttga agactatgtt gttaatacct ctaaacaaac agaacttctt ggagaattat 1740

cctttttctc tagtggcata gacgtgccag agatcaatga ggaacagagc aaagtttata 1800

acaatgcctt gataaattgg caccatccag aaaaggataa agctgatagc tatgttcttg 1860

aatatcggaa aatcaataga gatgatgaaa tgtcatggaa tgagatagaa gtgtgtggaa 1920

caagtaaaat aattcaagac ttggaaaaca gtagtaccta tgctttcaga gtaagagctt 1980

acaagggttc aatctgtagt ccttgcagca gagaattgat tcttcatact cctccagctc 2040

cagttttcag cttcctcttt gatgaaaaat gtggctataa taatgaacac ctcctgctga 2100

acttgaagag agaccgtgta gagagtagag ctggatttaa tcttctgctt gctgcagaac 2160

gcatccaagt gggttattac acaagcttag actacatcat tggagatact ggcattacaa 2220

aaggaaaaca cttctgggcc ttccgtgtgg aaccatattc atacctggta aaagtgggag 2280

ttgcttctag cgataaacta caagaatggc tccgttctcc ccgggatgca gttagtccaa 2340

gatatgagca agacagtggg catgacagtg gaagtgagga tgcctgtttt gattcttcac 2400

aaccatttac cttagttact ataggcatgc agaaattttt tatacccaag tcacctactt 2460

cttctaatga acctgaaaat agagttctcc ctatgccaac aagtattggg attttccttg 2520

actgtgataa aggcaaagta gatttctatg atatggatca gatgaaatgc ctttatgaac 2580

gccaagtgga ctgttcacat acactgtatc cagcatttgc attaatgggc agtggaggaa 2640

ttcagcttga agaacccatc acagcaaaat atctggaata ccaagaggac atgtagttga 2700

aacatctgat gtgactcagg ataaaaactg taaacaaagc aatgccttag cgttaacctt 2760

cgtaactaat tacatatcgc tgagtgttct gtcaccccac ttgtgtttga gtatgattct 2820

tcttgaaata cagaaaatct aaacagcctt gtgtcttcat gtcgttgttc tgcctttcac 2880

aaatgatgaa agtgaagatg ttctttccaa gttaatactg aagtggttaa ctttgcaact 2940

ggactgtctt ttactttatg tacaggatat ctaatttatt tctgtcagct ggtgttgctg 3000

acactttcct tatggtgggg gggatacaaa agtatttatt atgattctct tctgtttgac 3060

ttttcatttt tatatcatgt ttatgtccta agagaagata ttttttaaat gccttatgga 3120

taggaattag tactaagcaa tgtgtcttgc atttgttttc aataggaaaa caaagttcta 3180

gataggttaa aagaaaaaca ccataaaagt ggtagtatat ttaagtggaa aattatgtat 3240

tttaaagtgt gtaactatta aaaatatatt gcctggaaaa ggaatactac tgttttgttt 3300

tgttaagacc acaaactgta ttgctaagga cattttaaga gtctgcttag ccataacact 3360

catttaacag catgggtagt aagcatggtt tcttgcagaa aacacaaagt tctcagctaa 3420

cacattaaac ttctcacttt atatactttt tttcagagaa atttatcttt agtttttctt 3480

attctgtgaa aatatttgtt cttatgtcaa agaattattt ttcagatata ttggtaaaga 3540

tggactgata ttaaaagtca tatttattct tttaccccct tactgtgttt tgtgattttc 3600

atttttggcc caattattac atatcaggtt ttcctgattt atatttacat gtttgaaaag 3660

ttcattatat attttagtag cgttacatat attataaatt tttgtaacat ttaaatgttc 3720

acttatgtgg cattttctgt ttctcattgg taataagcat ttctaatata agcaagtaaa 3780

aatacaaatt taattactta gaaatgataa ctaagatcac aatgactgtg taccctttaa 3840

aatatctttt aatttgagga attacattct atctaaattg tgtgtctgtt tttgaaaggt 3900

gttgctttca agtaggtggt ttttatttaa agtacttatt tttttgaggt taaaatattc 3960

agaaactact tttatgcatt gtagcagtaa tcatttatgg aatgcttttg acatgtttta 4020

acagggtaac caaaactttt aggcctgttt tttttggttg tccttgttgt tatattttta 4080

atggaaatct gttactcagg ctggtagcat taaattagct ttttaccctc aactcaagta 4140

gcaaatatca tggagaactt cagtgtttag caggctgtta atttcccatc tatgaatagc 4200

actttatgat gtaggctaaa ctaagcaaat aatgcagcgt tatgtggatc attatgcaat 4260

aaagctgcta aagtcacatt tgtggttgat actcaagcct tgtacaagca catatgatgt 4320

agacattttt aaatgtgaag ctttaacagg aataggatag ctgtttcaaa ccttgtgcaa 4380

ttgtaaataa attgtttagc tgtgtttctt tcatttttaa taaaaaatta aaaagaaaaa 4440

aaaaaaaaaa aaaaaa 4456

<210> 2
<211> 2387
<212> DNA
<213> Homo sapiens

<223> chromosome 6 open reading frame 204 (C6orf204), transcript variant
 2, mRNA

<400> 2
   agtatcgcaa aataggccaa gtcgctgccc tctactcaac tcgcagggcg ggaggatgga 60
   gacagaggga tgcggcggga gggcgggaca gcccagtcgc cgccaaggag gcgaagcggg 120
   gtcggggtag ctcacccgcc ccccgcagtc cccgcccccc gcagtccccg ccccgcgcgg 180
   cgccgcgcac gcctgaagtc attgcccgag gccccgcccc ctcgcggccc cagccccggc 240
   tctggctctg ccggcggagg gggcggagag aacccgcagc tgcaacttcg cctggagcct 300
   ttgagcctgg gctcagccgg ggatctagca gccgtcaccg ccgagtcgcc gcttccccgg 360
   cgcgggcctc agcccagccg gcttggccat ggcaggcgcc ggcagtgcgg cgaatctggc 420
   ccctgcgctc cccgcccgta gctgagcagc cgttaaccag ccatcccgcc tttcctcctg 480
   cccgtgtcgc cgaggcccac gctccgcgca agtttccgcc gccgaagaag caggaggacg 540
   gacgtgagga gtcgggcgtc cctggcccac ccggccctct cgcccgcgat gtgggggcgc 600
   ttcctggctc cggaggccag cggccgggat agccccggcg gagcccgcag cttccctgcc 660
   ggcccagatt attcatcagc atggctacct gctaatgaat cattgtggca ggccacaact 720
   gttccatcta accatcgaaa taaccatatc aggagacaca gtatagcttc tgacagtgga 780
   gacactggca ttggaacttc ctgttctgat agcgtggaag atcattcaac ttcaagtggc 840
   acattatctt ttaagcctag tcaatcattg attactcttc ctactgccca tgtgatgccg 900
   tctaattcca gcgcttcaat ttccaaactt agggaatcat tgacaccaga tggctcaaaa 960
   tggagtacaa gcctcatgca aacattggga aaccacagta gggggagcag ggactcttcc 1020
   ctagacatga aggacttccg gccacttcgg aaatggtcat ctttatccaa actcactgcc 1080
   ccggataact gtggccaggg tggcactgta tgcagagaag agtcaaggaa tggtttggag 1140
   aagataggga aggctaaggc tttaacatca caactgagga caattgggcc tagctgttta 1200
   catgatagta tggagatgct taggttagag gacaaggaaa taaataaaaa acggtcatca 1260
   actctggact gcaagtataa atttgagagc tgtagcaagg aggactttag agcctcttcc 1320
   tctactctta ggagacagcc tgtagacatg acatatagtg ccttacctga aagcaagccc 1380
   attatgacaa gctcagaggc ttttgaacct ccaaaatatt taatgcttgg tcaacaggca 1440
   gtaggtggag ttcccattca gccttccgta aggactcaga tgtggcttac agagcagctg 1500
   cggacaaatc ctttggaagg tagaaataca gaggattctt acagtttagc tccttggcaa 1560
   cagcagcaaa ttgaagactt tcgacaagga agtgaaacac caatgcaggt tttgactgga 1620
   tcatctcgtc aaagttattc acctggctat caggatttca gtaagtggga atcaatgttg 1680
   aaaataaaag aaggacttct aaggcagaaa gaaattgtaa tcgatcggca gaagcaacaa 1740
   attacccacc tgcatgagag gataagggat aatgaattac gggctcaaca tgccatgtta 1800
   ggacattatg taaattgtga ggattcttat gtggctagtt tgcagccaca atatgagaac 1860
   acttcactcc agacaccatt ttcagaggaa tcagtttccc attcccaaca aggggaattt 1920
   gagcaaaagc ttgcatctac tgagaaagaa gttttacagc ttaatgagtt tctcaaacaa 1980
   agactaagcc tatttagtga agagaagaag aaactagagg aaaaagtagg tttttctaat 2040
   aaagttgaat taggtcaaca acatttcctc agcatataat tatttgtctg tcatttaccc 2100
   tgtcaggtgt catgccaggt tatgtataca tttatgtgga ggtgattaat tttgtagtaa 2160
   cttttgagaa gccctctgct gtctgcagaa atccaaggat tttctttgag gatagaaatg 2220
   ctcttgtata tctttctaac aaatagtaaa atctttaaaa aattaattgc ctacaccaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaa 2387

<210> 3
<211> 1892
<212> DNA
<213> Homo sapiens

<223> melanoma antigen family A, 11 (MAGEA11), transcript variant 1, mRNA

<400> 3
gatctgagag aagcgaaagc gtctttctga ggggtgtctt gagagtggca gagggcagcg 60

ggtccaggct ccatgaggag gcaagccttg ggaatctgag ggatggagac tcagttccgc 120

agagggggtc tggggtgcag ccctgccagc atcaagagga agaagaagag ggaggactca 180

ggagactttg gactccaggt gagcactatg ttctcagagg acgacttcca gtcaacagaa 240

agagccccat atggtccaca actacagtgg tcccaggatc tgccaagagt ccaggttttt 300

agagaacagg ccaacctgga ggacaggagt cccaggagaa cccagaggat cactggagga 360

gaacaagtgc tgtggggccc catcacccag atatttccca cagttcggcc tgctgaccta 420

accagagtca tcatgcctct tgagcaaaga agtcagcact gcaagcctga ggaaggcctt 480

caggcccaag aagaagacct gggcctggtg ggtgcacagg ctctccaagc tgaggagcag 540

gaggctgcct tcttctcctc tactctgaat gtgggcactc tagaggagtt gcctgctgct 600

gagtcaccaa gtcctcccca gagtcctcag gaagagtcct tctctcccac tgccatggat 660

gccatctttg ggagcctatc tgatgagggc tctggcagcc aagaaaagga ggggccaagt 720

acctcgcctg acctgataga ccctgagtcc ttttcccaag atatactaca tgacaagata 780

attgatttgg ttcatttatt gctccgcaag tatcgagtca aggggctgat cacaaaggca 840

gaaatgctgg ggagtgtcat caaaaattat gaggactact ttcctgagat atttagggaa 900

gcctctgtat gcatgcaact gctctttggc attgatgtga aggaagtgga ccccactagc 960

cactcctatg tccttgtcac ctccctcaac ctctcttatg atggcataca gtgtaatgag 1020

cagagcatgc ccaagtctgg cctcctgata atagtcctgg gtgtaatctt catggagggg 1080

aactgcatcc ctgaagaggt tatgtgggaa gtcctgagca ttatgggggt gtatgctgga 1140

agggagcact tcctctttgg ggagcccaag aggctcctta cccaaaattg ggtgcaggaa 1200

aagtacctgg tgtaccggca ggtgcccggc actgatcctg catgctatga gttcctgtgg 1260

ggtccaaggg cccacgctga gaccagcaag atgaaagttc ttgagtacat agccaatgcc 1320

aatgggaggg atcccacttc ttacccatcc ctgtatgaag atgctttgag agaggaggga 1380

gagggagtct gagcatgaga tgcaaccagg gccagcgggc agggaaatgg gccaatgcat 1440

gcttcagggc cacacccagc agtttccctg tcctgtgtga aatcaggccc attcttccct 1500

ctgtgtttga tgagagaagt cagtgttctc agtagtagaa ggcacagtga atggaaggga 1560

acacattgta tactgccttt aggtttctct tccatcgggt gacttggaga tttctttttg 1620

tttccctttg gtaattttca aatattgttc ctgtaataaa agttttagtt agcttcaaca 1680

tctaagtgta tggatgatac tgaccacaca tgttgttttg cttatccatt tcaagtgcaa 1740

gtgtttgcca ttttgtaaaa cattttggga aatcttccat cttgctgtga tttgcaatag 1800

gtattttctt ggagaatgta agaacttaac aataaagctg aactggtgtt gtgaaacaga 1860

gaaataaaag gagaaggtca ttaaaaaaaa aa                                    1892

<210>  4
<211>  2339
<212>  DNA
<213>  Homo sapiens

<223>  solute carrier family 39 (zinc transporter), member 13 (SLC39A13),
       mRNA

<400>  4
gccgcccgga tcgccgccgc cgccgccgca cggtggcctt ttgtgttttt cagtacgtgg      60

catgcctgga tgtccctgcc ctggctgtgg catggcgggc ccaaggctcc tcttcctcac     120

tgcccttgcc ctggagctct tgggaagggc tgggggttcc cagccggccc tccggagccg     180

ggggactgcg acggcctgtc gcctggacaa caaggaaagc gagtcctggg gggctctgct     240

gagcggagag cggctggaca cctggatctg ctccctcctg ggttccctca tggtggggct     300

cagtggggtc ttcccgttgc ttgtcattcc cctagagatg gggaccatgc tgcgctcaga     360

agctggggcc tggcgcctga agcagctgct cagcttcgcc ctggggggac tcttgggcaa     420

tgtgtttctg catctgctgc ccgaagcctg ggcctacacg tgcagcgcca gccctggtgg     480

tgaggggcag agcctgcagc agcagcaaca gctggggctg tgggtcattg ctggcatcct     540

gaccttcctg gcgttggaga agatgttcct ggacagcaag gaggagggga ccagccaggc     600

ccccaacaaa gaccccactg ctgctgccgc cgcactcaat ggaggccact gtctggccca     660

gccggctgca gagcccggcc tcggtgccgt ggtccggagc atcaaagtca gcggctacct     720

caacctgctg gccaacacca tcgataactt cacccacggg ctggctgtgg ctgccagctt     780

ccttgtgagc aagaagatcg ggctcctgac aaccatggcc atcctcctgc atgagatccc     840

ccatgaggtg ggcgactttg ccatcctgct ccgggccggc tttgaccgat ggagcgcagc     900

caagctgcaa ctctcaacag cgctgggggg cctactgggc gctggcttcg ccatctgtac     960

ccagtccccc aagggagtag aggagacggc agcctgggtc ctgcccttca cctctggcgg    1020

ctttctctac atcgccttgg tgaacgtgct ccctgacctc ttggaagaag aggacccgtg    1080

gcgctccctg cagcagctgc ttctgctctg tgcgggcatc gtggtaatgg tgctgttctc    1140

gctcttcgtg gattaacttt ccctgatgcc gacgcccctg ccccctgcag caataagatg    1200

ctcggattca ctctgtggcc gcatatgtga gaggcagaga gggcgagtgg ctgcgagaga    1260

gaatgagcct cccgccagac aggagggagg tgcgtgtgga tgtatgtggt gtgcacatgt    1320

ggccagaggt gtgtgcgcga gaccgacact gtgatccctg tgctgggtcc ggggcccagt 1380

gtagcgcctg tccccagcca tgctgtggtt acctctcctt gccgccctgt caccttcacc 1440

tcctggagta agcagcgagg aagagcagca ctggtcccaa gcagaggcct tgccctgctg 1500

ggaccccggg agtgagagca gcccaaggat cccagggtgc agggaactcc agagctgccc 1560

acctcccact gccccctcag cacacacaca gtccccaggc ggcctagggg ccaaggctgg 1620

ggcggctttg gtcccttttc ctggcccttc cttccccact tctaagccaa agaaaggaga 1680

ggcaggtgct cctgtacccc agccccactc agcactgaca gtccccagct cctagtagtg 1740

agctgggagg cgcttcctaa gaccctttcc tcagggctgc cctgggagct cattcctggc 1800

caacacgccc tggcagcacc agcagctctt gccacctcca gctgccaaac agcagcctgc 1860

cgggcaggga gcagccccag gccagagagg cctcccggtc cagctcaggg atgctcctgc 1920

cagcacaggg gccagggact cctggagcag gcacatagtg agcccgggca gccctgccca 1980

gctcaggccc ctttccttcc ccattgaggt tggggtaggt gggggcggtg agggctccac 2040

gttgtcagcg ctcaggaatg tgctccggca gagtgctgaa gccataatcc ccaaccattt 2100

cccttggctg acgcccaggt actcagctgg cccactccac agccaggcct ggccctgccc 2160

ttcaccgtgg atgttttcag aagtggccat cgagaggtct ggatggtttt atagcaactt 2220

tgctgtgatt ccgtttgtat ctgtaaatat ttgttctata gataagatac aaataaatat 2280

tatccacata aaaaaaaaaa aaaaaaaaaa aaaaaaaaaa aaaaaaaaaa aaaaaaaaa 2339

<210> 5
<211> 2530
<212> DNA
<213> Homo sapiens

<223> transketolase-like 1 (TKTL1), mRNA

<400> 5
attcgctctt cagacgccgg agacgtagga gtgggtcttc agactccaaa ggggttggac 60

taatggcgga tgctgaggcg agggctgagt tcccggagga ggccagacct gacaggggca 120

ccttgcaggt gtttcaagat atggccagcc gcttgcgaat ccattccatc agggccacat 180

gctccacgag ctccggccac cctacatcat gtagcagttc ttctgagatc atgtctgtgc 240

tgttcttcta catcatgagg tacaagcagt cagatccaga gaatccggac aacgaccgat 300

ttgtcctcgc aaagagactg tcgtttgtgg atgtggcaac aggatggctc ggacaaggac 360

tgggagttgc atgtggaatg gcatatactg gcaagtactt cgacagggcc agctaccggg 420
tgttctgcct catgagtgat ggcgagtcct cagaaggctc tgtctgggag gcaatggcct 480
ttgcttccta ctacagtctg gacaatcttg tggcaacctt tgatgtgaac cgcctgggac 540
acagtggtgc attgcccgcc gagcactgca taaacatcta tcagaggcgc tgcgaagcct 600
ttgggtggaa cacttatgtg gtggacggcc gggacgtgga ggcactgtgc caggtattct 660
ggcaggcttc tcaggtgaag cacaagccca ctgctgtggt ggccaagacc ttcaagggcc 720
ggggcacccc aagtattgag gatgcagaaa gttggcatgc aaagccaatg ccgagagaaa 780
gagcagatgc cattatcaaa ttaattgaga gccagataca gaccagcagg aatcttgacc 840
cacagccccc cattgaggac tcacctgaag tcaacatcac agatgtaagg atgacctctc 900
cacctgatta cagagttggt gacaagatag ctactcggaa agcatgcggt ctggctctgg 960
ctaagctggg ctacgcgaac aacagagtcg ttgtgctgga tggtgacacc aggtactcta 1020
ctttctctga gatattcaac aaggagtacc ctgagcgctt catcgagtgc tttatggctg 1080
aacaaaacat ggtgagcgtg gctctgggct gtgcctcccg tggacggacc attgcttttg 1140
ctagcacctt tgctgccttt ctgactcgag catttgatca catccggata ggaggcctcg 1200
ctgagagcaa catcaacatt attggttccc actgtggggt atctgttggt gacgatggtg 1260
cttcccagat ggccctggag gatatagcca tgttccgaac cattcccaag tgcacgatct 1320
tctacccaac tgatgccgtc tccacggagc atgctgttgc tctggcagcc aatgccaagg 1380
ggatgtgctt cattcggacc acccgaccag aaactatggt tatttacacc ccacaagaac 1440
gctttgagat cggacaggcc aaggtcctcc gccactgtgt cagtgacaag gtcacagtta 1500
ttggagctgg aattactgtg tatgaagcct tagcagctgc tgatgagctt tcgaaacaag 1560
atatttttat ccgtgtcatc gacctgttta ccattaaacc tctggatgtc gccaccatcg 1620
tctccagtgc aaaagccaca gagggccgga tcattacagt ggaggatcac tacccgcaag 1680
gtggcatcgg ggaagctgtc tgcgcagccg tctccatgga tcctgacatt caggttcatt 1740
cgctggcagt gtcgggagtg ccccagagtg ggaagtccga ggaattgctg gatatgtatg 1800
gaattagtgc cagacatatc atagtggccg tgaaatgcat gttgctgaac taaaatagct 1860
gttagctttg gtcttttggc ctctttaccc tgtgtttatg tttgttccaa aaccatcatt 1920
taaatctcta ctgtcacatt ttgtttctta aaagcaaagc cagctaacac cttcattcat 1980
ccctagttcg gaaattcaag ctaactactt accctttaaa ctgtcactgc atatgcaagt 2040

accgctctaa tttttggatc attaagggga gttacacaac ttttaagtga aaaaaatagg 2100

taacaaaaca accacctgat agtaagtttt ctgataagac tatagataag tggtagaggt 2160

aatcaattct tccgaagtgt ttccttcgtg aataactggt agaggtaata gttttttcaa 2220

tgtatttcct tcatgagtaa agaaaatgtg gattgaagta tagattccag tagcctagtt 2280

tccacagcac gataacacca tgacgcctac tgctgttccc accttgggat tctgtgtgct 2340

gccatcccac ctgcagctgc cctggaattc ccttcgctgt ttgccttcat ctccctccac 2400

gtttgagagg ctgtcaggca gcagcgaaag cttgttagga tgtcctgtgc tgcttgtgat 2460

gagagcctcc acactgtact gttcaagtca atgttaataa agcatttcaa aaccaaaaaa 2520

aaaaaaaaaa 2530

<210> 6
<211> 3280
<212> DNA
<213> Homo sapiens

<223> SRY (sex determining region Y)-box 30 (SOX30), transcript variant 1,
      mRNA

<400> 6
gggggaggga tgactaaaga caacggctgt aagagaactc cacaagagag tcagaacgaa 60

aatgtgcaac aagtgcggcg gctcctacca tggcaggtga ttcgaggact cagcacgagg 120

cgagagtagg gaccaggaag agccggaaaa cccgcctgtg attggccgtc cacgggtatc 180

ggtcgttgtg attgggtgag gcccagacag acagctgcgt tttgaaccgc gtagggttct 240

gggtagcaaa ggccttgcaa ggctcttaac cgaaaggggg agggggaagg tcgccaacaa 300

acggctgagc tcacaatcct ggccggggcg tccccctccc ccatggagag agccagaccc 360

gagccgccgc ctcagccgcg cccgttgcgt cccgctccgc ccccgctgcc ggtcgagggc 420

acctcctttt gggcagcagc catggagccc cctccgtcgt ctcccacact gagcgcggca 480

gccagtgcga ccttggcctc gtcgtgcggg gaggcagtgg cgtccggctt acagcccgcg 540

gtgcggcggc tgctgcaggt gaagccagag caggtgttgc tgctaccaca gcctcaggcc 600

cagaacgagg aagccgctgc ctcgtccgcg caggcgcggc tgttgcagtt caggcccgac 660

ctgcggctcc tgcagccgcc gacagcgtca gacggcgcca cctccaggcc cgagttgcac 720

ccggtgcagc ccctggcgct gcatgtcaag gccaagaagc agaagctggg gcccagcctg 780

gatcagtcag tggggcctcg aggggccgtc gaaaccggtc ctagagcctc cagggtggtc 840

aagttggaag gccccgggcc ggccctcggc tacttccgag gggacgagaa gggcaagctg 900
gaggcggagg aggtcatgag agactcgatg caaggcgggg caggcaaaag cccggcagcc 960
atccgagaag gtgtgatcaa aacggaggaa cccgagagac tcctcgagga ctgcaggctc 1020
ggcgcggagc ccgcgtccaa tggcctggtt catggcagcg cggaggtcat cttggcccca 1080
acgtccggtg cctttgggcc gcaccagcaa gaccttagga tccctttgac gctccacacg 1140
gtcccccctg gggcccggat ccagtttcag ggagctccgc cttcagagct gataagattg 1200
accaaggtcc ccctgacacc agtgcctact aaaatgcagt ccctactgga gccttctgta 1260
aaaattgaaa ccaaagatgt cccgctcacc gtgttgccct cagatgcagg cataccagat 1320
actcccttca gtaaggacag aaatggtcat gtgaagcgac ccatgaacgc atttatggtt 1380
tgggcaagga tccaccgacc agcactagcc aaagctaacc cagcagccaa caatgcagaa 1440
atcagtgtcc agcttgggtt agagtggaac aaacttagtg aagaacaaaa gaaaccctat 1500
tacgatgaag cacaaaagat taaggaaaag cacagagagg aatttcctgg ttgggtttat 1560
cagcctcgtc cagggaagcg aaaacgattc cctctaagtg tttccaatgt attttctggt 1620
accacacaga atatcatctc tacaaatcct acaacagttt atccttaccg ctcacctacg 1680
tactctgtgg taattcccag cctacagaat cccatcactc atccagttgg tgaaacctca 1740
cctgctatcc agctgcccac acctgcagtc cagagcccaa gccctgtcac acttttccag 1800
cccagcgtct ccagtgctgc tcaggtggct gtccaggatc caagtctacc tgtctatcca 1860
gcactcccac cccaacgctt tactgggcct tcccaaacag acactcatca gctgcattct 1920
gaagccactc acactgtgaa gcaacccact cctgtctctc tagagagcgc caacaggatt 1980
tcaagtagtg caagtactgc ccatgccaga tttgcaactt cgaccatcca acctcctagg 2040
gagtattcca gcgtttcccc ttgtcccaga agtgctccaa tcccccaggc ttctcccatt 2100
ccacacccac atgtctacca gccccctccc cttggccatc cagccacact gttcgggaca 2160
ccaccaagat tctcttttca tcacccttac ttcctacccg gacctcacta cttcccatca 2220
agtacatgcc cttacagtcg gcctcccttt ggctatggaa attttccgag ttcaatgcca 2280
gaatgcctta gttattatga agacaggtac ccaaaacatg agggtatctt ttcaacttta 2340
aatagagact attcttttag agactactca agtgaatgca cacacagtga aaattctcgg 2400
agttgtgaga acatgaatgg aacttcttac tataacagtc atagccacag tggggaagaa 2460
aacttaaacc ctgtgcctca gctggacatt ggaaccttgg agaatgtctt cacagccccg 2520

acatcaactc cttctagcat ccagcaagtc aatgtcaccg acagtgatga ggaggaagaa 2580

gaaaaagtgc tcagggattt ataattttaa aacaaatatg cacagaaaat aaacatttct 2640

taaaatatat tctgggtcag ttggtatgag aaaaaaaaaa gcctagaatt ctttgttgaa 2700

agttttcagt cgtgatttga ggagttaaaa ccaaatgcaa tttatgtctt cataaaattt 2760

tgattagtga aactagagtc tggatgtttc attgtaggaa tatttaagtt attaagtagt 2820

ttaattttaa tggctgaaat ttgcatcaac atgtattatt attactttat cctggaacat 2880

gcaaaatact gaagcctcac agttgtatgt gaggggaaag gggaaataaa tctagcatag 2940

tgtgattttt attttatctc aggatacatt ttttaaatga ttttttgttt gctttttatg 3000

taatacttat ggatgttgtc aatttttgat gtaacatttt gaaagtattt tgacaactcc 3060

tagtgaactt ggacttggtt gctaaattta acttacacta ataaccaatt ataagttcca 3120

aatgtgtttt aatggcacct gggtgattct tcagctaaat ttagtcattt ctgtttctaa 3180

atatttttat cattttaaaa tatttttttt ccatttggca tacatcgttc tttgttgtaa 3240

ttaaataaac atagataaaa ttgttaaaaa aaaaaaaaaa 3280

<210> 7
<211> 1842
<212> DNA
<213> Homo sapiens

<223> FGFR1 oncogene partner (FGFR1OP), transcript variant 2, mRNA

<400> 7
cggccgcgta gcccgcgcgc ggagcgtacc ctgctgcggc cgttggccgt tagcgcggct 60

tcggcggttg tcttggagaa gcaagatggc ggcgacggcg gccgcagtgg tggccgagga 120

ggacacggag ctgcgggacc tgctggtgca gacgctggag aacagcgggg tcctgaaccg 180

catcaaggct gaactccgag cagctgtgtt tttagcacta gaggagcaag aaaaagtaga 240

gaacaaaact cctttagtta atgagagcct gaaaaagttt ttaaatacca aagacggtcg 300

tttagtggct agtcttgttg cagaatttct tcagtttttt aaccttgact ttactttggc 360

tgtttttcaa cctgaaacta gcacactgca aggtctcgaa ggtcgagaga atttagcccg 420

agatttaggt ataattgaag cagaaggtac tgtgggtgga cccttattat tagaagtgat 480

caggcgctgt caacagaaag aaaaagggcc aaccactggg gaaggtgcac ttgatctatc 540

tgatgtacat tctccaccaa agtcaccaga gggaaaaaca agtgcacaga caacaccaag 600

taagaaggcc aatgatgagg ccaatcagag tgatacaagt gtctccttgt cagaacccaa 660

gagcaaaagc agccttcact tactgtccca tgaaacaaaa attggatctt ttctaagcaa 720

cagaacttta gatggcaaag acaaagctgg cctttgtcca gatgaagatg atatggaagg 780

agattctttc tttgatgatc ccattcctaa gccagagaaa acttacggtt tgaggaagga 840

acctaggaag caagcaggaa gtctggcctc gctctcggat gcacccccct taaaaagtgg 900

actcagctcc ctggcgggag ccccttcttt aaaagactct gagagtaaaa ggggaaatac 960

agttttgaaa gatctgaaat tgatcagtga taaaattgga tcacttggat taggaactgg 1020

agaagatgat gactatgttg atgattttaa tagtaccagc catcgctcag agaaaagtga 1080

gataagtatt ggtgaagaga tagaagaaga cctttctgtg gaaatagatg acatcaatac 1140

cagtgataag cttgatgacc tcacacaaga tctgactgta tcccagctca gtgatgttgc 1200

ggattatctg gaagatgttg catagacacg aagaaggaag tattctaatt aacaaggaca 1260

gaggactgac cggttccatt tttttttttt ccagacaatc actcagctgg aatgtctgct 1320

ctctattggt gccttgcatt tcaaaaacac tgcagatatt ttttaaaagt aattttcatt 1380

ttactaaaca aaatacttcc tatttgagcc catgtgtgga agatttaata ttcttaattt 1440

aactgtacat ttctttatgg aaattgatta tctacactca gtttcattac agggaaggaa 1500

cccatgaaaa catcagtgtt aagagcatga tgaaaggtgt caataaagcc gtaggatcgc 1560

gcaacccttt gtgtgtgtgg ctgctggtac gtgtgatctt tgaaaacctt ggctttagcc 1620

ctctggaatc agagcttacc caccatagta tattttgata ttaggtggtt ctacacatag 1680

ttggcaaaat gacttggtaa atttgtaatg ctgaagtata ttagtataag ttaaatttga 1740

tgtgtcaact ttattttgta tttccttcca tttggaaggt ttgttagacc attaaggtta 1800

tattaaagta ctcttgtgtg tgctaaaaaa aaaaaaaaaa aa 1842

<210> 8
<211> 1278
<212> DNA
<213> Homo sapiens

<223> lactate dehydrogenase C (LDHC), transcript variant 1, mRNA

<400> 8
cgtgcgtgtc tcgagtcgca cggacggcaa ccgtcgacgg gcttagcgcc tcaactgtcg 60

ttggtgtatt tttctggtgt cacttctgtg ccttccttca aaggtggtgc tttgtccctg 120

tgggtcatct gtactgattg cgccaagaaa gcatttgttc tccaaatgtc aactgtcaag 180

gagcagctaa ttgagaagct aattgaggat gatgaaaact cccagtgtaa aattactatt 240

gttggaactg gtgccgtagg catggcttgt gctattagta tcttactgaa ggatttggct 300

gatgaacttg cccttgttga tgttgcattg gacaaactga agggagaaat gatggatctt 360

cagcatggca gtcttttctt tagtacttca aagattactt ctggaaaaga ttacagtgta 420

tctgcaaact ccagaatagt tattgtcaca gcaggtgcaa ggcagcagga gggagaaact 480

cgccttgccc tggtccaacg taatgtggct ataatgaaat caatcattcc tgccatagtc 540

cattatagtc ctgattgtaa aattcttgtt gtttcaaatc cagtggatat tttgacatat 600

atagtctgga agataagtgg cttacctgta actcgtgtaa ttggaagtgg ttgtaatcta 660

gactctgccc gtttccgtta cctaattgga gaaaagttgg gtgtccaccc cacaagctgc 720

catggttgga ttattggaga acatggtgat tctagtgtgc ccttatggag tggggtgaat 780

gttgctggtg ttgctctgaa gactctggac cctaaattag gaacggattc agataaggaa 840

cactggaaaa atatccataa acaagttatt caaagtgcct atgaaattat caagctgaag 900

gggtatacct cttgggctat tggactgtct gtgatggatc tggtaggatc cattttgaaa 960

aatcttagga gagtgcaccc agtttccacc atggttaagg gattatatgg aataaaagaa 1020

gaactctttc tcagtatccc ttgtgtcttg gggcggaatg gtgtctcaga tgttgtgaaa 1080

attaacttga attctgagga ggaggccctt ttcaagaaga gtgcagaaac actttggaat 1140

attcaaaagg atctaatatt ttaaattaaa gccttctaat gttccactgt ttggagaaca 1200

gaagatagca ggctgtgtat tttaaatttt gaaagtattt tcattgatct taaaaaataa 1260

aaacaaattg gagacctg 1278

<210> 9
<211> 1354
<212> DNA
<213> Homo sapiens

<223> testes-specific protease 50 (TSP50), mRNA

<400> 9
   agcggactgg gagcgccttc cggagagacg cagtcggctg ccaccccggg atgggtcgct 60
   ggtgccagac cgtcgcgcgc gggcagcgcc cccggacgtc tgccccctcc cgcgccggtg 120
   ccctgctgct gctgcttctg ttgctgaggt ctgcaggttg ctggggcgca ggggaagccc 180
   cgggggcgct gtccactgct gatcccgccg accagagcgt ccagtgtgtc cccaaggcca 240
   cctgtccttc cagccggcct cgccttctct ggcagacccc gaccacccag acactgccct 300
   cgaccaccat ggagacccaa ttcccagttt ctgaaggcaa agtcgaccca taccgctcct 360
   gtggcttttc ctacgagcag gaccccaccc tcagggaccc agaagccgtg gctcggcggt 420
   ggccctggat ggtcagcgtg cgggccaatg gcacacacat ctgtgccggc accatcattg 480
   cctcccagtg ggtgctgact gtggcccact gcctgatctg gcgtgatgtt atctactcag 540
   tgagggtggg gagtccgtgg attgaccaga tgacgcagac cgcctccgat gtcccggtgc 600
   tccaggtcat catgcatagc aggtaccggg cccagcggtt ctggtcctgg gtgggccagg 660
   ccaacgacat cggcctcctc aagctcaagc aggaactcaa gtacagcaat tacgtgcggc 720
   ccatctgcct gcctggcacg gactatgtgt tgaaggacca ttcccgctgc actgtgacgg 780
   gctggggact ttccaaggct gacggcatgt ggcctcagtt ccggaccatt caggagaagg 840
   aagtcatcat cctgaacaac aaagagtgtg acaatttcta ccacaacttc accaaaatcc 900
   ccactctggt tcagatcatc aagtcccaga tgatgtgtgc ggaggacacc cacagggaga 960
   agttctgcta tgagctaact ggagagccct tggtctgctc catggagggc acgtggtacc 1020
   tggtgggatt ggtgagctgg ggtgcaggct gccagaagag cgaggcccca cccatctacc 1080
   tacaggtctc ctcctaccaa cactggatct gggactgcct caacgggcag gccctggccc 1140
   tgccagcccc atccaggacc ctgctcctgg cactcccact gcccctcagc ctccttgctg 1200
   ccctctgact ctgtgtgccc tccctcactt gtgggccccc cttgcctccg tgcccaggtt 1260
   gctgtaggtg cagctgtcac agccctgaga gtcagggtgg agatgaggtg ctcaattaaa 1320
   cattactgtt ttccatgcaa aaaaaaaaaa aaaa 1354

<210> 10
<211> 872
<212> DNA
<213> Homo sapiens

<223> lysozyme-like 6 (LYZL6), mRNA

<400> 10
gagattggaa acagccaggt tggagcagtg agtgagtaag gaaacctggc tgccctctcc 60

agattcccca ggctctcaga gaagatcagc agaaagtctg caagacccta agaaccatca 120

gccctcagct gcacctcctc ccctccaagg atgacaaagg cgctactcat ctatttggtc 180

agcagctttc ttgccctaaa tcaggccagc ctcatcagtc gctgtgactt ggcccaggtg 240

ctgcagctgg aggacttgga tgggtttgag ggttactccc tgagtgactg gctgtgcctg 300

gcttttgtgg aaagcaagtt caacatatca aagataaatg aaaatgcaga tggaagcttt 360

gactatggcc tcttccagat caacagccac tactggtgca acgattataa gagttactcg 420

gaaaaccttt gccacgtaga ctgtcaagat ctgctgaatc ccaaccttct tgcaggcatc 480

cactgcgcaa aaaggattgt gtccggagca cgggggatga acaactgggt agaatggagg 540

ttgcactgtt caggccggcc actcttctac tggctgacag gatgccgcct gagatgaaac 600

agggtgcggg tgcaccgtgg agtcattcca agactcctgt cctcactcag ggattcttca 660

tttcttcttc ctactgcctc cacttcatgt tattttcttc ccttcccatt tacaactaaa 720

actgaccaga gccccaggaa taaatggttt tcttggcttc ctccttactc ccatctggac 780

ccagtcccct ggttcctgtc tgttatttgt aaactgagga ccacaataaa gaaatcttta 840

tatttatcga aatattaaag cactcgctct gc 872

<210> 11
<211> 2674
<212> DNA
<213> Homo sapiens

<223> proprotein convertase subtilisin/kexin type 4 (PCSK4), mRNA

<400> 11
cccactcggc ggtttggcgg gagggagggg atttgcgcag gccccgctcc cgccccgcct 60

ccatgcggcc cgccccgatt gcgctgtggc tgcgcctggt cttggccctg gcccttgtcc 120

gcccccgggc tgtggggtgg gccccggtcc gagcccccat ctatgtcagc agctgggccg 180

tccaggtgtc ccagggtaac cgggaggtcg agcgcctggc acgcaaattc ggcttcgtca 240

acctggggcc gatcttccct gacgggcagt actttcacct gcggcaccgg ggcgtggtcc 300

agcagtccct gaccccgcac tggggccacc gcctgcacct gaagaaaaac cccaaggtgc 360

agtggttcca gcagcagacg ctgcagcggc gggtgaaacg ctctgtcgtg gtgcccacgg 420

acccctggtt ctccaagcag tggtacatga acagcgaggc ccaaccagac ctgagcatcc 480

tgcaggcctg gagtcagggg ctgtcaggcc agggcatcgt ggtctctgtg ctggacgatg 540

gcatcgagaa ggaccacccg gacctctggg ccaactacga ccccctggcc agctatgact 600

tcaatgacta cgacccggac ccccagcccc gctacacccc cagcaaagag aaccggcacg 660

ggacccgctg tgctggggag gtggccgcga tggccaacaa tggcttctgt ggtgtggggg 720
tcgctttcaa cgcccgaatc ggaggcgtac ggatgctgga cggtaccatc accgatgtca 780
tcgaggccca gtcgctgagc ctgcagccgc agcacatcca catttacagc gccagctggg 840
gtcccgagga cgacggccgc acggtggacg gccccggcat cctcacccgc gaggccttcc 900
ggcgtggtgt gaccaagggc cgcggcgggc tgggcacgct cttcatctgg gcctcgggca 960
acggcggcct gcactacgac aactgcaact gcgacggcta caccaacagc atccacacgc 1020
tttccgtggg cagcaccacc cagcagggcc gcgtgccctg gtacagcgaa gcctgcgcct 1080
ccaccctcac caccacctac agcagcggcg tggccaccga cccccagatc gtcaccacgg 1140
acctgcatca cgggtgcaca gaccagcaca cgggcacctc ggcctcagcc ccactggcgg 1200
ccggcatgat cgccctagcg ctggaggcca acccgttcct gacgtggaga gacatgcagc 1260
acctggtggt ccgcgcgtcc aagccggcgc acctgcaggc cgaggactgg aggaccaacg 1320
gcgtggggcg ccaagtgagc catcactacg gatacgggct gctggacgcc gggctgctgg 1380
tggacaccgc ccgcacctgg ctgcccaccc agccgcagag gaagtgcgcc gtccgggtcc 1440
agagccgccc cacccccatc ctgccgctga tctacatcag ggaaaacgta tcggcctgcg 1500
ccggcctcca caactccatc cgctcgctgg agcacgtgca ggcgcagctg acgctgtcct 1560
acagccggcg cggagacctg gagatctcgc tcaccagccc catgggcacg cgctccacac 1620
tcgtggccat acgacccttg gacgtcagca ctgaaggcta caacaactgg gtcttcatgt 1680
ccacccactt ctgggatgag aacccacagg gcgtgtggac cctgggccta gagaacaagg 1740
gctactattt caacacgggg acgttgtacc gctacacgct gctgctctat gggacggccg 1800
aggacatgac agcgcggcct acaggccccc aggtgaccag cagcgcgtgt gtgcagcggg 1860
acacagaggg gctgtgccag gcgtgtgacg gccccgccta catcctggga cagctctgcc 1920
tggcctactg ccccccgcgg ttcttcaacc acacaaggct ggtgaccgct gggcctgggc 1980
acacggcggc gcccgcgctg agggtctgct ccagctgcca tgcctcctgc tacacctgcc 2040
gcggcggctc cccgagggac tgcacctcct gtcccccatc ctccacgctg gaccagcagc 2100
agggctcctg catgggaccc accacccccg acagccgccc ccggcttaga gctgccgcct 2160
gtccccacca ccgctgccca gcctcggcca tggtgctgag cctcctggcc gtgaccctcg 2220
gaggccccgt cctctgcggc atgtccatgg acctcccact atacgcctgg ctctcccgtg 2280
ccagggccac ccccaccaaa ccccaggtct ggctgccagc tggaacctga agttgtcagc 2340

tcagaaagcg accttgcccc cgcctgggtc cctgacaggc actgctgcca tgctgcctcc 2400

ccaggctggc cccagaggag cgagcaccag cacccgacgc ctggcctgcc agggatgggc 2460

cccgtggaac cccgaagcct ggcgggagag agagagagag aagtctcctc tgcattttgg 2520

gtttgggcgg gagtgggctg gggggagagg ctggagcacc ccaaaagcca ggggaaagtg 2580

gagggagaga aacgtgacac tgtccgcctc gggcaccgcg tccaacctca gagtttgcaa 2640

ataaaggttg cttagaaggt gaaaaaaaaa aaaa 2674

<210> 12
<211> 1727
<212> DNA
<213> Homo sapiens

<223> melanoma antigen family A, 4 (MAGEA4), transcript variant 2, mRNA

<400> 12
ggttagagag aagcgagctg ctctgtctga ccagcagctt gggattggcg gagggaagcg 60

ggccaggccc tgtgaggagt caaggttctg agcagacagg ccaaccggag gacaggattc 120

cctggaggcc acagaggagc accaaggaga agatctgcct gtgggtcccc attgcccagc 180

ttttgcctgc actcttgcct gctgccctga ccagagtcat catgtcttct gagcagaaga 240

gtcagcactg caagcctgag gaaggcgttg aggcccaaga agaggccctg ggcctggtgg 300

gtgcacaggc tcctactact gaggagcagg aggctgctgt ctcctcctcc tctcctctgg 360

tccctggcac cctggaggaa gtgcctgctg ctgagtcagc aggtcctccc cagagtcctc 420

agggagcctc tgccttaccc actaccatca gcttcacttg ctggaggcaa cccaatgagg 480

gttccagcag ccaagaagag gaggggccaa gcacctcgcc tgacgcagag tccttgttcc 540

gagaagcact cagtaacaag gtggatgagt tggctcattt tctgctccgc aagtatcgag 600

ccaaggagct ggtcacaaag gcagaaatgc tggagagagt catcaaaaat tacaagcgct 660

gctttcctgt gatcttcggc aaagcctccg agtccctgaa gatgatcttt ggcattgacg 720

tgaaggaagt ggaccccgcc agcaacacct acacccttgt cacctgcctg ggcctttcct 780

atgatggcct gctgggtaat aatcagatct ttcccaagac aggccttctg ataatcgtcc 840

tgggcacaat tgcaatggag ggcgacagcg cctctgagga ggaaatctgg gaggagctgg 900

gtgtgatggg ggtgtatgat gggagggagc acactgtcta tggggagccc aggaaactgc 960

tcacccaaga ttgggtgcag gaaaactacc tggagtaccg gcaggtaccc ggcagtaatc 1020

ctgcgcgcta tgagttcctg tggggtccaa gggctctggc tgaaaccagc tatgtgaaag 1080

tcctggagca tgtggtcagg gtcaatgcaa gagttcgcat tgcctaccca tccctgcgtg 1140

aagcagcttt gttagaggag gaagagggag tctgagcatg agttgcagcc agggctgtgg 1200

ggaaggggca gggctgggcc agtgcatcta acagccctgt gcagcagctt cccttgcctc 1260

gtgtaacatg aggcccattc ttcactctgt ttgaagaaaa tagtcagtgt tcttagtagt 1320

gggtttctat tttgttggat gacttggaga tttatctctg tttcctttta caattgttga 1380

aatgttcctt ttaatggatg gttgaattaa cttcagcatc caagtttatg aatcgtagtt 1440

aacgtatatt gctgttaata tagtttagga gtaagagtct tgttttttat tcagattggg 1500

aaatccgttc tattttgtga atttgggaca taataacagc agtggagtaa gtatttagaa 1560

gtgtgaattc accgtgaaat aggtgagata aattaaaaga tacttaattc ccgccttatg 1620

cctcagtcta ttctgtaaaa tttaaaaaat atatatgcat acctggattt ccttggcttc 1680

gtgaatgtaa gagaaattaa atctgaataa ataattcttt ctgttaa 1727

<210> 13
<211> 594
<212> DNA
<213> Homo sapiens

<223> serine peptidase inhibitor, Kazal type 2 (acrosin-trypsin inhibitor)
      (SPINK2), mRNA

<400> 13
ggggagcgct ccctgcgagt ggcgcaggta acagaccgca ggggcaagac agccggcgcg 60

ggaggaggat ggcgctgtcg gtgctgcgct tggcgctgct gctcctggca gtcaccttcg 120

cagcctctct gatccctcaa tttggtctgt tttcaaaata tagaacgcca aactgctctc 180

agtatagatt accaggatgt cccagacact ttaaccctgt gtgtggcagt gacatgtcca 240

cttatgccaa tgaatgtact ctgtgcatga aaatcaggga aggtggtcat aatattaaaa 300

tcattcgaaa tggaccctgc tgatggagca gtttacagaa aagaagatgg agagaccacc 360

ttcactggca gactagataa attgcatttc ccctttttct cttttcctgt gtttctttac 420

atgagatttg ttaacacaca ttttctgaga gcaggtatgg aagacagcca tgtgtagtga 480

tggataattt aaagaaaaaa agaatccttg tttcttggct tttgctcctg gagttaagct 540

tactgcccag gtgacttgtg cattgcttta tttagttgaa ataaaatcag catt 594

<210>  14
<211>  1731
<212>  DNA
<213>  Homo sapiens

<223>  maelstrom homolog (Drosophila) (MAEL), mRNA

<400>  14
ttagggcggg agcccggcga gggcgccggt gctttgttct gtctgaggcc aggaagtttg      60

accgcgctgc catgccgaac cgtaaggcca gccggaatgc ttactatttc ttcgtgcagg     120

agaagatccc cgaactacgg cgacgaggcc tgcctgtggc tcgcgttgct gatgccatcc     180

cttactgctc ctcagactgg gcgcttctga gggaggaaga aaaggagaaa tacgcagaaa     240

tggctcgaga atggagggcc gctcagggaa aggaccctgg gccctcagag aagcagaaac     300

ctgttttcac accactgagg aggccaggca tgcttgtacc aaagcagaat gtttcacctc     360

cagatatgtc agctttgtct ttaaaaggtg atcaagctct ccttggaggc attttttatt     420

ttttgaacat ttttagccat ggcgagctac ctcctcattg tgaacagcgc ttcctccctt     480

gtgaaattgg ctgtgttaag tattctctcc aagaaggtat tatggcagat ttccacagtt     540

ttataaatcc tggtgaaatt ccacgaggat ttcgatttca ttgtcaggct gcaagtgatt     600

ctagtcacaa gattcctatt tcaaattttg aacgtgggca taaccaagca actgtgttac     660

aaaaccttta tagatttatt catcccaacc cagggaactg gccacctatc tactgcaagt     720

ctgatgatag aaccagagtc aactggtgtt tgaagcatat ggcaaaggca tcagaaatca     780

ggcaagatct acaacttctc actgtagagg accttgtagt ggggatctac caacaaaaat     840

ttctcaagga gccctctaag acttggattc gaagcctcct agatgtggcc atgtgggatt     900

attctagcaa cacaaggtgc aagtggcatg aagaaaatga tattctcttc tgtgctttag     960

ctgtttgcaa gaagattgcg tactgcatca gtaattctct ggccactctc tttggaatcc    1020

agctcacaga ggctcatgta ccactacaag attatgaggc cagcaatagt gtgacaccca    1080

aaatggttgt attggatgca gggcgttacc agaagctaag ggttgggagt tcaggattct    1140

ctcatttcaa ctcttctaat gaggaacaaa gatcaaacac acccattggt gactacccat    1200

ctagggcaaa aatttctggc caaaacagca gcgttcgggg aagaggaatt acccgcttac    1260

tagagagcat ttccaattct tccagcaata tccacaaatt ctccaactgt gacacttcac    1320

tctcacctta catgtcccaa aaagatggat acaaatcttt ctcttcctta tcttaatgat    1380

ggtactcttt tcaatttctg aaaacagtaa caggcccaac ttccttctta ctacagtcat    1440

attaaacaga tcacatcaat gacaaatgtc actactataa aaactactta atttgtaagg   1500

aaattgtttc atagatttaa aaaaattgtg gttggagagc atcttggcat ttgtgctttt   1560

tttcttgagg gattgttctg cttcctggct gtatgatggg tatatcatta aagtttggag   1620

tcctatatga acaaaactga catttttaga gttgtacttt tgggaatgtt atagattgat   1680

cattctttct cctgataata aaggtattga atatctgtta tgaaaggttc t            1731

<210>  15
<211>  1483
<212>  DNA
<213>  Homo sapiens

<223>  eukaryotic initiation factor 5A isoform II (EIF5A2) alternative mRNA
       B, complete cds

<400>  15
ggaactgccg cggccgccac cgccgctgcc ggcgccgctg ctgtagggga ccagcgcggg     60

tgcgcagacg aaaggcgctc tttgccagct gaaagttccc acggaaaaac taccatctcc    120

cctgcccacc atggcagacg aaattgattt cactactgga gatgccgggg cttccagcac    180

ttaccctatg cagtgctcgg ccttgcgcaa aaacggcttc gtggtgctca aaggacgacc    240

atgcaaaata gtggagatgt caacttccaa aactggaaag catggtcatg ccaaggttca    300

ccttgttgga attgatattt tcacgggcaa aaaatatgaa gatatttgtc cttctactca    360

caacatggat gttccaaata ttaagagaaa tgattatcaa ctgatatgca ttcaagatgg    420

ttacctttcc ctgctgacag aaactggtga agttcgtgag gatcttaaac tgccagaagg    480

tgaactaggc aaagaaatag agggaaaata caatgcaggt gaagatgtac aggtgtctgt    540

catgtgtgca atgagtgaag aatatgctgt agccataaaa ccctgcaaat aaacggaaac    600

atcaggcatg aacactgttt atgtctgaat caactgcaga tctaatttgg ttctaagttg    660

tcaccaaagc tatagccttc ataagcaacc tcatttcttt ttttaattgt tttcagattg    720

tgctgggtta gttttgcaag caattgataa tttttaaaaa attatcaata tgtataattt    780

tttttgaatt ttgtagatat gtttcctata atattcctgt ggttttcagt atgtcagtcc    840

aagaaacaaa caagatagct tcagcagatg tgatttgtct gagcaaatca ttcctgtgtt    900

ttagttagat gataaaccag ggttttaaat caaacaatgt agcataaagt ggtattgaag    960

taccatattt aagttgaact gctctgcatt aattacagat ttaatataaa atgaactgat   1020

tatatattgg aattgttgca ctttcagcct gtgtagggca aacacaaatc cttaaacaaa 1080

aatcatgtta tcaaaaactg tcagttattt tggtggccac atgcaaccat gtgattactt 1140

tggtcatact atttaccaaa ttgttggcaa acttgatgta accttatatg gtggttttag 1200

tcccttctta tggttggcag aagggcactg aactgttggg tgaaagttag ttagcaagta 1260

agattataag gaagacacaa aagacagtgg caaagagggt tgattaaatc tataagtttt 1320

tatgtgagga ctttgtaagt cagcataaaa atgaaaagtt gtttctcagt tgtttttgct 1380

tttaactttg cccccaacgt ttaaagggag tgatctgctt tacatgatac tatgcaatgc 1440

ttgttttcca actatgttga ataaatagta atatttatca gta 1483

<210> 16
<211> 2323
<212> DNA
<213> Homo sapiens

<223> Homo sapiens olfactory receptor, family 4, subfamily N, member 4
 (OR4N4)

<400> 16
attaaacttt aatgggtaac tagtagacag taacactctg ggagggctca aaccacataa 60

ccaaatgtgc caagataatc ctgagctcac tattgtcagt cctgaagatt ttcctcagat 120

aatctttcat ctctttgttc ctgagactat atattagagg attacagagt ggtgttatca 180

cagaatagaa caaggtcact tgatattctt ggcttgatga aaaaaaacaa gattctaacg 240

tgacagaact tgttcttctg ggcctatcat cttcttggga gctgcagcta tttctcttat 300

tactattttt gtttttttac attgctattg tcctgggaaa cctcttgata gtggtaacag 360

tgcaagccca tgctcatctg ctccaatctc ctatgtatta ttttttaggt catctctctt 420

tcattgacct atgcctaagc tgtgttactc tgccaaagat gttaggggat ttcctacagc 480

agggcaagag catctctttt tcaggatgcc tggcccagat ctacttcctc cactttctag 540

gagccagtga gatgtttctg ctgacagtta tggcctatga cagtggattt ttatctgaga 600

tctacaactt atgaagaata caaatgatgt atattctttt ctactcttca tgtatggact 660

agaggggctt gcacagtagt aagaaaaaaa tggctgagac ccacaggaac aaagaaacag 720

gacaggagtt agatgcagag aagagatttc aacaaaagtt tggaaaagat gctaacggta 780

gcatatgagt cacagacaga acattggcac ctttgtttaa gaaactgaat gttaatgtca 840

catctgcctt tggagattac aaatgaaatg gctacctttc tactggaacc ctgaccagaa 900

gtctgccagt tagtaagctg gcttaagaga tcaatctaaa atttacataa ggcttttgaa 960

aaaaagaaaa aagcagaaga aatgaagata gcaaacaaca cagtagtgac agaatttatc 1020

ctccttggtc tgactcagtc tcaagatatt cagctcttgg tctttgtgct gatcttaatt 1080

ttctacctta tcatcctccc tggaaatttt ctcattattt tcaccataag gtcagaccct 1140

gggctcacag cccccctcta tttatttctg ggcaacttgg ccttcctgga tgcatcctac 1200

tccttcattg tgcctcccag gatgttggtg gacttcctct ctgagaagaa ggtaatctcc 1260

tacagaggct gcatcactca gctctttttc ttgcacttcc ttggaggagg ggagggatta 1320

ctccttgttg tgatggcctt tgaccgctac atcgccatct gccggcctct gcactgttca 1380

actgtcatga accctagagc ctgctgtgca atgatgttgg ctctgtggct tgggggtttt 1440

gtccactcca ttatccaggt ggtcctcatc ctccgcttgc ctttttgtgg cccaaaccag 1500

ctggacaact tcttctgtga tgtccgacag gtcatcaagc tggcttgcac cgacatgttt 1560

gtggtggagc ttctaatggt cttcaacagt ggcctgatga cactcctgtg ctttctgggg 1620

cttctggctt cctatgcagt catcctctgc catgttcgta gggcagcttc tgaagggaag 1680

aacaaggcca tgtccacgtg caccactcgt gtcattatta tacttcttat gtttggacct 1740

gctatcttca tctacatgtg ccctttcagg gccttaccag ctgacaagat ggtttctctc 1800

tttcacacag tgatctttcc attgatgaat cctatgattt ataccettcg caaccaggaa 1860

gtgaaaactt ccatgaagag gttattgagt cgacatgtag tctgtcaagt ggattttata 1920

ataagaaact gagaaggagg aattctggct ggaattcata tcattcattt aacaagtcct 1980

gtttttcact ggtacctccc atttgccagg taccattgta ggcaatggag gagagttatg 2040

cataatgaga gaataaactt attatattta aagaatataa aggaaacccc agagtggttg 2100

aagtataatg agtaagtgtg agaaatttaa gggttaagtt ttatgtgact gcaagggtct 2160

ttcggtctga ggtaagaatt ttttcatatt ttaattgtgg taagaaccca ttttaatgtt 2220

ttaagcaaag gagcagttca tctacaatgc tttcctctac tggttagagc aacatcagca 2280

agattttagg cagagattaa taaactgtaa aatatcaaaa acc 2323

<210> 17
<211> 2178
<212> DNA

<213>  Homo sapiens

<223>  ariadne homolog, ubiquitin-conjugating enzyme E2 binding protein, 1
       (Drosophila) (ARIH1), mRNA

<400>  17
gctcagtagc gatagcagcg gccgtggagg tggcgttggg gactgttttc tctcggaggc      60

cggagcggag ccgcgtctga ctgaggcggg cagcaagcgg ccccctcgct ccctccctcc     120

ctcctccgcg ccctccccgc cgccaccagc cgtcaaacgc caaccgccgc tcctggggag     180

gagccgcggc tcgcggggcc ggagccaggc ctgcgtccgg acatcagccg gagccggagc     240

gagagccggg gcctcggcgt ccccgccctc tccccgcctc ggccagcgtc cgccgggccc     300

ccgcgcgtcg cgccatggac tcggacgagg gctacaacta cgagttcgac gaggacgagg     360

agtgcagtga ggaggacagc ggcgccgagg aggaggagga cgaagacgac gacgagccgg     420

acgatgatac cctggatctg ggcgaggtgg agctggtgga gcccgggctg ggcgtcggcg     480

gggagcggga cggactgctg tgcggggaga cgggcggtgg cggcggcagc gctctggggc     540

ccggcggtgg cggcggcggc ggcggcggcg gtggtggtgg cgggccgggg catgagcagg     600

aggaggatta ccgctacgag gtgctcacgg ccgagcagat tctacaacac atggtggaat     660

gtatccggga ggtcaacgag gtcatccaga atccagcaac tatcacaaga atactcctta     720

gccacttcaa ttgggataaa gagaagctaa tggaaaggta ctttgatgga aacctggaga     780

agctctttgc tgagtgtcat gtaattaatc caagtaaaaa gtctcgaaca cgccagatga     840

atacaaggtc atcagcacag gatatgcctt gtcagatctg ctacttgaac taccctaact     900

cgtatttcac tggccttgaa tgtggacata agttttgtat gcagtgctgg agtgaatatt     960

taactaccaa aataatggaa gaaggcatgg gtcacactat ttcgtgtcct gctcatggtt    1020

gtgatatctt agtggatgac aacacagtta tgcgcctgat cacagattca aaagttaaat    1080

taaagtatca gcatttaata acaaatagct ttgtagagtg caatcgactg ttaaagtggt    1140

gtcctgcccc agattgccac catgttgtta aagtccaata tcctgatgct aaacctgttc    1200

gctgcaaatg tgggcgccaa ttttgcttta actgtggaga aaattggcat gatcctgtta    1260

aatgtaagtg gttaaagaaa tggattaaaa agtgtgatga tgacagtgaa acctccaatt    1320

ggattgcagc caacacaaag gaatgtccca aatgccatgt cacaattgag aaggatggtg    1380

gttgtaatca catggtctgt cgtaaccaga attgtaaagc agagttttgc tgggtgtgtc    1440

ttggcccatg ggaaccacat ggatctgcct ggtacaactg taaccgctat aatgaggatg    1500

atgcaaaggc agcaagagat gcacaggagc gatctagggc agccctgcag aggtacctgt 1560

tctactgtaa tcgctatatg aaccacatgc agagcctgcg ctttgagcac aaactatatg 1620

ctcaggtgaa acagaaaatg gaggagatgc agcagcacaa catgtcctgg attgaggtgc 1680

agttcctgaa gaaggcagtt gatgtcctct gccagtgtcg tgccacactc atgtacactt 1740

atgtcttcgc tttctacctc aaaaagaata accagtccat tatctttgag aataaccaag 1800

cagatctaga gaatgccaca gaggtgctct cgggctacct tgaacgagat atttcccaag 1860

attctctgca ggatataaag cagaaagtac aagacaagta cagatactgt gagagtcgac 1920

gaagggtttt gttacagcat gtgcatgaag gctatgaaaa agatctgtgg gagtacattg 1980

aggactgaga atggccctgc ataaaatgaa ctctgaaaac tttaccatct agagtgctca 2040

tgcaattaaa acaaaacaaa cacaaacaag gaggcactaa gcctattctg acaccactgg 2100

tctgtagtac cagaattgtt ttgttaatgg aaagtttaag taaattatat tgtaataaaa 2160

aggtagataa accattgt 2178

<210> 18
<211> 1836
<212> DNA
<213> Homo sapiens

<223> coiled-coil domain containing 19 (CCDC19), mRNA

<400> 18
gggcgtgtgg ctgaggagat gccactaagc acagctggca tcctgagctc ctcttctgcc 60

gcttccaaca ggtcaaggaa taaggctcgc tatcggacca aagccgtgag ctctgaggtg 120

gatgagagcc tctttggaga tatcaagtcc ccagcccagg gccagagcga cagccccatt 180

gtgctgctcc gagataagca tacccttcaa aaaactctca ctgctttggg cttggatcgc 240

aagccagaga ccatccagct catcacccgg gacatggtcc gagaactcat tgttcccaca 300

gaggatccct ccggggagtc cctaatcatc agccctgagg agtttgagcg aatcaaatgg 360

gcatcccatg tcctgaccag agaagaactt gaggccaggg accaggcctt caagaaggag 420

aaggaagcca ccatggatgc agtgatgaca cgaaagaaga tcatgaaaca gaaggagatg 480

gtgtggaaca acaacaagaa gctcagtgac ctggaggagg tggccaagga acgggcccag 540

aacctcctgc agagagccaa caagctgcgg atggagcagg aggaggagct caaggacatg 600

agcaagatta tcctcaatgc taagtgccat gccatccggg atgcccaaat cctggagaag 660

cagcagatcc aaaaagaact ggacacagaa gagaagcggt tggatcagat gatggaagtg 720

gagcggcaga aatccattca aaggcaggag gaactggaga ggaagaggag ggaggaaaga 780

attagaggaa ggcggcaaat tgtggaacag atggaaaaga accaggagga gcgatcgctg 840

cttgctgagc agcgggagca ggagaaggag cagatgctgg aatatatgga acagctccaa 900

gaggaagatc taaaggacat ggaacgaagg cagcaacaaa aactgaagat gcaagctgag 960

attaagcgca tcaatgatga aaaccagaaa cagaaagcag aactgctggc tcaggagaag 1020

ctggcagacc agatggtgat ggagtttacc aagaagaaga tggctcgaga agcagagttt 1080

gaggctgagc aggagagaat ccggagggag aaagagaagg agatcgcacg cttgagggcc 1140

atgcaggaga aggcccagga ttaccaggca gaacaggatg ccttgcgggc caagcgcaac 1200

caggaggttg cagacagaga gtggcgcaga aaggaaaagg aaaatgcgcg gaagaagatg 1260

gaaacagagg ctgagctgcg aaaaagtcgg ctcgaacagg tggctttcaa ggagcacgct 1320

ctggctgttc aggtgcaacg ggaccgggat gagttcgaga ggattcttcg ggctcagaga 1380

gaacagattg agaaggagcg gctggaggag gagaaaaagg ccacagggcg cttacagcat 1440

gccaatgagc tccggcgcca ggtgcgcgag aaccagcaga aggaagtgca gaaccggatt 1500

gccacctttg aggagggccg gcgcctcaaa gaggaggccc agaaacgccg tgagcgcatc 1560

gatgagatca agaggaaaaa gcttgaagag ctgagagcca ctggccttcc cgagaagtac 1620

tgcattgaag ctgagcgcaa agctaacatc ctgccagcta cctctgtgaa ctgaggggag 1680

ccttcgtggc cctcaggatg ccttcggggg acagattctg cccagtctct gggcatccat 1740

aattgctgct aacctagaca tttcatagtt acagattaaa tctacttgac tttaaaaaaa 1800

aaaaaaaaaa aaaaaaaaaa aaaaaaaaaa aaaaaa 1836

<210> 19
<211> 726
<212> DNA
<213> Homo sapiens

<223> chromosome 9 open reading frame 9 (C9orf9), mRNA

<400> 19
   ctcccaagtc ctggaacggg cctccccacc ctccggggca ccgacactca cggagtcact 60
   ccaacttctc tgggtgctcc gtggagcgaa gagcgctcta ggacaagcaa ggattcctct 120
   tctcctgtaa atgaccacag aggaagacaa tgaatgaggt gaaagaatcc cttcgcagca 180
   tcgagcagaa gtacaagctc ttccagcagc agcagctcac cttcaccgcc gctctggagc 240
   actgcaggga gaacgcccac gacaagatcc ggcccatctc cagcattgga caggtgcaga 300
   gctacatgga acactactgc aacagctcca cagaccggcg ggttctgctc atgttcctgg 360
   acatctgttc agagctgaat aagctctgcc agcactttga ggccgtgcac tctggcaccc 420
   cagtcaccaa caacctcctg gagaaatgca aaaccctcgt tagccaaagc aacgacttaa 480
   gcagcctcag agcaaaatac cctcatgatg tggtgaacca cctcagctgt gacgaggccc 540
   ggaaccacta cggcggcgtg gtcagcctca tccccctcat cctagactta atgaaagaat 600
   ggatcgccca ctccgagaag ttgccgcgca aggtgctgca gcacgggacg acttagcttc 660
   tgtattattg ttgcttcctc agggggagac agtcaagaat aaaaagtatt ctaccaccta 720
   aaaaaa 726

<210> 20
<211> 9697
<212> DNA
<213> Homo sapiens

<223> HUMCHIKER Human keratin (psi-K-alpha) pseudogene, exons 4,5,6,7 and
      8, and keratin (psi-K-beta) pseudogene, complete cds

<400> 20
   atattcatag ccctgtatgt ttgagtaatg acaacatcca tctccacctt cagggtgagg 60
   agctgttggc ctatctgtgt ctgtctgtct gtctgtctgt catccgtacc tgtctctccg 120
   agggagagaa ggcaggcccc agggtgtttc cccaggatgg ccttgggtga gttcctgctg 180
   tcctctctgc ccaggaaatg aacgcccttt gaggtcaggt ggacgaggat gtcagtgtga 240
   agatggacac tgtgcctgga gtgaacctga gctgcatcct gaatgagatg cgtgaccagg 300
   acaagaaact ggtggagaag agctgcaagg atgcccaggg ctggttcttc agcgtggtgg 360
   gtggccgtgt gtaagcaggt gtgcacacgt gtgggccaca tacgccgtgt gctggtgcag 420
   ttggaacacc ggcagattca caggctgtcc cagttggaag gacttttgga aaccagtcgg 480
   accagccctt catgtcttcg atgtaaaatg tgaggctcag agaggactca agctcacaca 540
   gcccttcact gtggcctgca aaatagatcc agttctctgc aagtctggtc ttgggtttcc 600
   accacagctg tttacaggat gtgtgtattt gagtactaca catacccttg gcaagcacag 660
   gctgagtgtg tccggtgtcc tagggacagc aacaggtgca aaaggataac acccagtgcc 720
   tgtctttgag gtgctgtagt tcggtaggag taagaaatgc aaacgaccgc agagtaggct 780
   gaattcctcc aaggtccaac atgggtgcag agagtctttg tgtgcaggtg cttttggggc 840
   cccatagagg ccagggaggg tttaggggat ggttctggga ggcgagggct caggaacagc 900
   ccctttcctg tgccccactt gtgaaagtct ccataaaccc ctgtggccca ctctgccttc 960
   cagagagagg ggctgaactg caaggtggcc accaacacag aggccctgca gagcggcagg 1020
   atggagatgt ggagctctac atctctgtgc agaacctgag ccgtcccagc tcagcaaggt 1080
   agggcccctg tcccctcccc accctgtacc tgtcacccca gggtgagcgc acgcttatag 1140
   acccctgctt ggagaaccag agtccccacc cagcctgtcc cttcacactc agtggctggg 1200
   tgcactacca cccaactgtg ccggcagcac ccacagctcc atacaggagg ggctcctctc 1260
   tagtgctctg ccatggggaa gggtcccacg tccatctcat cgcaccacct atctcttggc 1320
   ccacagaaag catcgctgga gggcagcctg gtggagacgg aggtgtgtta caggacccag 1380
   ctggcccagc tgcaggggct catcagaagc atggaacagc agctgtgcga gctctgctgt 1440
   gatgcagagc accaggacca cgagcaccag gtccttctgg acgtgaagac gcggctggag 1500
   caggagatcg ccacctacag ccgcttgcta gaggttgagg acgcccggtg agtgtgcacc 1560
   caggtacctg ctggggcggg ctgggggcca cttcacccag ggagaagttg gtgtctgagc 1620
   accagcaaag ttcaggaggt gtgaatgtgg gaacctgtgg ggtttgcagg aggtgaaact 1680
   gacgatgcag gctggagtct gactgaggag ccttgactgc caagttaaag cgtctggact 1740
   agatcacgta ggcaatgggg agccatggag ggatttggag cagaagaagg gaatgaacat 1800
   caagatattt tagaacattc actctggctg cagagggaga aatggatcag aggggtcagg 1860
   gtggggccag agagacgcgt cagggggcta gagcagggag tctggccaga gaagtcttgc 1920
   gcggggtgga ggctgggtgg gggggcaggg gaaggaaggt ggtgcacgca gaagagaggt 1980
   tatagctcaa aacagcagga ctggatgcct ggatctcagg gtaagcgtgg ctcacggtca 2040
   ggactcagta agcgttgggt gaacacatat gaaggagtgg gcattgatgg ccctgggttt 2100
   ctggttccga tgactgtgtg agtccatcag ccacagggtg aagagcatgg tgggtggtgg 2160
   tcgggtttgc agttgggaag ggtgatcagg ccttcagctg agtgtgtcct ggagtctcca 2220
   tgcttagtca cacgttgcag ctttttgctc cccgaaaatg gtgaagtcca tctatagtct 2280
   aacgacagtc tctcctgctt taattgggtc tatttgttgg gtcctctggg acatggaaaa 2340
   accacttgct cagcttctcc ttgtaaattc ctggtgagtg cctccaggcc tactgctgtg 2400
   ctgtttcttt ttcttcttcc tgctgcactg aacccctgcc ctttcattct tgggcctgtg 2460
   ctaatttctg tgcattccca actgtgattt ttcaccaatt taggggaacc tcctctgcca 2520
   gggcctgctt ctccccagca gtgcttgcag gggcctgggc tggctggcat ccctgggtcg 2580
   atgggtgctc ctctccctgc aggctggcca ctcagtactc cttgtccctg gcctcgcagc 2640
   cacccggaag gtaagaagct gccctgtccc tgctgtgtgg accccaacgg cggagaggct 2700
   gagacccttc tgtgtgggag actgggggac cacagactca gggcaggtgg ggggaagacg 2760
   tcctgcctga tgttccctgt gcctccctcc tccatcgcct gcagccacag tgaccagcca 2820
   ccaggtgtgc catcgtggag gaagtccagg ttggagaggt ggtcttctgt aagcaggtcc 2880
   atctctccac ccactgaggc ccctttctgc ctgtgacagc cccacctcga gggtcatgca 2940
   cagccatcag ctgcagctcc cagcatgcta ctgccacgcc ccgagtgtcc gtctgggcac 3000
   tggtccatga cctgttgtct ttctgtatct actttctgca gcccctcact gaggaggcct 3060
   cctgggtttg tccagtgcct gctattaaag ctttgctcca agttcaatgc ctcgtgtgat 3120
   ctggtctgtt cattggtttg gggcagtgtg ggctgaggga ctgggatagg tggagagttt 3180
   attgagtgtt tgggtattgg cttgtcacct gccaatctgt tctgcccact ccatccaggc 3240
   ctggggctcc tagaagacca gaggggagga ggaggaagga ttaagggtgg agggaaggga 3300
   cagccccttg tattgggaaa agtttcaaga atcagcccct gagtccctgt cacctggtgg 3360
   ctggtccctg ggagggccac acatactttc caaggaccac acctgttggg gagcactccc 3420
   tgcctcagcc cagggtgggt tcaagagcag aaggagaggg gctcggtggg gagggcttct 3480
   gaaggagtgg tccagggcac tgatgagtga gggggtgaga gcgaggcagc tagcccagga 3540
   aagcagaccg gctctcccag ctcggggtgt gtgtggggca gggtggccgc tgtggctgta 3600
   cgcatggggg gtgtcctgtt tgctttgtgg cttggcagcc actgggctat tgaggaatct 3660
   ctctccgctg ctggtggggc tacctgggcc tgtctgggaa gtggaagcca ttggcagtct 3720
   gaaacatgac caccttccac ccagcggcct ggcgggaccc tgggacccag ggcggggctt 3780
   ggggtggtgg gcagggcagg gtggggctgt ggagtggggc acttggccgc ccgaaggagc 3840
   cacatcttgg ggagcctgga gtggggaggg ggctggctgg atggggtctc tgtaagagcc 3900
   tcttcttggg caatgtcctt taggtttcct gtgggctgag acctgagaga aatgcccctc 3960
   ccctgaccct gaggtctctt ggggtacaga agggtgagag gagtggaagg gggcatcccc 4020
   ctctcagttg gtttagatcc tctcttagca cggcccacct ttcccccatc ctatagctga 4080
   gcccctgttt gcccacccca gcctggagtg taaacctggg gaaaggttta cacgaggaga 4140
   ctgggttagc aagggcttct agggatgggg cagggtcaga agctgactgg atggaactgt 4200
   agcccctaga gtgcattcct gggtgtcaga ctctgcagaa ggggccagtg tggacactgc 4260
   acccctctgg agccccaatt tcttatcgcc gatgtgcgtt ggtctccgag gccttgatgg 4320
   catttctatg ctgtgaggtg ggcctggtgg gtttgggctc agtttccaag gcaggatagg 4380
   ctctcggtcc cttctttctc ttcagcaccc agccccgggc cagcaccttg ggtggagcag 4440
   tgtggggtga aagacagcaa cttcaggggg tggtcacctg acattgcaac ccatttcccc 4500
   accagacagg gttgaggtgg gccgggctgg ggtggtggct gcctgggagg gcctggggac 4560
   ggtgaagtcc tgtattctcc tcttctccca tattaggtca tgggaaagca tagctggagg 4620
   gcccgcccaa atcacaggtg acgggcctca gagcagtggc acgcacacgc gtggcaccca 4680
   gcacgaggat ttggagaaat gaggcaaatt cctgatgatg ggtggggagg gggtccccag 4740
   ccacctggga gctggcaggt ggcccgtggt gatgaaagcc caggggaacg gaaacagagg 4800
   agcctgttgt aatcgtacgc ccacttggtg gcctataaag gaagcctgtg aaccccggca 4860
   gccctacact acttggggcc cctcttcttt ccagcccttc tcctgtgtgc ctgcctcccg 4920
   ccgccgccac catgaccacc tccatccgcc agttcacctc ctccagctcc atcaagggct 4980
   cctctggcct ggggggcggc tcgtcccgca cctcctgcca gctgtctggc ggcctgggtg 5040
   ccggctcctg caggccggga tctgctggtg gcctgggcag cgccctcggg ggtagcagct 5100
   actccagctg ctacagcttt ggctctggcg gtggctatgg cagcagcagc tttgagggcg 5160
   ttgatgggct gctggtcgga ggtgagaagg ccaccatgca gaacctcaat gaccgcctgg 5220
   cctcctacct ggacaaggtg cgtgccctgg aggaggccga cactgagctg gaggtaaaga 5280
   tccgtgactg gtaccagagg caggccccgg ggccgcccgt gactacagcc agtactacag 5340
   gataatcgag gagctgcaga acaaggtagg gcctgctggt gggaggggtc tccggggggc 5400
   atgacttttt ccccccaact cctgccctgg ccaaaggctg gagtccagcc atagggtctc 5460
   agggagccaa gggtggtttg gctgtggctt agcttctggg aacctgcctt ggggcccctg 5520
   tgtggccaca tccccctttt ctggggcagt aggctgagtc aggaacaaac aggcctcgtg 5580
   gagccccttg gagactcagt ttctccctcg tggagctcct ccacctggag aggttgtagg 5640
   atgaggcagg aggatgcaga tggagggctg ggcatgggca ctggatgcgt ggtgtccttg 5700
   ctcctttgga gcaggggtca gcaggaaggg gttttgggag gtgtggagtg ggggtgtctg 5760
   agtgagcgtc ccgatagcac ctgctgcggg tgggaggcag aaaggagggg gtgggacctc 5820
   agggtgggga aggcctctga tgtgccttat ttggggattt ttctggcttc tcctttcctc 5880
   ctgctgtccc ttaagacagg ctcagcaaac cgcagggggc ggggctgctg gctggagccc 5940
   agggttaggg attaggaagg gtcctgactt ctgatttggg accactcttt ggtgagggct 6000
   cctttagcct ccttttgggg gagcctgtca ggggcacctc tagctgactg taaaacgagg 6060
   gggttgccca catcccctcc cttgttctag aattctggga cagtttctgc cctggggaca 6120
   ttttcccttt cttttctggt tgcctcatac tcccagccag ctgtctcttc tcctttaagg 6180
   gctgagcctg gcatgggggt ctggtggggt actgagtagc gggggaagaa gaggcacctt 6240
   tgagcccttc agactcctgc ttgcccctcc tctgccaata atacagcacg gggcaaggga 6300
   ggggctgggc gggaagagag gccccaggca ggaagatctg ttcagaactc tggtgtgggc 6360
   tcagcacccc catccgatga cctgactact ctcccatctc cgctgatcct cacagccacc 6420
   gtggacaatg ccaacatcct gctacagaat gacaatgccc atctggctgc tgctgacttc 6480
   cgcaccaagt gagtcctagc tgtgggcttg ggcagcctgg gtcagctggg ggaggatctc 6540
   aggtacccct cctgacccca ggactccttg gttgcttgtg caaggcccag gagctcaggg 6600
   tggggcaatc ctaggagccc cactacttag tccaggatgc agtgaaggca gccagttctg 6660
   aaggttgctg ggcttaggca gggaatagaa gagagggagg ggaggcagga ggcagagaga 6720
   agtaaggaag ctggtgggcg taggatctgg ccctgtgatg gtcccaaggc ccccgggctg 6780
   gaattcgttt ccactcgacc ctctcatcag cccttccaac cccttagagt cctggcaaaa 6840
   tgaaggcagg tgagcagcca ggacctggac ctgcaattcc aagcagcctg ggctgaagtc 6900
   cctgattccc atggcaggtt tgagacagag caggccctgt gctgagtgtg gaggccgaca 6960
   tcaatggccc gtgcaggtgc tggatgagct gaccctggcc agagcgaccc ggagatgcac 7020
   attgagaacc tcaaggagga gctggcctac ctgaagaaga accacgaggt gaggtggctg 7080
   gggcagaagg tcaaagatgc tgaggagtgg gtgcagagcc ctggggttgg gccatggctg 7140
   aggccgtgcg agagagcaca gcaggcgcac tgggattagt caccttagag ggcttccctg 7200
   tctgcggagc cctgatcctt ggggtccagc gtgcagggta gactcctctt tgtaccacac 7260
   tgcttctctg tacccaagga acctcccagg ggcccgcaga ggctccctct acctgccctg 7320
   gcctccctga tgagggcagg ggataagtaa ggaagtctcc ttcttgtccc atttcaaact 7380
   ctcaaagctg aacatctaca cagaagcttg gaaattagag ccggaaattt ttggggcata 7440
   ggcttaataa ttagatttta ttttggagag cccttggtct aatgggggag atagagtctg 7500
   atggtggagg caatactgag cagatgaata aaaatcattt agagggtcag atagagcaga 7560
   ggaagaacaa aggaggggtc cttgtgggga atggggtcac ctcgtggggg aaggcttggg 7620
   agtgagagat caggatgggt ccagatgcgc acatccacat cccctttttc cataggagat 7680
   gaacgccccg cgagccaggt gggcggtgag atcaatgtgg agatgcgctg cccagtgtgg 7740
   acctgagccg catcctgaac gagatgcgtg agcagtatga gaagatggca gagaagaacc 7800
   gcaaggatgc cgaggattgg ttcttcagca aggtgggagc tgctgcaggc cagaggtctc 7860
   tcttcggggc tgggctcagg ggccttagca ctgacagtag gcccacggac aggtgtcttg 7920
   gagatgctcc ctcctcagca agctgcatgg accacagggt cacccactgc atcaacagac 7980
   ctggagctga gctcaagctg ggatctgggg ggtgagtggg gagctaggga gcccccacag 8040
   aataaaggca gagggtaaag accttgggag tccccacctc tctctcaaga agtcagaaac 8100
   tagcaccaag agccaggcta catgttctgg ctggttctca agtttccggt ctgtgcctcc 8160
   cacacgcagg gattaaccat aaaaagttaa catttcaaat ggcatgtttc tgggctttgg 8220
   gacgtggaag ctggtgagaa ggcatcactc tgtgccacag ttaggatttg ggaggaggcc 8280
   tgactgagga gagggatcca ggctcacacc accctgtcct gtgttctgtc tgcagacaga 8340
   ggagctgaac cgcgagtggc taccaacagt gagctggtcc agagcggcaa gagcgagatt 8400
   tcggagctcc ggtgcaccat gcaggccttg gagatcgagc tgcagtccca gctcagcatg 8460
   gtaggaacag tcctgtgcat ggggatgggc ccagaagagg acactgacaa ccctcactga 8520
   cccctggtct tcctgccctc ctgcagaaag catcctggag ggcaacctcg gcggagacag 8580
   agaaccgcta ctgcatgcag ctgtcccaga tccagggact gactgattgg cagtgtggag 8640
   gagcaggctg gcccagcttc tctgcgagat ggagcagcag aaccaggagt acaagatcct 8700
   cctggacatg aagatgcggc tggagctgga gatcaccacc taccaccgcc tgctggaggg 8760
   cgaggatgcc cagtgagtgg gggagcctgg ggtcagggct gggggcctct tggcaggggt 8820
   ggggctctca gactcacatc taatttcctc tctgtttttt ttttctttca gcctgactca 8880
   gtacaagaaa gaacataagc atcttggtgg ctgaggcctt ggggattggg tgcatgggac 8940
   aggcagccca cctgcacgtt gctggagctg ggtccccagg agttctagga gttgatggct 9000
   gtccctcagc aggggtggga gaagtgaccc gttagcactg aggattgata ctcaggaaaa 9060
   gatcaaatga gagagatgct gtcttgggtc attggatggg gtgggccagg gaactggtcc 9120
   ttaccttgga gatcctagtc tgatggagga gacatgtccc agctctggag attgtcatct 9180
   gatgggaaga taggaacatg gtctcatgat ctttgctctt gacagctttc ggatgagcga 9240
   aacgagtcct gtctctgggg actctagcct gatgggaaat agggacctgg tcttgtcctc 9300
   caaattctag tctgatggag aagatatgat tctagcccca gggttcctag tctaatggag 9360
   gagatagggg cctgattttt gtcttggcga tcccagtctg atgggggaga tgggggcaaa 9420
   tctatgccct ggagactgca gagaaatgga gatggatttc atggagtcta catggctcct 9480
   ccctggcagg acacactggg tcagaatcaa ataacccatc tgcggaggca agactcacac 9540
   acgggccacc ggcagaggga tgggatggaa gggaggcggt ggcagggaca ggagggatgt 9600
   gtgtgcagtg tgagtattga ggtgccagtg gaggcactca cagcacctgg gggagacgag 9660
   ggagagagcc ggctcctgtc catgagggct agggggt 9697

<210> 21
<211> 2519
<212> DNA
<213> Homo sapiens

<223> cAMP responsive element modulator (CREM), transcript variant 21,
      mRNA

<400> 21
gataaataaa gaaaacagga aaggaggaaa gcattgatta caaatatctt aacaatgagc 60

aaatgtgcaa ggaaaaaata tattaagaca aatccaagac aaatgaccat ggaaacagtt 120

gaatcccagc atgatggaag tataacagct tctttgacag agagcaagtc tgctcatgtg 180

cagactcaga ctggccaaaa ttcaatccct gctttagctc aggtagcagc aattgcagag 240

acagatgaat ctgcagaatc agaaggtgta attgattctc ataaacgtag agaaatcctt 300

tcacgaagac cctcttatag gaaaatactg aatgaactgt cctctgatgt gcctggtgtt 360

cccaagattg aagaagagag atcagaggaa gaaggaacac cacctagtat tgctaccatg 420

gcagtaccaa ctagcatata tcagactagc acggggcaat acattgctat agcccaaggt 480

ggaacaatcc agatttctaa cccaggatct gatggtgttc agggactgca ggcattaaca 540

atgacaaatt caggagctcc tccaccaggt gctacaattg tacagtacgc agcacaatca 600

gctgatggca cacagcagtt ctttgtccca ggcagccagg ttgttgttca agctgccact 660

ggtgacatgc caacttacca gatccgagct cctactgctg ctttgccaca gggagtggtg 720

atggctgcat cgcccggaag tttgcacagt ccccagcagc tggcagaaga agcaacacgc 780

aaacgagagc tgaggctaat gaaaaacagg gaagctgccc gggagtgtcg caggaagaag 840

aaagaatatg tcaaatgtct tgaaaatcgt gtggctgtgc ttgaaaacca aaacaagact 900

ctcattgagg aactcaaggc cctcaaagat ctttattgcc ataaagtaga gtaactgtct 960

ttgacttgga ccttgtttac tctaatcaag gcaggagatg cagcagtcct acttattgcc 1020

atgtggactt gtgggaagga cacgtgtgac ccttaagaat ccagtttgga ttagtgtttg 1080

aaattgaatt gggaatgttg ttccaggatg tggaatgcag ccgtgatcac acttaccgag 1140

cttactttga tctgtttgtc aatagcatgc aaaaaatgct ttgtttgccc tttgcttctg 1200

ctttttttca gggaagctgc caaagaatgt cgacgtcgaa agaaagaata tgtaaaatgt 1260

ctggagagcc gagttgcagt gctggaagtc cagaacaaga agcttataga ggaacttgaa 1320

accttgaaag acatttgttc tcccaaaaca gattactaga aatatttaac tatgaactga 1380

aggcagcatg tatagttgct tttgaaggaa tacaatatat agctggcaag aatggtggct 1440

tcttttcttt gtatcattca tcttcttctt taatcactta acattcctaa aatgcttcac 1500

tgtacgtagt taagtcgtag ctataacttc aaatttttta aaagagacaa actgtaaaaa 1560

atgtgtgtat tcttaaaatg caatatttgt aaggcttgtt ccaatgccac atacttgcag 1620

ctcccattct atgtgtcatc aatagtgtcc tatgcaataa aattatttgc aggtctttaa 1680

atcattttag gaaaggatga tcaaaaataa tgcatccagc agtacaataa aagtaaacca 1740

caaaaaaata cctcaggaaa gaatagaaag aaagtctatc taatgacatg cctatatgag 1800

aagaatagcc tagatatgaa tatatggcat ttgcagattt ttatattagt tgctttgtta 1860

aaaaaaaaaa gattgtattg ctgtccttga atgccatagt caaagagagt ttttaataga 1920

accatgttgg ttgcactttg tagtgtttgg tgctcattta aatatctgaa catttactac 1980

agtttttaac tctactgtgt aatataaaag atcttgcaga agttcttagt gttggtttaa 2040

tattacctaa tgaaagtgat gacatatttt ttatatctgg aatgagcctg ttgggatcgc 2100

attgcatacc ttcggggtac aagtcagttt ctacactggg tgctgatcct tgcacgcgcc 2160

ctttctacca tctcacggtg gggatggccg cagggctgtg cacccagaag aagatggctg 2220

ttagcgtttc ggccttcata atggcctgag actttctttc tgtaggtggt ctggagctgt 2280

ccggctggtg gccccctatt ttgccattta gcgaacaacc acaggaattt taaaaacaaa 2340

aacatcccaa gattttttca tttcaaaatg cttcaaagtc cacattagat cagatactcc 2400

gctgtcggca cattcagctg aggttcatta caatcgagac tgcaatgtga tctatgtttc 2460

atcttgtttt tataataaaa agcttcaggg aacaagccca aagccctcac cacaaaaaa    2519

<210>  22
<211>  2825
<212>  DNA
<213>  Homo sapiens

<223>  kinesin family member 2C (KIF2C), mRNA

<400>  22
gcgaaattga ggtttcttgg tattgcgcgt ttctcttcct tgctgactct ccgaatggcc      60

atggactcgt cgcttcaggc ccgcctgttt cccggtctcg ctatcaagat ccaacgcagt     120

aatggtttaa ttcacagtgc caatgtaagg actgtgaact tggagaaatc ctgtgtttca     180

gtggaatggg cagaaggagg tgccacaaag ggcaaagaga ttgattttga tgatgtggct     240

gcaataaacc cagaactctt acagcttctt cccttacatc cgaaggacaa tctgcccttg     300

caggaaaatg taacaatcca gaaacaaaaa cggagatccg tcaactccaa aattcctgct     360

ccaaaagaaa gtcttcgaag ccgctccact cgcatgtcca ctgtctcaga gcttcgcatc     420

acggctcagg agaatgacat ggaggtggag ctgcctgcag ctgcaaactc ccgcaagcag     480

ttttcagttc ctcctgcccc cactaggcct tcctgccctg cagtggctga aataccattg     540

aggatggtca gcgaggagat ggaagagcaa gtccattcca tccgtggcag ctcttctgca     600

aaccctgtga actcagttcg gaggaaatca tgtcttgtga aggaagtgga aaaaatgaag     660

aacaagcgag aagagaagaa ggcccagaac tctgaaatga gaatgaagag agctcaggag     720

tatgacagta gttttccaaa ctgggaattt gcccgaatga ttaaagaatt tcgggctact     780

ttggaatgtc atccacttac tatgactgat cctatcgaag agcacagaat atgtgtctgt     840

gttaggaaac gcccactgaa taagcaagaa ttggccaaga aagaaattga tgtgatttcc     900

attcctagca agtgtctcct cttggtacat gaacccaagt tgaaagtgga cttaacaaag     960

tatctggaga accaagcatt ctgctttgac tttgcatttg atgaaacagc ttcgaatgaa    1020

gttgtctaca ggttcacagc aaggccactg gtacagacaa tctttgaagg tggaaaagca    1080

acttgttttg catatggcca gacaggaagt ggcaagacac atactatggg cggagacctc    1140

tctgggaaag cccagaatgc atccaaaggg atctatgcca tggcctcccg ggacgtcttc    1200

ctcctgaaga atcaaccctg ctaccggaag ttgggcctgg aagtctatgt gacattcttc    1260

gagatctaca atgggaagct gtttgacctg ctcaacaaga aggccaagct gcgcgtgctg    1320

gaggacggca agcaacaggt gcaagtggtg gggctgcagg agcatctggt taactctgct 1380

gatgatgtca tcaagatgct cgacatgggc agcgcctgca gaacctctgg gcagacattt 1440

gccaactcca attcctcccg ctcccacgcg tgcttccaaa ttattcttcg agctaaaggg 1500

agaatgcatg gcaagttctc tttggtagat ctggcaggga atgagcgagg cgcagacact 1560

tccagtgctg accggcagac ccgcatggag ggcgcagaaa tcaacaagag tctcttagcc 1620

ctgaaggagt gcatcagggc cctgggacag aacaaggctc acaccccgtt ccgtgagagc 1680

aagctgacac aggtgctgag ggactccttc attggggaga actctaggac ttgcatgatt 1740

gccacgatct caccaggcat aagctcctgt gaatatactt taaacaccct gagatatgca 1800

gacagggtca aggagctgag cccccacagt gggcccagtg gagagcagtt gattcaaatg 1860

gaaacagaag agatggaagc ctgctctaac ggggcgctga ttccaggcaa tttatccaag 1920

gaagaggagg aactgtcttc ccagatgtcc agctttaacg aagccatgac tcagatcagg 1980

gagctggagg agaaggctat ggaagagctc aaggagatca tacagcaagg accagactgg 2040

cttgagctct ctgagatgac cgagcagcca gactatgacc tggagacctt tgtgaacaaa 2100

gcggaatctg ctctggccca gcaagccaag catttctcag ccctgcgaga tgtcatcaag 2160

gccttacgcc tggccatgca gctggaagag caggctagca gacaaataag cagcaagaaa 2220

cggccccagt gacgactgca aataaaaatc tgtttggttt gacacccagc ctcttccctg 2280

gccctcccca gagaactttg ggtacctggt gggtctaggc agggtctgag ctgggacagg 2340

ttctggtaaa tgccaagtat gggggcatct gggcccaggg cagctgggga gggggtcaga 2400

gtgacatggg acactccttt tctgttcctc agttgtcgcc ctcacgagag gaaggagctc 2460

ttagttaccc ttttgtgttg cccttctttc catcaagggg aatgttctca gcatagagct 2520

ttctccgcag catcctgcct gcgtggactg gctgctaatg gagagctccc tggggttgtc 2580

ctggctctgg ggagagagac ggagccttta gtacagctat ctgctggctc taaaccttct 2640

acgcctttgg gccgagcact gaatgtcttg tactttaaaa aaatgtttct gagacctctt 2700

tctactttac tgtctcccta gagtcctaga ggatccctac tgttttctgt tttatgtgtt 2760

tatacattgt atgtaacaat aaagagaaaa aataaaaaaa aaaaaaaaaa aaaaaaaaaa 2820

aaaaa 2825

<210> 23
<211> 1052

<212>  DNA
<213>  Homo sapiens

<223>  ropporin 1-like (ROPN1L), mRNA

<400>  23
gcgcgtccgt agtggcggct ggcgctaggg aactgcaggg tctagggtgt tgttggagtg      60

gcagttggtc cgaatttctc ccgaagcccg cggaggagcg ggtaagagcc ccgcgaatcc     120

ggccccaacc tcgggaacgg gatgggaggc ggccctggcc gcaagccccg cgctgctagc     180

gggtccaccg cgtcgtagcc gacagccgcc cttcttcctc gcagcgcgcc gcgattcacc     240

agcctggtcc cttctgcgga gagcgatgcc gcttcccgac accatgttct gcgctcagca     300

gatccacatt cccccggagc tgccggacat cctgaagcaa ttcaccaagg ctgccatccg     360

cacccagccg gccgacgtgc tgcggtggtc cgcgggctat tttcagctct tgtcgagagg     420

agatccactt cctgtaaagg acagaatgga aatgcccacg gcaacccaga aaacagacac     480

aggcctgact caaggactcc tgaaagtttt gcacaagcag tgtcaccaca agcggtatgt     540

ggaattaaca gatcttgagc agaagtggaa gaacttgtgc ctgccgaagg aaaaattcaa     600

agcgctctta caactggatc cttgtgaaaa caaaatcaag tggataaact ttttagcgct      660

tggatgcagc atgcttggtg ggtccttgaa cactgcgctg aagcacctgt gcgagatcct     720

cacggacgat ccggagggcg ggcccgctcg catccccttc aagacgtttt cctacgttta     780

ccgctacttg gccagattag actcagatgt gtctcccttg gagacggaat cctaccttgc     840

ctctctaaag gaaaatatag acgccaggaa gaacggcatg ataggtcttt cagatttctt     900

ctttccaaag aggaaacttt tagaaagcat tgaaaactct gaagatgtag gccattaata     960

cagagaagaa tacattttaa tgtcaaaata gtgctcttta aaattctggc accaaataca    1020

acttaccctg aatcacaaaa aaaaaaaaaa aa                                  1052


<210>  24
<211>  1397
<212>  DNA
<213>  Homo sapiens

<223>  mitochondrial carrier triple repeat 1 (MCART1), mRNA

<400>  24
atcaagggtg aagcatggtg gctagcggag cccgcatcct gacaggtgtg ctggcgtggc      60

cctctacctg ccccgccgcg gcggcctgcg gccaggagaa gtaggctggc gacatctggc     120

gggccgcggg ccgagggagg ggaggcgggg cggggcggtg tctccctcac gtgaccccaa 180

cagcgcccag cgcgtcgcgc tgcgccccaa cctcggggtc ggtcacagtc tgcgcctgcg 240

cgggccccgg ctcgctagcc gtcctgcggg acgccggcgc tgatgggaaa tccagttatc 300

aaaattgact caagaagaga gaacctaaca gaacaataac aatggaagaa attgggaaca 360

ttatcacaaa gctatcatcc tgccaaactc caggctcaga tgtcacaggt taaaaaaagt 420

ccttcatgaa aaagaaagat cttaagcagc atgatggatt cagaagctca tgaaaagagg 480

ccaccaatac taacatcttc aaaacaagat atatcacctc atattacaaa tgttggtgag 540

atgaagcatt acttgtgtgg ctgctgtgca gccttcaaca atgtcgcaat cacatttccc 600

attcagaagg tcctctttcg acaacagctg tatggcatca aaacccggga tgcaatactt 660

cagttgagaa gggatggatt tcgaaatttg tatcgtggaa tccttccccc attgatgcag 720

aagacaacta cgcttgcact tatgtttggt ctgtatgagg atttatcctg ccttctccac 780

aagcatgtca gtgctccaga gtttgcaacc agtggcgtgg cggcagtgct tgcaggaaca 840

acagaagcaa ttttcactcc actggaaaga gttcagacat tgcttcaaga ccacaagcat 900

catgacaaat ttaccaacac ttaccaggct ttcaaggcac tgaaatgtca tggaattgga 960

gagtattatc gaggcttggt gcccattctt ttccggaatg gactcagcaa tgtcttgttt 1020

ttcggccttc gaggtcccat taaggagcat ctgcctaccg caacgactca cagtgctcat 1080

ctggtcaatg attttatctg tggaggtcta ttgggtgcca tgttgggatt cttgtttttt 1140

ccaattaatg ttgtaaaaac tcgcatacag tctcagattg gtggggaatt tcagtctttc 1200

cccaaggttt tccaaaaaat ctggctggaa cgggacagaa aactgataaa tcttttcaga 1260

ggtgcccatc tgaattacca tcggtccctc atctcttggg gcataatcaa tgcaacttat 1320

gagttcttgt taaaggttat atgaaaaaac catcagttaa gtgccattta tcaactgaat 1380

agaccttcta agaagaa 1397

<210> 25
<211> 760
<212> DNA
<213> Homo sapiens

<223> P antigen family, member 5 (prostate associated) (PAGE5), transcript
      variant 1, mRNA

<400> 25
cagagctctg caaggagagg ttgtgtcttc gttctttccg ccatcttcgt tctttccaac 60

atcttcgttc tttctcactg accgagactc agccggtagg tctgcagagt ggtcttcctg    120

gtaatttagt tgtgagtgaa tgtgtggagg agccagcggg cttaggacag gtcctgtggc    180

acagtccgtg gctttgaggg aaaagggcct cgcggtggtc ctccgccttc ccccaggtcg    240

tgatgcaggc gccatgggcc ggtaatcgtg gctgggctgg aacgagggag gaagtgagag    300

atatgagtga gcatgtaaca agatcccaat cctcagaaag aggaaatgac caagagtctt    360

cccagccagt tggacctgtg attgtccagc agcccactga ggaaaaacgt caagaagagg    420

aaccaccaac tgataatcag ggtattgcac ctagtgggga gatcaaaaat gaaggagcac    480

ctgctgttca agggactgat gtggaagctt ttcaacagga actggctctg cttaagatag    540

aggatgcacc tggagatggt cctgatgtca gggaggggac tctgcccact tttgatccca    600

ctaaagtgct ggaagcaggt gaagggcaac tataggttta aaccaagaca aatgaagact    660

gaaaccaaga atattgttct tatgctggaa atttgactgc taacattctc ttaataaagt    720

tttacagttt tctgcaaaga aaaaaaaaaa aaaaaaaaaa                          760

<210>  26
<211>  766
<212>  DNA
<213>  Homo sapiens

<223>  ubiquitin-conjugating enzyme E2C (UBE2C), transcript variant 2, mRNA

<400>  26
aaacgcgggc gggcgggccc gcagtcctgc agttgcagtc gtgttctccg agttcctgtc     60

tctctgccaa cgccgcccgg atggcttccc aaaaccgcga cccagccgcc actagcgtcg    120

ccgccgcccg taaaggagct gagccgagcg ggggcgccgc ccggggtccg gtgggcaaaa    180

ggctacagca ggagctgatg accctcatga tgtctggcga taaagggatt tctgccttcc    240

ctgaatcaga caaccttttc aaatgggtag ggaccatcca tggagcagct ggaacagcag    300

tggggagcat cagaaccagc tcaacagttt gtctactgtc cggtcccaga gaaactcaag    360

attctagcaa gccccttgtg tggggcttgg gttgggacat gaggctgctg ctggagctta    420

ctctgcaact gtttctccaa atgccagaac ccaacattga tagtcccttg aacacacatg    480

ctgccgagct ctggaaaaac cccacagctt ttaagaagta cctgcaagaa acctactcaa    540

agcaggtcac cagccaggag ccctgaccca ggctgcccag cctgtccttg tgtcgtcttt    600

ttaatttttc cttagatggt ctgtcctttt tgtgatttct gtataggact ctttatcttg    660

agctgtggta tttttgtttt gtttttgtct tttaaattaa gcctcggttg agcccttgta 720

tattaaataa atgcattttt gtcctttttt agacaaaaaa aaaaaa 766

<210> 27
<211> 1326
<212> DNA
<213> Homo sapiens

<223> ankyrin repeat domain 7 (ANKRD7), transcript variant 2, mRNA

<400> 27
ggccgtaact gccataacgg ccggctgcgc gaggcccttc cagcatttcc actttcgtca 60

ggtactggag agggctgccg gccggatgcc agggcagagg ggcagggcgg acggctagga 120

gttcaagaaa catcctggtc tgagggaaag gctgcagcct gcaccgccat gaataagctt 180

ttcagcttct ggaagaggaa gaatgagacc cgcagccagg gctacaacct tcgagaaaag 240

gatttaaaga aacttcacag agctgcttca gtcggggatt tgaagaagct gaaggaatac 300

cttcagatca agaaatatga tgtaaatatg caggacaaaa aatacagaac acctttgcac 360

ctagcctgtg ctaatggaca tacagatgtt gtacttttcc taattgagca acaatgcaaa 420

ataaatgtcc gggatagtga aaacaaatcc ccattgatta aggcagtaca gtgtcaaaat 480

gaggattgtg ctactattct tctaaacttt ggtgcagacc cagatctgag ggatattcgt 540

tataatactg ttcttcacta tgctgtttgt ggtcaaagtt tgtcattagt tgaaaaactg 600

cttgaatacg aagctgatct tgaagcgaaa aataaggatg ggtatactcc actattagtt 660

gccgttatta acaataatcc aaaaatggta aaatttcttc tggagaaagg ggctgatgtg 720

aatgcttcag ataattatca aagaacagcc cttattcttg ctgtcagtgg tgaaccacca 780

tgtttagtaa agcttcttct tcagcaaggt gtggaattat gttacgaagg tattgtggat 840

tcacagctga ggaatatgtt tatttccatg gttttactgc atagataccc acaattcact 900

gcgagccatg gaaagaagaa acatgctaaa tagacacctt attcttggca ctacatgtga 960

ctaaaggaag atatggaacc catttctaca atttctttgc cgcttccttg aattggaaaa 1020

atgtactttg aaagaaccgt taagtgaact attataatat ttttgctgac tacccagttg 1080

aagaaaagtt tgttaattgg atgggatttt ttttttttca cgttagaaga catgaagaaa 1140

ttttaaaaga taaacatcta tattgtgaac catcagctga aaagataaat ttgtgttcaa 1200

tatataggca gaaaaaaatt tgtgtcaaaa tgttgaatgg aataataatg aggaaactgt 1260

gttaggcatg tattaaaaca tttaaataaa ataaaaatac atttcagcta caaaaaaaaa 1320

aaaaaa 1326

<210> 28
<211> 626
<212> DNA
<213> Homo sapiens

<223> X antigen family, member 1 (XAGE1), transcript variant 1, mRNA

<400> 28
ggacctggga aggagcatag gacagggcaa ggcgggataa ggaggggcac cacagccctt 60

aaggcacgag ggaacctcac tgcgcatgct cctttggtgc ccacctcagt gcgcatgttc 120

actgggcgtc ttcccatcgg ccccttcgcc agtgtgggga acgcggcgga gctgtgagcc 180

ggcgactcgg gtccctgagg tctggattct ttctccgcta ctgagacacg gcggacacac 240

acaaacacag aaccacacag ccagtcccag gagcccagta atggagagcc ccaaaaagaa 300

gaaccagcag ctgaaagtcg ggatcctaca cctgggcagc agacagaaga agatcaggat 360

acagctgaga tcccagtgcg cgacatggaa ggtgatctgc aagagctgca tcagtcaaac 420

accggggata aatctggatt tgggttccgg cgtcaaggtg aagataatac ctaaagagga 480

acactgtaaa atgccagaag caggtgaaga gcaaccacaa gtttaaatga agacaagctg 540

aaacaacgca agctggtttt atattagata tttgacttaa actatctcaa taaagttttg 600

cagctttcac caaaaaaaaa aaaaaa 626

<210> 29
<211> 2922
<212> DNA
<213> Homo sapiens

<223> perilipin (PLIN), mRNA

<400> 29
   agcctgggct ctgtgagact gaggtggcgg tcagccggag tgagtgttgg ggtcctgggg 60
   cacctgcctt acatggcttg tttatgaaca ttaaagggaa gaagttgaag cttgaggagc 120
   gaggatggca gtcaacaaag gcctcacctt gctggatgga gacctccctg agcaggagaa 180
   tgtgctgcag cgggtcctgc agctgccggt ggtgagtggc acctgcgaat gcttccagaa 240
   gacctacacc agcactaagg aagcccaccc cctggtggcc tctgtgtgca atgcctatga 300
   gaagggcgtg cagagcgcca gtagcttggc tgcctggagc atggagccgg tggtccgcag 360
   gctgtccacc cagttcacag ctgccaatga gctggcctgc cgaggcttgg accacctgga 420
   ggaaaagatc cccgccctcc agtacccccc tgaaaagatt gcttctgagc tgaaggacac 480
   catctccacc cgcctccgca gtgccagaaa cagcatcagc gttcccatcg cgagcacttc 540
   agacaaggtc ctgggggccg ctttggccgg gtgcgagctt gcctgggggg tggccagaga 600
   cactgcggaa tttgctgcca acactcgagc tggccgactg gcttctggag gggccgactt 660
   ggccttgggc agcattgaga aggtggtgga gtacctcctc cctgcagaca aggaagagtc 720
   agcccctgct cctggacacc agcaagccca gaagtctccc aaggccaagc caagcctctt 780
   gagcagggtt ggggctctga ccaacaccct ctctcgatac accgtgcaga ccatggcccg 840
   ggccctggag cagggccaca ccgtggccat gtggatccca ggcgtggtgc ccctgagcag 900
   cctggcccag tggggtgcct cagtggccat gcaggcggtg tcccggcgga ggagcgaagt 960
   gcgggtaccc tggctgcaca gcctcgcagc cgcccaggag gaggatcatg aggaccagac 1020
   agacacggag ggagaggaca cggaggagga ggaagaattg gagactgagg agaacaagtt 1080
   cagtgaggta gcagccctgc caggccctcg aggcctcctg ggtggtgtgg cacataccct 1140
   gcagaagacc ctccagacca ccatctcggc tgtgacatgg gcacctgcag ctgtgctggg 1200
   catggcaggg agggtgctgc acctcacacc agcccctgct gtctcctcaa ccaaggggag 1260
   ggccatgtcc ctatcagatg ccctgaaggg cgttactgac aacgtggtgg acacagtggt 1320
   gcattacgtg ccgctcccca ggctgtcgct gatggagccc gagagcgaat tccgggacat 1380
   cgacaaccca ccagccgagg tcgagcgccg ggaggcggag cgcagagcgt ctggggcgcc 1440
   gtccgccggc ccggagcccg ccccgcgtct cgcacagccc cgccgcagcc tgcgcagcgc 1500
   gcagagcccc ggcgcgcccc ccggcccggg cctggaggac gaagtcgcca cgcccgcagc 1560
   gccgcgcccg ggcttcccgg ccgtgccccg cgagaagcca aagcgcaggg tcagcgacag 1620
   cttcttccgg cccagcgtca tggagcccat cctgggccgc acgcattaca gccagctgcg 1680
   caagaagagc tgagtcgccg caccagccgc cgcgccccgg gccggcgggt ttctctaaca 1740
   aataaacaga acccgcactg cccaggcgag cgttgccact ttcaaagtgg tcccctgggg 1800
   agctcagcct catcctgatg atgctgccaa ggcgcacttt ttatttttat tttattttta 1860
   tttttttttt agcatccttt tggggcttca ctctcagagc cagtttttaa gggacaccag 1920
   agccgcagcc tgctctgatt ctatggcttg gttgttacta taagagtaat tgcctaactt 1980
   gatttttcat ctctttaacc aaacttgtgg ccaaaagata tttgaccgtt tccaaaattc 2040
   agattctgcc tctgcggata aatatttgcc acgaatgagt aactcctgtc accactctga 2100
   aggtccagac agaaggtttt gacacattct tagcactgaa ctcctctgtg atctaggatg 2160
   atctgttccc cctctgatga acatcctctg atgatctagg ctcccagcag gctactttga 2220
   agggaacaat cagatgcaaa agctcttggg tgtttattta aaatactagt gtcactttct 2280
   gagtacccgc cgcttcacag gctgagtcca ggcctgtgtg ctttgtagag ccagctgctt 2340
   gctcacagcc acatttccat ttgcatcatt actgccttca cctgcatagt cactcttttg 2400
   atgctgggga accaaaatgg tgatgatata tagactttat gtatagccac agttcatccc 2460
   caaccctagt cttcgaaatg ttaatatttg ataaatctag aaaatgcatt catacaatta 2520
   cagaattcaa atattgcaaa aggatgtgtg tctttctccc cgagctcccc tgttcccctt 2580
   cattgaaaac caccacggtg ccatctcttg tgtatgcagg gctatgcacc tgcaggcacg 2640
   tgtgtatgca ctccccgctt gtgtttacac aagctgtggg gtgttacgca tgcctgcttt 2700
   tttcacttaa taatacagct tggagagatt tttgtatcac attataaatc ccactcgctc 2760
   tttttgatgg ccacataata actactgcat aatatggata cgccttattt gatttaacta 2820
   gttccctaat gatggacttt taagttgttt cctttttttt tcttttttgc tactgcaaac 2880
   gatgctataa taaatgtcct tatcaaaaaa aaaaaaaaaa aa 2922

<210> 30
<211> 1626
<212> DNA
<213> Homo sapiens

<223> melanoma antigen family B, 2 (MAGEB2), mRNA

<400> 30
acttccgctt tggaggcgag gacccgagcg agtgtagggg gtgcggcgtc tggtcagcca 60

ggggtgaatt ctcaggactg gtcggcagtc aagccatcat gcctcgtggt cagaagagta 120

agctccgtgc ccgtgagaaa cgccgcaagg cccgagatga gacccggggt ctcaatgttc 180

ctcaggtcac tgaagcagag gaagaagagg ccccctgctg ttcctcttct gtttctgggg 240

gtgctgcttc aagctctcct gctgctggca ttccccagaa gcctcagaga gccccaacca 300

ctgccgctgc tgcggctgcg ggtgtttcat ccacaaaatc taaaaaaggt gccaagagcc 360

accaaggtga gaaaaatgca agttcctccc aggcctcaac atctactaag agcccaagcg 420

aagatcctct aaccaggaag tcagggtcgt tggtgcagtt cctgttgtac aagtataaaa 480

taaaaaagtc cgttacaaag ggagaaatgc tgaaaattgt tggcaaaagg ttcagggagc 540

acttccctga gatcctcaag aaagcctctg agggcctcag tgttgtcttt ggccttgagc 600

tgaataaagt caaccccaac ggccacactt acaccttcat cgacaaggta gacctcactg 660

atgaggaatc cctgctcagt tcctgggact ttcccaggag aaagcttctg atgcctctcc 720

tgggtgtgat cttcttaaat ggcaactcag ctactgagga agagatctgg gaattcctga 780

atatgttggg agtctatgat ggagaggagc actcagtctt tggggaaccc tggaagctca 840

tcaccaaaga tctggtgcag gaaaaatatc tggagtacaa gcaggtgccc agcagtgatc 900

ccccacgctt tcaattcctg tggggtccga gagcctatgc tgaaaccagc aagatgaaag 960

tcctggagtt tttggccaag gtaaatggta ccaccccctg tgccttccca acccattacg 1020

aagaagcttt gaaagatgaa gagaaagccg gagtctgagc cagagttgta gccaggcctt 1080

gcactactgc catagccaat caatctccca aagccaagtt tacctgctgt tctcaccccc 1140

aatgaggtct taggcagatt ctttactttg taattcaaaa ggcctgttaa cctttgttct 1200

tgttatgcat gaataacttg ttgacttttt ttttttctct ttttcaacta gtgtttcaac 1260

aggtttattt agattcagaa tgtaaattta caaatgatat agatcaccct gttattgctg 1320

tttttcaggg acagtagaaa gtgttttgtt ttttgagtga aacaacttat taataaaaat 1380

ccttaaatca cttttgtaat ccaggacaag aaaatgtggc attagagtag aaatatcttt 1440

ggaaatgtga aagaccccat agtgaaatat ttgggatcag aagccagagg ggtaaaagtg 1500

gtcaattctt ggtttacttc atttaatctt tcttttcata aagatacata cctggatttg 1560

tttatgttat tcaagaatgt gtgagaaatt aaaccatagt tagttaaaaa aaaaaaaaaa 1620

aaaaaa 1626

<210> 31
<211> 1273
<212> DNA
<213> Homo sapiens

<223> chromosome 11 open reading frame 70 (C11orf70), mRNA

<400> 31
acgatggcta ctggggagct cggggacttg ggtggctact acttcaggtt cttgcctcag 60

aaaaccttcc agtctctgag ctctaaggag atcaccagcc ggctccgcca gtggtgagga 120

accgaggcgc agggagagaa gaggcccttg gtcttcgcgg ctgtggaggc gcaggccggg 180

cgtcgagggg cccgcgcggg tcggcgccat tctgagtgaa ccctgaggct tcctgagtga 240

accccgaacc acccgggaag ggcggatgct gtgggtggga tcggttatcg gtgcgctctg 300

agagggtgcc agcccccaga tgatttcttt ttcctcctct gccccaggtc catgctgggc 360

agaatcaagg cgcaggcgtt cggctttgac cagacctttc agtcctatcg gaaggatgat 420

ttcgttatgg cttttttcaa agacccaaat gttattccca atttgaagtt actttcagat 480

tcttctggac aatggatcat attaggaact gaagtgaaaa aaattgaagc tataaatgtt 540

ccttgcacac agctttcaat gtcatttttt catcggttat atgatgaaga tattgtacga 600

gacagtggac atattgttaa atgtttagat tctttttgtg atccatttct catttctgat 660

gagttacgaa gagttttgct agtggaagac tcagaaaaat atgaaatatt cagccaacca 720

gatagagaag agttcctgtt ttgtcttttc aaacatcttt gccttggtgg agccctttgt 780

caatatgagg atgtgattag cccatatctg gaaacaacaa agcttatcta taaggatctg 840

gtgagtgttc gaaagaatcc tcaaaccaag aaaatacaga ttacctcttc tgtctttaaa 900

gtttcagctt atgattctgc tggtatgtgc tatccttcag caaagaatca tgaacagaca 960

ttttcttact ttattgtgga tcctatcagg cgtcaccttc atgttttata ccactgttat 1020

ggtgtgggag acatgtctta atgttctttc agattatgta cctctactat tttgtattta 1080

tcatttttct atcttaatac taacttatag ataaacatat actttgcaaa ttaattcaag 1140

aaaaatgtaa ggagcctact tagagcagaa gaaagcaaac accaagatgc ctttttaaaa 1200

atttgtgtgg aatactaacc ggggatcaaa tttcatccat aataaaatct ataccaaaaa 1260

aaaaaaaaaa aaa 1273

<210> 32
<211> 735
<212> DNA
<213> Homo sapiens

<223> ribosomal protein L39-like (RPL39L), mRNA

<400> 32
cggcggttga attgctgcgc ccagcgaggc aaccgcctcc gaacgccagg tgggggcgag 60

gcgtctcgga gtctcagaga caccaaggcc cctgcgacaa ggtggctgca gctaggccgg 120

gggcgtcagg acgacggagc gggttcgggt cggtgacacg cagacctgag ggagctgggc 180

ccgccttttc cgcccgcgcc ccaggccctt gcagatcgag atttgcgtcc tagagtggga 240

aaaaagcaga ggccagggcg ccgattttat ttggagagaa gcaagcatct ttgcctcttt 300

ggagtaggaa attcagactt gaaaaagtgg tgtgtggttg actctgtttc tcgccatgtc 360

ttctcacaag actttcacca ttaagcgatt cctggccaag aaacaaaagc aaaatcgtcc 420

catcccccag tggattcaga tgaaacctgg tagtaaaatc aggtacaact ccaaaaggag 480

gcattggaga agaaccaagc tgggtctata aggaattgca catgagatgg cacacatatt 540

tatgctgtat caagttcacg atcatcttac gatatcaagc tgaaaatgtc accactacct 600

ggacagttgc acatgtttta ctgggaatat ttttttctgt ttttctgtat gctctgtgct 660

agtagggtgg attcagtaat aaatatgtga aagcttttgt ttccaaaaaa aaaaaaaaaa 720

aaaaaaaaaa aaaaa 735

<210> 33
<211> 1284
<212> DNA
<213> Homo sapiens

<223> calcium binding tyrosine-(Y)-phosphorylation regulated (fi-
brousheathin 2) (CABYR), transcript variant 3, mRNA

<400> 33
cctatggctc cgaagtcccc gctggcatcc tgaaagttga gttacagaca tcctgccaaa 60

atgatttctt caaagcccag acttgtcgta ccctatggcc tcaagactct gctcgaggga 120

attagcagag ctgttctcaa aaccaaccca tcaaacatca accagtttgc agcagcttat 180

tttcaagaac ttactatgta tagagggaat actactatgg atataaaaga tctggttaaa 240

caatttcatc agattaaagt agagaaatgg tcagaaggaa cgacaccaca gaagaaatta 300

gaatgtttaa aagaaccagg aaaaacatct gtagaatcta aagtacctac ccagatggaa 360

aaatctacag acacagacga ggacaatgta accagaacag aatatagtga caaaaccacc 420

cagtttccat cagtttatgc tgtgccaggc actgagcaaa cggaagcagt tggtggtctt 480

tcttccaaac cagccacccc taagactact accccaccct catcaccacc tccaacagct 540

gtctcaccag agtttgccta cgtcccagct gacccagctc agcttgctgc tcagatgtta 600

gcaatggcaa caagtgaacg aggacaacca ccaccatgtt ctaacatgtg gaccctttat 660

tgtctaactg ataagaatca acaaggtcac ccatcaccgc cacctgcacc tgggcctttt 720

ccccaagcaa ccctctattt acctaatcct aaggatccac agtttcagca gcatccacca 780

aaagtcactt ttccaactta tgtgatgggc gacaccaaga agaccagtgc ccccaccttt 840

atcttagtag gctcaaatgt tcaggaagca cagggatgga aacctcttcc tggacatgct 900

gtcgtttcac agtcagatgt cttgagatat gttgcaatgc aagtgcccat tgctgttcct 960

gcagatgaga aataccagaa acatacccta agtccccaga atgctaatcc tccaagtgga 1020

caagatgtcc ccaggccaaa aagccctgtt ttcctttctg ttgctttccc agtagaagat 1080

gtagctaaaa aaagttcagg atctggtgac aaatgtgctc cctttggaag ttacggtatt 1140

gctggggagg taaccgtgac tactgctcac aaacgtcgca aagcagaaac tgaaaactga 1200

tccagaaatg acgctgtctg ggtcaacatt tcagggagga gtctgccacc agtgtaatgt 1260

atcaataaac ttcatgcaag cata 1284

<210> 34
<211> 1625
<212> DNA
<213> Homo sapiens

<223> leucine rich repeat containing 6 (LRRC6), mRNA

<400> 34
gcaggggtga agggtgaaaa gcgtcattcg aggtccgggt ccggcttgcg gggtcagcga 60

actggagagg cgccatgggc tggatcacag aagatcttat tagacggaat gctgaacaca 120

acgactgtgt cattttttcc ctggaggaac tctcgttgca tcagcaagaa atagaaagac 180

tagaacacat tgataaatgg tgccgggatt taaaaattct ctatcttcaa aataatctta 240

ttgggaaaat tgaaaatgtt agcaaactca agaaacttga atatttgaat ttagctttaa 300

acaacattga aaaaatagaa aacttggaag gatgtgaaga gctggcaaaa cttgacctga 360

ctgtgaattt cattggagag ctgagcagca ttaaaaactt gcagcacaat atccatctga 420

aggagctctt tctcatgggg aacccatgtg cttcctttga ccactatagg gagttcgtgg 480

tagcaactct tccacaatta aagtggttgg atggtaaaga aatagagcct tcagaaagga 540

ttaaggcatt gcaggactat tcagtaattg aaccacaaat cagagagcag gaaaaagatc 600

actgtcttaa acgagccaaa ctcaaggaag aggctcagag gaaacaccaa gaagaggata 660

aaaatgaaga caagagaagt aacgcaggct ttgatggacg ttggtacaca gacatcaatg 720

ctactctttc ctctttagag agcaaagacc acctacaggc accagacaca gaggaacaca 780

acacaaagaa attagacaac agtgaagatg acttggaatt ctggaataag ccctgtttgt 840

ttactcctga atcaagattg gaaactctta gacacatgga aaaacaacgg aagaaacagg 900

aaaaattaag tgaaaaaaag aagaaagtga aaccacccag gactttgatc actgaagatg 960

ggaaagccct aaatgtgaat gagcccaaaa ttgacttctc tttgaaagat aacgaaaagc 1020

agatcatcct ggaccttgct gtctataggt atatggatac ctctttaatc gatgttgatg 1080

tgcaaccaac ttacgtgcga gtaatgatca aaggaaagcc atttcagctt gtccttcctg 1140

cagaagtgaa acccgatagt agttctgcta aaagatctca gacaacgggt catttggtca 1200

tctgcatgcc caaggtagga gaagtaatca caggtggtca gcgagcattc aaatctatga 1260

aaactacctc ggacaggagc agagaacaaa caaatacaag aagcaagcac atggagaaac 1320

tagaagtaga ccctagcaag cactcattcc ctgatgtgac taacatagtt caagagaaaa 1380

aacacacacc cagaagacga cctgaaccca aaattatacc aagtgaggaa gacccaacct 1440

ttgaagacaa ccctgaagtg cctccgctga tttgaaacat ctggctgcgt tgccattggc 1500

tgagacccac caggtccagt tttggttggt gtagagacca tatgcatatt attcctggga 1560

taaacacaga gttatttgtc aaaaaaaaaa aaaaaaaaaa aaaaaaaaaa aaaaaaaaaa 1620

aaaaa 1625

<210> 35
<211> 627
<212> DNA
<213> Homo sapiens

<223> CDC28 protein kinase regulatory subunit 2 (CKS2), mRNA

<400> 35
agtctccggc gagttgttgc ctgggctgga cgtggttttg tctgctgcgc ccgctcttcg 60

cgctctcgtt tcattttctg cagcgcgcca cgaggatggc ccacaagcag atctactact 120

cggacaagta cttcgacgaa cactacgagt accggcatgt tatgttaccc agagaacttt 180

ccaaacaagt acctaaaact catctgatgt ctgaagagga gtggaggaga cttggtgtcc 240

aacagagtct aggctgggtt cattacatga ttcatgagcc agaaccacat attcttctct 300

ttagacgacc tcttccaaaa gatcaacaaa aatgaagttt atctggggat cgtcaaatct 360

tttcaaattt taatgtatat gtgtatataa ggtagtattc agtgaatact tgagaaatgt 420

acaaatcttt catccatacc tgtgcatgag ctgtattctt cacagcaaca gagctcagtt 480

aaatgcaact gcaagtaggt tactgtaaga tgtttaagat aaaagttctt ccagtcagtt 540

tttctcttaa gtgcctgttt gagtttactg aaacagttta cttttgttca ataaagtttg 600

tatgttgcat ttaaaaaaaa aaaaaaa 627

<210> 36
<211> 879
<212> DNA
<213> Homo sapiens

<223> chromosome 1 open reading frame 61 (C1orf61), mRNA

<400> 36
ctcctcacag aagcctggag ctgggcatcc aagaagaagc agcctcattt gttttctggt 60

gtcatcgtag gtggccacct atggcttttg ggaatgtaaa aagggcagct ctctggcatg 120

ttcctgactg aggatctcat aacatttaac ttgaggaact tcctcctttt ccagctttgg 180

gagtcaagct tctcacctgg ggcgggtggg ttctgcacca ccctcccacc ctccttcctc 240

cgtgtggacg atagagccac atccagcacc acggacagct cccgggcgcc ttcatctcct 300

cgtcctccag gcagcacaag ccattgtgga atctccacca ggtgtacaga acggtgcctc 360

tgcgtcctgc cactcaggac ctctcaagtc cccgatgtga tggctcctca gcatgatcag 420

gagaaattcc atgatcttgc ttattcctgt cttgggaagt ccttctccat gtctaaccaa 480

gatctatatg gctatagcac cagctctttg gctcttggct tggcatggct aagttgggag 540

accaaaaaga agaatgtact tcatctggtt gggctggatt ccctctgata agccttccca 600

gttgactgaa agatgaggct aggctctagc aagttgaagt caaaccagct ccttcaagaa 660

gctttgagca gaatgaagtg gggaggaccc agcttccagc ccaggaagcc cactgtacct 720

ggagccatct gggataagac tttgacccat gactcccata tccacagcct gtccatccta 780

gcccatccca gtttatcctg tatcatttga gctgggattc ccacatcctc tgagttggaa 840

gtcccatctc aagtcttcaa taaagactct tgaatattg 879

<210> 37
<211> 2867
<212> DNA
<213> Homo sapiens

<223>  A kinase (PRKA) anchor protein 4 (AKAP4), transcript variant 2, mRNA

<400>  37
caggggtggc agccaactgc aggtgcccaa gaacttggca cttctcagtt ccatctaaag      60

gggcacatct cccttctggg tgtcacgttt tcagccaaac atctaaaaga acttcatcat     120

caagatgtct gatgatattg actggttacg cagccacagg ggtgtgtgca aggtagatct     180

ctacaaccca gaaggacagc aagatcagga ccggaaagtg atatgctttg tcgatgtgtc     240

caccctgaat gtagaagata aagattacaa ggatgctgct agttccagct cagaaggcaa     300

cttaaacctg ggaagtctgg aagaaaaaga gattatcgtg atcaaggaca ctgagaagaa     360

agaccagtct aagacagagg gatctgtatg ccttttcaaa caagctccct ctgatcctgt     420

aagtgtcctc aactggcttc tcagtgatct ccagaagtat gccttgggtt tccaacatgc     480

actgagcccc tcaacctcta cctgtaaaca taaagtagga gacacagagg gcgaatatca     540

cagagcatcc tctgagaact gctacagtgt ctatgccgat caagtgaaca tagattattt     600

gatgaacaga cctcaaaacc tacgtctaga aatgacagca gctaaaaaca ccaacaataa     660

tcaaagtcct tcagctcctc cagccaaacc tcctagcact cagagagcag tcatttcccc     720

tgatggagaa tgttctatag atgacctttc cttctacgtc aaccgactat cttctctggt     780

aatccagatg gcccataagg aaatcaagga gaagttggaa ggtaaaagca aatgccttca     840

tcattcaatc tgtccatccc ctgggaacaa agagagaatc agtccccgaa ctcctgcgag     900

caagattgct tctgaaatgg cctatgaagc tgtggaactg acagctgcag aaatgcgtgg     960

cactggagag gagtccaggg aaggtggcca gaaaagcttt ctatatagcg aattatccaa    1020

caagagcaaa agtggagaca aacagatgtc ccagagagag agcaaagaat ttgcagattc    1080

catcagcaag gggctcatgg tttatgcaaa tcaggtggca tctgacatga tggtctctct    1140

catgaagacc ttgaaagtgc acagctctgg gaagccaatt ccagcatctg tggtcctgaa    1200

gagggtgttg ctaaggcaca ccaaggagat tgtgtccgat ttgattgatt cttgcatgaa    1260

gaacctgcat aatattactg gggtcctgat gactgactca gactttgtct cagctgtcaa    1320

gagaaatctg ttcaaccagt ggaaacaaaa tgctacagac atcatggagg ccatgctgaa    1380

gcgcttggtc agtgccctta taggtgagga gaaggagact aagtctcaga gtctgtcata    1440

tgcatcttta aaagctgggt cccatgatcc caaatgcagg aatcagagtc ttgaattctc    1500

caccatgaaa gctgaaatga aagagaggga caaaggcaaa atgaaatcag acccatgcaa    1560

gtcactgact agtgctgaga aagtcggtga acacattctc aaagagggcc taaccatctg    1620

gaaccaaaag caaggaaact catgcaaggt ggctaccaaa gcatgcagca ataaagatga 1680

gaaaggagaa aagatcaatg cttccacaga ttcactggcc aaggacctga ttgtctctgc 1740

ccttaagctg atccagtacc atctgaccca gcagactaag ggcaaagata catgtgaaga 1800

agactgtcct ggttccacca tgggctatat ggctcagagt actcaatatg aaaagtgtgg 1860

aggtggccaa agtgccaaag cactttcagt gaaacaacta gaatctcaca gagcccctgg 1920

accatccacc tgtcaaaagg agaaccaaca cctggactcc cagaaaatgg atatgtcaaa 1980

catcgttcta atgctgattc agaaactgct taatgagaac cccttcaaat gtgaggatcc 2040

atgcgaaggt gagaacaagt gttctgagcc cagggcaagc aaagcagctt ccatgtccaa 2100

cagatctgac aaagcggaag aacaatgcca ggagcatcaa gaacttgact gtaccagtgg 2160

gatgaagcaa gcgaacgggc aatttataga taaactagta gaatctgtga tgaagctctg 2220

ccttatcatg gctaagtata gcaacgatgg ggcagccctt gctgagttgg aagaacaagc 2280

agcctcggca aataagccca atttcagggg caccagatgc attcacagtg gtgcaatgcc 2340

acagaactat caagactctc ttggacatga agtaattgtc aataatcagt gctctacaaa 2400

tagcttgcag aagcagctcc aggctgtcct gcagtggatt gcagcctccc agtttaacgt 2460

gcccatgctc tacttcatgg gagataagga tggacaactg gaaaagcttc ctcaggtttc 2520

agctaaagca gcagagaagg ggtacagtgt aggaggtctt cttcaagagg tcatgaagtt 2580

tgccaaggaa cggcaaccag atgaagctgt gggaaaggtg gccaggaaac agttgctgga 2640

ctggctgctc gctaacctgt gagctgatcc ttgactcctc ttcatcttag cccccctagc 2700

agcattccat cccagccaga gcacccccac catcaggcca gtcaactgca caatacacaa 2760

ctgtatttcc caatacactt gagcagttgc ctgtgaatgt aagaggtgtc aacaaactgg 2820

gaaataaaat aaaaaaaaat aataaaaaaa aaaaaaaaaa aaaaaaa 2867

<210> 38
<211> 3482
<212> DNA
<213> Homo sapiens

<223> sapiens EH-domain containing 1 (EHD1), mRNA

<400> 38
ccctaccgcc cccaattccg ccctgccccc gccgcggcgg cgctagccgc cactgaggga 60

ccgaccctat aaaggccgct ccgcgagggg tgcgcagcat tcggcagagg gcgcttcgac 120

gggctgggct gtgcgcctgc gcagtgtggg tcgctcccga ttccctgccc cggccggccc 180

cgcctcggct ccgcaccctc gccccgctct cagccgccgc tctgccccgc agcagccagc 240

cccgtgtccg gcagtatgtt cagctgggtc agcaaggatg cccgccgcaa gaaggagccg 300

gagctcttcc agacggtggc tgaggggctg cggcagctgt acgcgcagaa gctgctaccc 360

ctggaggagc actaccgctt ccacgagttc cactcgcccg cgctggagga cgctgacttc 420

gacaacaagc ctatggtgct cctcgtgggg cagtacagca cgggcaagac caccttcatc 480

cgacacctga tcgagcagga cttcccgggg atgcgcatcg ggcccgagcc caccaccgac 540

tccttcatcg ccgtcatgca cggccccact gagggcgtgg tgccgggcaa cgcgctcgtg 600

gtggacccgc ggcgcccctt ccgcaagctc aacgcgtttg gcaacgcttt cctcaacagg 660

ttcatgtgtg cccagctgcc caaccccgtc ctggacagca tcagcatcat cgacaccccc 720

gggatcctgt ctggagagaa gcagcggatc agcagaggct atgactttgc agccgtcctg 780

gagtggttcg cggagcgtgt ggaccgcatc atcctgctct tcgacgccca caagctggac 840

atctccgatg agttctcgga agtgatcaag gctctgaaga accatgagga caagatccgc 900

gtggtgctga acaaggcaga ccagatcgag acgcagcagc tgatgcgggt gtacggggcc 960

ctcatgtggt ccctgggcaa gatcatcaac acccccgagg tggtcagggt ctacatcggc 1020

tccttctggt cccacccgct cctcatcccc gacaaccgca agctctttga ggccgaggag 1080

caggacctct tcaaggacat ccagtcactg ccccgaaacg ccgccctcag gaagctcaat 1140

gacctgatca agcgggcacg gctggccaag gttcacgcct acatcatcag ctccctcaag 1200

aaagagatgc ccaatgtctt tggtaaagag agcaaaaaga aagagctggt gaacaacctg 1260

ggagagatct accagaagat tgagcgcgag caccagatct cccctgggga cttcccgagc 1320

ctccgcaaga tgcaggaact cctgcagacc caggacttca gcaagttcca ggcgctgaag 1380

cccaagctgc tggacacggt ggatgacatg ctggccaacg acatcgcgcg gctgatggtg 1440

atggtgcggc aggaggagtc cctgatgcct tcccaggtgg tcaagggcgg cgcctttgac 1500

ggcaccatga acgggccgtt cgggcacggc tacggcgagg gggccggcga gggcatcgac 1560

gacgtggagt gggtggtggg caaggacaag cccacctacg acgagatctt ctacacgctg 1620

tcccctgtca acggcaagat cacgggcgcc aacgccaaga aggagatggt gaagtccaag 1680

ctccccaaca ccgtgctagg gaagatctgg aagctggccg acgtggacaa ggacgggctg 1740

ctggacgacg aggagttcgc gctggccaac cacctcatca aggtcaagct ggagggccac 1800

gagctgcccg ccgacctgcc cccgcacctg gtgccgccct ccaagcgcag acatgagtga 1860

tggcgcccgg ccccgcacct gccatttgca cgcccggccg ggaggcagag acggggggag 1920

gggaagcctc accatttctc aaggtccata aagactgagc ggatgtttcc tcgcctctcg 1980

aaaaggaaaa ccaccatctt tcttttaagg ctgttcctgg gcctggcggg ggaggcaggg 2040

gtgagaggat ggaattgtgt gcacaagaac tgtggctatt ttaatatata acgttagagg 2100

ctgcgttctt tgtcgccgcc tcccctgtgt gccagccctg tgtgcacggc ctctgccccc 2160

cggcctttgc tgtggctgga gctggacagt gcagcgactg cgaccgtggg ggagccaggt 2220

cgcccttttg gcagctgcta ggctgaggct gcatggacag gaacaccagg caccctccgt 2280

gtgcttctga gctgaggttg cttcacggga ccgtggcttc cttcctcacc tggctctgcc 2340

tcccccgtgc tctcgggcga agtgggttct tgtgccttcc cctcccgggc ccaggctccc 2400

cgtgcgcggg ccctgccctt tcctcccgcg ccccaccggc tccgacgcgc aaccccgctc 2460

agcagtcaca gaagcagggc ccagccacct tggtcttttt ttgggagttc aggggagtag 2520

gagaatgtct tccagaaaaa tacataagct agtttctgtt ctgtaaagtg atatctttca 2580

tacttgacca aagttcccaa taacttccca accactgttc aaaagctgtg atttttgtct 2640

ccccttccca ccctccagcc aaggagcagc cctgcccagg gggcattagg tgtgggtacc 2700

cggggagcac cccgttcctg gaccccagtg ttgcatttcc tggctgagga agggtggtca 2760

tcccagctcc tgccctaccc tctcacttaa ctggagcttt gggacgcacc ctccacagtg 2820

ggaggtggtg gtgggtggcg gtggcggggc ctcacgacag cttggtgctg gtaagaggaa 2880

gcccgtggtt ctggctaggc tctcatgtcc agacagcggg gaccagggga aaacccagcc 2940

ccttctgtaa tcccccttca tttcctacct tccttcctcc tctgtttagc aaaggagggc 3000

agctcacttg gatgtcctta caacgcccct ggcccccagg ttgagcaata agaaaccaga 3060

accttgcggc ccagtggccc gggccagttc aggccgcctc cccctcctct gcctggggcc 3120

attgagccca gcctccaggg cccgggcgcg tttgcaggcc agtggccact gtccgggctg 3180

tgatggcacc aaggcaggtg gagcaccagg taccacacag ctgggcttcc caccaggctt 3240

tcccgcgggg gtctcaggga gcttctcccc agcgctgctc ggagtctgca ggaactggcc 3300

ttgttctcct tagcccgtca ctccatacag tattaggtga ggatggatgc gggcgctgtc 3360

cttgccggga agtcactgtt gaagttgcag tggcttgttc acacctgtgg gaagagaagt 3420

gaagactttc tccttgcatt aaaaagtctg aactgtgaaa aaaaaaaaaa aaaaaaaaaa 3480

aa 3482

<210> 39
<211> 1196
<212> DNA
<213> Homo sapiens

<223> uncharacterized hematopoietic stem/progenitor cells protein MDS028
      mRNA, complete cds

<400> 39
ttttttttta caaagaaggt ataactttaa taaccctatt tacagataag aaaaccaaga 60

ctcagaagtt aaatggaaag gtgaggtctc tgaactaatc cagactctcc catgaggttc 120

ctttggcaag tcctgggctt ctgtcctcat ctgcaaaatc gaaagcattc ctgaggtttc 180

ttccagctct gcatcatcag agttgggaag cccgcctcca agcccggaag acccgcctcc 240

tctgtaaggt taaaggagga agaggcagag attggaggtt caggcagtga cgtaacttgc 300

tgccttaggt ggccttccgc tctggcggct gtcgcgacgg gggttcaggg aatatttact 360

gggcctctcc gctccctctg ctcttggagg tgccatgagg tcagttagct acgtgcagcg 420

cgtggcgctg gagttcagcg ggagcctctt cccgcacgca atctgcctcg gagacgttga 480

taacgatacg ttaaatgaac tggtggtggg agacaccagc gggaaggtgt ctgtgtataa 540

aaatgatgac agtcggccat ggctcacctg ttcctgccag ggaatgctga cttgcgttgg 600

ggttggagac gtgtgtaata aaggaaagaa cctgttggtg gcagtgagtg ctgaaggctg 660

gtttcatttg tttgacctga cacctgccaa ggtgttggat gcttctgggc accacgagac 720

actaatcgga gaggagcagc gtccagtctt caagcagcac atccctgcca acaccaaggt 780

catgctgatc agcgacatcg atggagatgg gtgtcgtgag ctggtggtgg gctacacaga 840

ccgtgtggtg cgagctttcc gctgggagga gctaggtgag ggtcctgaac atctgacagg 900

gcagctggtg tccctcaaga aatggatgct ggagggtcac catgataagc caggcatcac 960

tcaaggcact ggaattgcca tagtgaataa atagacaaaa attgctgccc tcctgggcct 1020

tattatgaag aaacagaaaa gaaacaatat aggctaggtg tgctgcctct cgcctgtgat 1080

accaacgctt tgggaggctg agatgggagg atcacttgag gccaggagtt ccaggctgta 1140

gtgagctatg atctcaccac tgcactccac tccagcgtgg gaccctgtct ctattt 1196

<210>  40
<211>  1440
<212>  DNA
<213>  Homo sapiens

<223>  tumor necrosis factor, alpha-induced protein 6 (TNFAIP6), mRNA

<400>  40
cagtcacatt tcagccactg ctctgagaat ttgtgagcag cccctaacag gctgttactt      60

cactacaact gacgatatga tcatcttaat ttacttattt ctcttgctat gggaagacac     120

tcaaggatgg ggattcaagg atggaatttt tcataactcc atatggcttg aacgagcagc     180

cggtgtgtac cacagagaag cacggtctgg caaatacaag ctcacctacg cagaagctaa     240

ggcggtgtgt gaatttgaag gcggccatct cgcaacttac aagcagctag aggcagccag     300

aaaaattgga tttcatgtct gtgctgctgg atggatggct aagggcagag ttggataccc     360

cattgtgaag ccagggccca actgtggatt tggaaaaact ggcattattg attatggaat     420

ccgtctcaat aggagtgaaa gatgggatgc ctattgctac aacccacacg caaaggagtg     480

tggtggcgtc tttacagatc caaagcaaat ttttaaatct ccaggcttcc caaatgagta     540

cgaagataac caaatctgct actggcacat tagactcaag tatggtcagc gtattcacct     600

gagtttttta gattttgacc ttgaagatga cccaggttgc ttggctgatt atgttgaaat     660

atatgacagt tacgatgatg tccatggctt tgtgggaaga tactgtggag atgagcttcc     720

agatgacatc atcagtacag gaaatgtcat gaccttgaag tttctaagtg atgcttcagt     780

gacagctgga ggtttccaaa tcaaatatgt tgcaatggat cctgtatcca aatccagtca     840

aggaaaaaat acaagtacta cttctactgg aaataaaaac tttttagctg gaagatttag     900

ccacttataa aaaaaaaaaa aaggatgatc aaaacacaca gtgtttatgt tggaatcttt     960

tggaactcct ttgatctcac tgttattatt aacatttatt tattattttt ctaaatgtga    1020

aagcaataca taatttaggg aaaattggaa aatataggaa actttaaacg agaaaatgaa    1080

acctctcata atcccactgc atagaaataa caagcgttaa cattttcata tttttttctt    1140

tcagtcattt ttctatttgt ggtatatgta tatatgtacc tatatgtatt tgcatttgaa    1200

attttggaat cctgctctat gtacagtttt gtattatact ttttaaatct tgaactttat    1260

aaacattttc tgaaatcatt gattattcta caaaaacatg attttaaaca gctgtaaaat    1320

attctatgat atgaatgttt tatgcattat ttaagcctgt ctctattgtt ggaatttcag    1380

gtcattttca taaatattgt tgcaataaat atccttgaac acaaaaaaaa aaaaaaaaaa    1440

<210>  41
<211>  1812
<212>  DNA
<213>  Homo sapiens

<223>  testis-specific kinase substrate (TSKS), mRNA

<400>  41
acccccacac catggcgagc gtggtggtga agacgatctg gcagtccaaa gagatccatg      60

aggccgggga cacccccacg ggggtggaga gctgctccca gctagtccca gaggctcccc     120

ggagggtgac cagccgggcc aaggggatcc cgaagaaaaa gaaggccgtg tcgttccacg     180

gggtggagcc ccagatgtcc catcagccca tgcactggtg cctgaacctc aaacggtcct     240

cggcctgcac caacgtgtca ctgctcaacc tggccgccat ggagcccact gactccacgg     300

ggacagactc cacagtggaa gacctcagcg gccaactcac actggctggg ccccctgcct     360

cccctaccct accctgggat ccggatgacg cagacatcac ggaaatcctg agtggggtca     420

acagtggatt ggtccgcgcc aaagactcca tcaccagctt gaaggaaaag accaaccggg     480

ttaaccagca cgtgcagtct ctgcagagcg agtgttctgt gctgagcgag aatctggaga     540

gaaggcggca agaggcagaa gagttggagg ggtactgcat tcaactcaag gagaactgct     600

ggaaggtgac ccggtctgtg gaagatgctg aaatcaaaac caacgtcttg aagcagaatt     660

ctgccctgct ggaggagaag ctgcgctacc tccagcagca gctgcaggat gagacgccgc     720

gacggcagga ggccgagctg caggagccgg aggagaagca ggagccggag gagaagcagg     780

agccggagga gaagcagaag ccggaggctg gcctctcctg gaacagcctg ggccccgccg     840

ccacgtccca gggctgcccc ggcccgccag ggagtcccga caaaccctcg cggccacacg     900

gcctggtccc cgcaggctgg ggaatggggc ctcgggctgg cgagggcccc tacgtgagcg     960

agcaggaatt gcagaagctg ttcaccggca tcgaagagct gaggagagag gtgtcctcac    1020

tgaccgcccg gtggcatcag gaggaggggg cggtgcagga agccctgcgg ctgctcgggg    1080

gcctgggcgg cagggtcgac ggcttcctag gccagtggga gcgggcacag cgcgaacagg    1140

cacagacggc gcgggacttg caggagctgc gaggtcgggc ggatgagctg tgcaccatgg    1200

tggagcggtc agcagtgtct gtggcttcac tgaggagcga actggagggg ctgggcccac    1260

tgaaacccat tctggaggag ttcgggcggc aatttcagaa ctctcgaaga gggcctgacc    1320

tctccatgaa cctggatcgg tcccaccaag gcaactgtgc ccgctgtgcc agccaggggt    1380

cgcagttgtc tacggagtcc ctgcagcagc tgctggaccg agcactgacc tcactagtgg 1440

acgaggtgaa gcagaggggc ctgactcctg cctgtcccag ctgtcagagg ctacacaaga 1500

agattctgga gctggagcgc caggccttag ccaaacacgt cagggcagag gccctgagct 1560

ccacccttcg gctggcccaa gacgaggccc tgcgggccaa gaacctactg ctgacagaca 1620

agatgaagcc agaggagaag atggccactc tggaccatct acacttgaag atgtgctccc 1680

tccacgatca tctcagcaac ctgccacttg aggggtccac gggaacaatg gggggaggca 1740

gcagtgcagg aaccccccca aaacaggggg gctcagcccc tgaacaataa atggcctctc 1800

atgctagcat ga 1812

<210> 42
<211> 3563
<212> DNA
<213> Homo sapiens

<223> DDHD domain containing 1 (DDHD1), mRNA

<400> 42
aacgcagctg cggcggctgc gggtctcgtg ggggcggagc ggtcgccgct gccgccgcag 60

ctcgggtcgg gatttgaaag attagaaact tcgggtggag agggcggcgg cgttgaatgt 120

gtggcggaag cgctgggggt cacggctccg cgcgccgccg gacagccggc ggcgtctcca 180

cagcatgaat tacccgggcc gcgggtcccc acggagcccc gagcataacg gccgaggcgg 240

cggcggcggc gcctgggagc tgggctcaga cgcgaggcca gcgttcggcg gcggcgtctg 300

ctgcttcgag cacctgcccg gcggggaccc ggacgacggc gacgtgcccc tggccctgct 360

gcgcggggaa cccgggctgc atttggcgcc gggcaccgac gaccacaacc accacctcgc 420

gctggacccc tgcctcagtg acgagaacta tgacttcagc tccgccgagt cgggctcctc 480

gctgcgctac tacagcgagg gtgagagcgg cggcggcggc agctccttgt cgctgcaccc 540

gccgcagcag cctccgctgg tcccgacgaa ctcggggggc ggcggcgcga caggagggtc 600

ccccggggaa aggaaacgta cccggcttgg cggcccggcg gcccggcacc gctatgaggt 660

agtgacggag ctgggcccgg aggaggtacg ctggttctac aaggaggaca agaagacctg 720

gaagcccttc atcggctacg actcgctccg catcgagctc gccttccgga ccctgctgca 780

gaccacgggt gcccggcccc agggcgggga ccgggacggc gaccatgtgt gctcccccac 840

gggcccagcc tccagttccg gagaagatga cgatgaggac cgcgcctgcg gcttctgcca 900

gagtacgacg gggcacgagc cggagatggt ggagcttgtg aacatcgagc ctgtgtgcgt 960

gcggggcggc ctctacgagg tggatgtgac ccaaggagag tgctacccgg tgtactggaa 1020

ccaggctgat aaaataccag taatgcgtgg acagtggttt attgacggca cttggcagcc 1080

tctagaagag gaagaaagta atttaattga gcaagaacat ctcaattgtt ttaggggcca 1140

gcagatgcag gaaaatttcg atattgaagt gtcaaaatcc atagatggaa aagatgctgt 1200

tcatagtttc aagttgagtc gaaaccatgt ggactggcac agtgtggatg aagtatatct 1260

ttatagtgat gcaacaacat ctaaaattgc aagaacagtt acccaaaaac tgggattttc 1320

taaagcatca agtagtggta ccagacttca tagaggttat gtagaagaag ccacattaga 1380

agacaagcca tcacagacta cccatattgt atttgttgtg catggcattg ggcagaaaat 1440

ggaccaagga agaattatca aaaatacagc tatgatgaga gaagctgcaa gaaaaataga 1500

agaaaggcat ttttccaacc atgcaacaca tgttgaattt ctgcctgttg agtggcggtc 1560

aaaacttact cttgatggag acactgttga ttccattact cctgacaaag tacgaggttt 1620

aagggatatg ctgaacagca gtgcaatgga cataatgtat tatactagtc cactttatag 1680

agatgaacta gttaaaggcc ttcagcaaga gctgaatcga ttgtattccc ttttctgttc 1740

tcggaatcca gactttgaag aaaaaggggg taaagtctca atagtatcac attccttggg 1800

atgtgtaatt acttatgaca taatgactgg ctggaatcca gttcggctgt atgaacagtt 1860

gctgcaaaag gaagaagagt tgcctgatga acgatggatg agctatgaag aacgacatct 1920

tcttgatgaa ctctatataa ctaaacgacg gctgaaggaa atagaagaac ggcttcacgg 1980

attgaaagca tcatctatga cacaaacacc tgccttaaaa tttaaggttg agaatttctt 2040

ctgtatggga tccccattag cagttttctt ggcgttgcgt ggcatccgcc caggaaatac 2100

tggaagtcaa gaccatattt tgcctagaga gatttgtaac cggttactaa atatttttca 2160

tcctacagat ccagtggctt atagattaga accattaata ctgaaacgct acagcaacat 2220

ttcacctgtc cagatccact ggtacaatac ttcaaatcct ttaccttatg aacatatgaa 2280

gccaagcttt ctcaacccag ctaaagaacc tacctcagtt tcagagaatg aaggcatttc 2340

aaccatacca agccctgtga cctcaccagt tttgtcccgc cgacactatg gagaatctat 2400

aacaaatata ggcaaagcaa gcatattagg ggctgctagc attggaaagg gacttggagg 2460

aatgttgttc tcaagatttg gacgttcatc tacaacacag tcatctgaaa catcaaaaga 2520

ctcaatggaa gatgagaaga agccagttgc ctcaccttct gctaccaccg tagggacaca 2580

gacccttcca catagcagtt ctggcttcct cgattctgca ttggagttgg atcacaggat 2640

tgattttgaa ctcagagaag gccttgtgga gagccgctat tggtcagctg tcacgtcgca 2700

tactgcctat tggtcatcct tggatgttgc ccttttcttt ttaaccttca tgtataaaca 2760

tgagcacgat gatgatgcaa aacccaattt agatccaatc tgaactctct tgaaggacat 2820

gaatggccta aaactgattt tttttttttc cgttaaaatg tgtgtgtcaa gatacggaga 2880

tttcagggtt aaagtatatt tcagttttct ttagggcaac atatatttga atttaaaagc 2940

actttattta aaaaaaaaag aagttttcag ttctgaagaa gtcatttaca gtttgcatca 3000

ttttaattat gagtctgaca aaaccttctc cagagaatca agcaagacct ggatgtgaag 3060

aaggtttggg taaactgcat gtaaaggcta caaatcacaa tctgattcct cccaaatata 3120

aaggcatatg gaacataatg tattaaccaa agtatgttat aaatcaaaaa tggtcaaggt 3180

tcagcatatt ctatatgaag atcacaaggt ggtatcgttt tagatttcta tgaaggcttt 3240

catttgtaca tccctttgaa aaaatataac agatttaaaa tgttttgaat ttaacttgtt 3300

tagaaaaact aatgcttaaa acaatatttg aactactgta tttataattt attacctcta 3360

attgctttat ttcagtgtat gagacattac tgttttaatg tttgctttga acataattta 3420

agaaccagat ttattttcta tagtgataaa cccttttttc tcagaactcc atctttgtac 3480

tcttcagatg aatatataga cactgtggca tacatttttt ttcattaaaa acttatggct 3540

tcatacaaaa aaaaaaaaaa aaa 3563

<210> 43
<211> 895
<212> DNA
<213> Homo sapiens

<223> cystatin 8 (cystatin-related epididymal specific) (CST8), mRNA

<400> 43
ctgggccaca ggcatgaact aagagtgctg aagacctagg agaaccactg gtcaggtcag 60

ctcccagctc cacagggagt aagaaggagc tgcaggcaca gcaggctgaa agtccaccaa 120

gggctgaggc cacaaggcct ctggggcagc cacagttttc atgatcacat catacaggac 180

tgagaagcag ataacaagag tgacgctcac agggctgggc tgacgctaac aggaggcagt 240

gtgtggctcg aagattcttg aacccacagc agcagctgcg gccaccccat cctgcccaca 300

gctccagccc tgagacgacg aggaggagag tcgactttgc ctcttgccca agggaccatg 360

cccaggtgcc ggtggctctc cctgatcctc ctcaccattc ccctggccct ggtggccagg 420

aaagacccaa aaaagaatga gacaggggtg ctgaggaaat taaaacccgt caatgcctca 480

aatgccaacg tgaagcagtg tctgtggttt gccatgcaag aatacaacaa agagagcgag 540

gacaagtatg tcttcctggt ggtcaagaca ctgcaagccc agcttcaggt cacaaatctt 600

ctggaatacc ttattgatgt agaaattgcc cgcagcgatt gcagaaagcc tttaagcact 660

aatgaaatct gcgccattca agaaaactcc aagctgaaaa ggaaattaag ctgcagcttt 720

ttggtaggag cacttccctg gaatggtgaa ttcactgtga tggagaaaaa gtgtgaagat 780

gcttaatggt gttttgaggc atccctccaa cctctgtgac tactttatcc atgaaaatga 840

agcaatggca ggtgggaggc tcttcccaat gtgctttctt catgcatgcc aaaaa 895

<210> 44
<211> 4743
<212> DNA
<213> Homo sapiens

<223> homeodomain interacting protein kinase 3 (HIPK3), transcript variant
 1, mRNA

<400> 44
ccgggaagga agatgaggga gacgggcccg gcgcttagca gccagagcag cagcagcagc 60

agcagcggtc gggggagggt gtttcgccgt ttcctctcag ccgccaggac aagatggcag 120

cggccgcgga gaggggctga gcccgggctg ggtggtgccg cctgctgaag cgcctggctc 180

ccggtccccg gcacggccct gcgccccacc ccggacatgc tcagggctgc ggccgcccga 240

agaggagaga gcgcgggcct ctaggaaggt atggcctcac aagtcttggt ctacccacca 300

tatgtttatc aaactcagtc aagtgccttt tgtagtgtga agaaactcaa agtagagcca 360

agcagttgtg tattccagga aagaaactat ccacggacct atgtgaatgg tagaaacttt 420

ggaaattctc atcctcccac taagggtagt gcttttcaga caaagatacc atttaataga 480

cctcgaggac acaacttttc attgcagaca agtgctgttg ttttgaaaaa cactgcaggt 540

gctacaaagg tcatagcagc tcaggcacag caagctcacg tgcaggcacc tcagattggg 600

gcgtggcgaa acagattgca tttcctagaa ggcccccagc gatgtggatt gaagcgcaag 660

agtgaggagt tggataatca tagcagcgca atgcagattg tcgatgaatt gtccatactt 720

cctgcaatgt tgcaaaccaa catgggaaat ccagtgacag ttgtgacagc taccacagga 780

tcaaaacaga attgtaccac tggagaaggt gactatcagt tagtacagca tgaagtctta 840

tgctccatga aaaatactta cgaagtcctt gattttcttg gtcgaggcac gtttggccag 900

gtagttaaat gctggaaaag agggacaaat gaaattgtag caatcaaaat tttgaagaat 960

catccttctt atgcccgtca aggtcaaata gaagtgagca tattagcaag gctcagtact 1020

gaaaatgctg atgaatataa ctttgtacga gcttatgaat gctttcagca ccgtaaccat 1080

acttgtttag tctttgagat gctggaacaa aacttgtatg actttctgaa acaaaataaa 1140

tttagtcccc tgccactaaa agtgattcgg cccattcttc aacaagtggc cactgcactg 1200

aaaaaattga aaagtcttgg tttaattcat gctgatctca agccagagaa tattatgttg 1260

gtggatcctg ttcggcagcc ttacagggtt aaagtaatag actttgggtc ggccagtcat 1320

gtatcaaaga ctgtttgttc aacatatcta caatctcggt actacagagc tccagagatt 1380

atattggggt tgccattttg tgaagccata gacatgtggt cattgggatg tgtgattgca 1440

gaattatttc ttggatggcc gctctaccca ggagccttgg agtatgatca gattcgatac 1500

atttctcaga ctcaaggttt gccaggagaa cagttgttaa atgtgggtac taaatccaca 1560

agattttttt gcaaagaaac agatatgtct cattctggtt ggagattaaa gacattggaa 1620

gagcatgagg cagagacagg aatgaagtct aaagaagcca gaaaatacat tttcaacagt 1680

ctggatgatg tagcgcatgt gaacacagtg atggatttgg aaggaagtga tcttttggct 1740

gagaaagctg atagaagaga atttgttagt ctgttgaaga aaatgttgct gattgatgca 1800

gatttaagaa ttactccagc tgagaccctg aaccatcctt ttgttaatat gaaacatctt 1860

ctagatttcc ctcatagcaa ccatgtaaag tcctgttttc atattatgga tatttgtaag 1920

tcccacctaa attcatgtga cacaaataat cacaacaaaa cttcactttt aagaccagtt 1980

gcttcaagca gtactgctac actgactgca aattttacta aaatcggaac attaagaagt 2040

caggcattga ccacatctgc tcattcagtt gtgcaccatg gaatacctct gcaggcagga 2100

actgctcagt ttggttgtgg tgatgctttt cagcagacat tgattatctg tcccccagct 2160

attcaaggta ttcctgcaac acatggtaaa cccaccagtt attcaataag ggtagataat 2220

acagttccac ttgtaactca ggccccagct gtgcagccac tacagatccg accaggagtt 2280

ctttctcaga cgtggtctgg tagaacacag cagatgctgg tgcctgcctg gcaacaggtg 2340

acacccctgg ctcctgctac tactacacta acttctgaga gtgtggctgg ttcacacagg 2400

cttggagact gggggaagat gatttcatgc agcaatcatt ataactcagt gatgccgcag 2460

cctcttctga ccaatcagat aactttatct gcccctcagc cagttagtgt ggggattgca 2520
catgttgtct ggcctcagcc tgccactacc aagaaaaata aacagtgcca gaacagaggt 2580
attttggtaa aactaatgga atgggagcca ggaagagagg aaataaatgc tttcagttgg 2640
agtaattcat tacagaatac caatatccca cattcagcat ttatttctcc aaagataatt 2700
aatgggaaag atgtcgagga agtaagttgt atagaaacac aggacaatca gaactcagaa 2760
ggagaggcaa gaaattgctg tgaaacatct atcagacagg actctgattc atcagtttca 2820
gacaaacagc ggcaaaccat cattattgcc gactccccga gtcctgcagt gagtgtcatc 2880
actatcagca gtgacactga tgaggaagag acttcccaga gacattcact cagagaatgt 2940
aaaggtagtc tagattgtga agcttgccag agcactttga atattgatcg gatgtgttca 3000
ttaagtagtc ctgatagtac tctgagtacc agctcctcag ggcagtccag cccatccccc 3060
tgcaagagac cgaatagtat gtcagatgaa gagcaagaaa gtagttgtga tacggtggat 3120
ggctctccga catctgactc ttccgggcat gacagtccat ttgcagagag cacttttgtg 3180
gaggacactc atgaaaacac agaattggta tcctctgctg acacagaaac caagccagct 3240
gtctgttctg ttgtggtgcc accagtggaa ctagaaaatg gcttaaatgc cgatgagcat 3300
atggcaaaca cagattctat atgccagcca ttaataaaag gacgatctgc ccctggaaga 3360
ttaaaccagc cttctgcagt gggtactcgt cagcaaaaat tgacatcagc attccagcag 3420
cagcatttga acttcagtca ggttcagcac tttggatctg ggcatcaaga gtggaatgga 3480
aactttgggc acagaagaca gcaagcttat attcctacta gtgttaccag taatccattc 3540
actctttctc atggaagtcc caatcacaca gcagtgcatg cccacctggc tggaaataca 3600
cacctcggag gacagcctac tctacttcca tacccatcat cagccaccct cagtagtgct 3660
gcaccagtgg cccacctgtt agcctctccg tgtacctcaa gacctatgtt acagcatcca 3720
acttataata tctcccatcc cagtggcata gttcaccaag tcccagtggg cttaaatccc 3780
cgtctgttac catccccaac cattcatcag actcagtaca aaccaatctt cccaccacat 3840
tcttacattg cagcatcacc tgcatatact ggatttccac tgagtccaac aaaactcagc 3900
cagtatccat atatgtgaaa aacagtatat tggggaagct caatgataca aacatttgat 3960
taaaaataaa aacatggtat ttaatattag ccatggcaca agaaaattat ttttgaatca 4020
tgtagacttg ggtgcaattt aaacaacttt gagctttaaa aactcacttt tgatgtgttt 4080
tgcacatttg gtataacttg tctttggtca tgttatcttc ttatgtagta actctagaca 4140

ggtgacttat gggagcagaa gtccagtttt gctcctgcta ttttttataa attgccttct 4200

aactagtgca agacacgtct acatttggga agccattctg tgtacagact tagagcaaca 4260

gatgcacata tgtcagaatt acagcataca agtgaattgt attatccgtg tcttagtgta 4320

taaatgttgg gtcacttacc taagaaattg agctattgtt ctttacattt gcatgtgtct 4380

tttgcatggg caaaatgttg cctagacttt gctcttaaat gttgttctaa taatctcagc 4440

tgcattgtaa accgttccta cacatagtgc cttaaatatt tgaggttgtt aatgttatta 4500

cctatatata aatgttgagg actgcagcac ttaaaattca gacctactat ttagtttcct 4560

tttgatagcg taatgttcat ttttgttttt gtgtggtatg atttcaggta gtagctgttt 4620

ttttccttat taagagggca gcatgtttgc tatagctgaa ttctgctgtc tgatttttca 4680

gaatgatcta gcttcaagaa aagcaagcag ttagtagtgc ttaagaaaaa ttgattcagt 4740

atc 4743

<210> 45
<211> 2124
<212> DNA
<213> Homo sapiens

<223> cDNA FLJ10162 fis, clone HEMBA1003560, highly similar to GUANINE
      NUCLEOTIDE-BINDING PROTEIN G(I)/G(S)/G(O) GAMMA-2 SUBUNIT

<400> 45
acaacgctgc cgccgcgctc cgtgggcaac tcctactact gctgggctgg gctgggctgg 60

gctgggctgc gccggagctc gcctgcacag atcagctccg gagaggggag aaccacgctc 120

ctcggaccaa gcctcgggag ctaagccaga tctgccagtg agcctcaggc tttaggaact 180

gaagagtgtt tctgaaagat ctatccagca ctccgatggc cagcaacaac accgccagca 240

tagcacaagc caggaagctg gtagagcagc ttaagatgga agccaatatc gacaggataa 300

aggtgtccaa ggcagctgca gatttgatgg cctactgtga agcacatgcc aaggaagacc 360

ccctcctgac ccctgttccg gcttcagaaa acccgtttag ggagaagaag tttttctgtg 420

ccatccttta agtctttgag aggggcctga agagcctccg ggctcctggg acattgatgt 480

agagttttta gtgaagtggg cacctttcta gtccacggca tttgaagaga gcgaggagaa 540

ccattctgga aactctaggc tatgcatgtt taaagatctg gtccccttta tgagaatgca 600

agccgatcca catcctgact taagagatct gattctgacg aactgcctgg aggaggggaa 660

tatataaaaa taaaattggt gtcacttctt ttctgctatc ccccagcccc cccccccaaa 720

atcctcatgt ttctgcttca tattttgaaa aataacaatt aaaacagaca gctgtactga 780

ggtaagatat gtgtgacctt cttggaatga atattgtctt tagaataccc tttgataagc 840

tgagctgtcc cgtgtagatg caattcggtt taatggcatt gatgtatagt cactgtgcct 900

ttctttttct ttcttccttc tcctctaccc ctccttccac ccctccccat tagagtagtg 960

tggagataag gctggactgg tctatcagat tgaactccaa gaatgatcac acaaaatgtt 1020

tagggagatg ttccccgtgg tgtatcctca tggtaacaac gacaaaaaat gccggttgtc 1080

tgtgttctct tttcactatt cctaacatgt gtacatgata gttttgattc tgcaagtaaa 1140

agtaaatcct gtgttgtgac tggtgctttc atatatttgt gacaattttt gagtaatatt 1200

gcatgaaaat gtccctatgt tacatccatt cagaagtttt gttgttttac tctaaagctg 1260

ggaaaggaaa tgagagggaa aagaccccgg agagggaaga aaatctcagt attttgaaaa 1320

ttgagattac ttcagagcct tagccacacc taaaatacct cctagttaat agtggtataa 1380

atgcctcttc aatacgtttt ccagaatcca aagcattttg gtttatccag gacccagggc 1440

agcacagctg tcaccaagca ggagagttaa ggattcacca tgagctggga aatgcttttg 1500

ccatgagtat gagcaaattc cctctttccc tgaatcatgg acattctaga ttaaaagaac 1560

attttttgtg ctcttaacaa gaaaaccatg gccctccttt gttcaagtat cacaagaaat 1620

aaacccacag ctccagagaa ggtgaccatt ctcagaactc cagctattca ctctccaggg 1680

agaaggacct caaatcgcca ctctttgggc ggcaggtgcg gtccccacgg ccggctctac 1740

gaggaagagt tctggctctt ttgtccactg agatggtctt ggtttttcac ttaacaaatt 1800

ttttaatgga atctttgttt ttgttctcca tcttgtttgt tagagtctct cggcctttat 1860

ttacaaattc cttgcaacta gagcgctcct tccccaagat atggtagtga gagtaatttt 1920

tcattgtagc tgtagtctcc atcagtaaca gcaggccctg gaagacttga tcaccttttt 1980

ctgtgtcatt ttcagtcaaa gagggcctct cttacatctt gtttgctttc aaatccccaa 2040

atatcatctc catctcccaa ttaattttat tctctctttc ctctgtattc gctttctcct 2100

gttttttttt ttaaaaaaag catg 2124

<210> 46
<211> 1466
<212> DNA
<213> Homo sapiens

<223>  chromosome 11 open reading frame 71 (C11orf71), mRNA

<400>  46
atactcacca ggatacccct cgttaagggc aaggcggctt ctggctcttc cgcaggctca      60

gttgtgggtt cctgtgtgct tagtcatcgg gtccgacaca ggctggactg atctggagag     120

ccgcgaaggg cctgccttca caaagggacg taacgcaagt actgcgggca gtgtttgaat     180

atggccctga acaatgtgtc cctgtcctcc ggtgatcaga ggagcagggt ggcctaccgc     240

tcttcccatg gcgacctcag accgcgggcg tcagcgttgg cgatggtctc cggagacggc     300

ttcctcgttt ccaggcctga agcgattcat ctaggacctc ggcaggcggt gcgaccaagc     360

gttcgggccg agagccgtcg agtggatggt ggcggccgga gcccaaggga accagatggc     420

cggggccgga gccgccaagc cagattctca ccttacccaa tccctgccgt tgaacccgat     480

ctcctaagaa gtgtgctgca acagcgtttg attgcattag gaggtgttat cgcagctcga     540

atttcagttt aaacgaacac ctttcctctg gccctcactt agcttgtgaa caggcctttt     600

taaaatcctt tcttggtgta gcagcaatcg tgttgtttta tttttagatg gatgtatttt     660

catttaatac gtgggagtta tatgaatatg tcctagcact aagggaagtg taacagttga     720

ttgatttagg tggttttatg cagccagaac gattggaaca ggaaaggtgt ggaagggata     780

ttcttaggtc gtcacaaagc tgagctgatg ggagcaccct ccccagaggt caacaatcca     840

ttcgttaact cagacactta acctttacct ttacttctgt tggcgaaaaa gaagtgctcc     900

tttttattac actgatttat ccttccatct tttccttggc atttaccatc ttccatctcc     960

cccttcactt ttttcagata tgtctacaaa cagactccaa tctcagtcgc actaaaaata    1020

agaacaaaac ctaaacctta cagttctttc aaattattaa atcaatttat tattcctttc    1080

tttggcaaag gttccaaatt tttactttat atttgtaatt ttactatttg cattcagtcc    1140

cagcgcatta atctgaatct gctgtcctaa aatacactgg tgaaaacatt acttgtacaa    1200

tctcctatca gaaaactgtt catctactca ctgtagtgcc ctccttgaaa tgtgtgtttg    1260

ttcattcaac taacaatatt tggggatccc tgtagtaaac actgtatgaa ttttacacag    1320

tctggccatc aagaaagatc acggagtata ttctagatgg ggaggctact aagtgaatag    1380

gaatcaccac gctgggctgt ttattaggta cagtaataaa cataagtact ggttgcagtg    1440

aaaaaaaaaa aaaaaaaaaa aaaaaa                                          1466

210>  47

<211>  30
<212>  DNA
<213>  Artificial

<220>
<223>  synthetic oligonucleotide

<400>  47
ttacatatcg ctgagtgttc tgtcacccca                                        30

<210>  48
<211>  30
<212>  DNA
<213>  Artificial

<220>
<223>  synthetic oligonucleotide

<400>  48
cttcctactg cccatgtgat gccgtctaat                                        30

<210>  49
<211>  30
<212>  DNA
<213>  Artificial

<220>
<223>  synthetic oligonucleotide

<400>  49
ctttaggttt ctcttccatc gggtgacttg                                        30

<210>  50
<211>  30
<212>  DNA
<213>  Artificial

<220>
<223>  synthetic oligonucleotide

<400>  50
atcgtggtaa tggtgctgtt ctcgctcttc                                        30

<210>  51
<211>  35
<212>  DNA
<213>  Artificial

<220>
<223>  synthetic oligonucleotide

<400>  51
ctggaattcc cttcgctgtt tgccttcatc tccct                                35

<210>  52
<211>  28
<212>  DNA
<213>  Artificial

<220>
<223>  synthetic oligonucleotide

<400>  52
tcttcacagc cccgacatca actccttc                                        28

<210>  53
<211>  29
<212>  DNA
<213>  Artificial

<220>
<223>  synthetic oligonucleotide

<400>  53
gctggaatgt ctgctctcta ttggtgcct                                       29

<210>  54
<211>  30
<212>  DNA
<213>  Artificial

<220>
<223>  synthetic oligonucleotide

<400>  54
cctcttgggc tattggactg tctgtgatgg                                      30

<210>  55
<211>  30
<212>  DNA
<213>  Artificial

<220>
<223>  synthetic oligonucleotide

<400>  55

taccacaact tcaccaaaat ccccactctg                                        30

<210>  56
<211>  30
<212>  DNA
<213>  Artificial

<220>
<223>  synthetic oligonucleotide

<400>  56
aatggttttc ttggcttcct ccttactccc                                        30

<210>  57
<211>  24
<212>  DNA
<213>  Artificial

<220>
<223>  synthetic oligonucleotide

<400>  57
gagaagtctc ctctgcattt tggg                                              24

<210>  58
<211>  30
<212>  DNA
<213>  Artificial

<220>
<223>  synthetic oligonucleotide

<400>  58
attcccgcct tatgcctcag tctattctgt                                        30

<210>  59
<211>  30
<212>  DNA
<213>  Artificial

<220>
<223>  synthetic oligonucleotide

<400>  59
cagcctctct gatccctcaa tttggtctgt                                        30

<210>  60
<211>  30
<212>  DNA
<213>  Artificial

<220>
<223>  synthetic oligonucleotide

<400>  60
attgttctgc ttcctggctg tatgatgggt                                        30

<210>  61
<211>  30
<212>  DNA
<213>  Artificial

<220>
<223>  synthetic oligonucleotide

<400>  61
cattcaagat ggttaccttt ccctgctgac                                        30

<210>  62
<211>  30
<212>  DNA
<213>  Artificial

<220>
<223>  synthetic oligonucleotide

<400>  62
aatggctacc tttctactgg aaccctgacc                                        30

<210>  63
<211>  30
<212>  DNA
<213>  Artificial

<220>
<223>  synthetic oligonucleotide

<400>  63
atggatctgc ctggtacaac tgtaaccgct                                        30

<210>  64
<211>  30
<212>  DNA
<213>  Artificial

<220>
<223>  synthetic oligonucleotide

<400>  64
aacatcctgc cagctacctc tgtgaactga                                        30

<210>  65
<211>  30
<212>  DNA
<213>  Artificial

<220>
<223>  synthetic oligonucleotide

<400>  65
acctcctgga gaaatgcaaa accctcgtta                                        30

<210>  66
<211>  30
<212>  DNA
<213>  Artificial

<220>
<223>  synthetic oligonucleotide

<400>  66
tgtaagcagg tccatctctc cacccactga                                        30

<210>  67
<211>  30
<212>  DNA
<213>  Artificial

<220>
<223>  synthetic oligonucleotide

<400>  67
ctgaatgaac tgtcctctga tgtgcctggt                                        30

<210>  68
<211>  30
<212>  DNA
<213>  Artificial

<220>
<223>  synthetic oligonucleotide

[…]

<210>  73
<211>  30
<212>  DNA
<213>  Artificial

<220>
<223>  synthetic oligonucleotide

<400>  73
cccatttcta caatttcttt gccgcttcct                                        30


<210>  74
<211>  30
<212>  DNA
<213>  Artificial

<220>
<223>  synthetic oligonucleotide

<400>  74
ccctgaggtc tggattcttt ctccgctact                                        30


<210>  75
<211>  30
<212>  DNA
<213>  Artificial

<220>
<223>  synthetic oligonucleotide

<400>  75
agccacagtt catccccaac cctagtcttc                                        30


<210>  76
<211>  30
<212>  DNA
<213>  Artificial

<220>
<223>  synthetic oligonucleotide

<400>  76
caagtttacc tgctgttctc acccccaatg                                        30


<210>  77
<211>  30
<212>  DNA

<213>  Artificial

<220>
<223>  synthetic oligonucleotide

<400>  77
ggcgtcacct tcatgtttta taccactgtt                                        30

<210>  78
<211>  30
<212>  DNA
<213>  Artificial

<220>
<223>  synthetic oligonucleotide

<400>  78
cagatcgaga tttgcgtcct agagtgggaa                                        30

<210>  79
<211>  30
<212>  DNA
<213>  Artificial

<220>
<223>  synthetic oligonucleotide

<400>  79
accctaagtc cccagaatgc taatcctcca                                        30

<210>  80
<211>  30
<212>  DNA
<213>  Artificial

<220>
<223>  synthetic oligonucleotide

<400>  80
aagacaaccc tgaagtgcct ccgctgattt                                        30

<210>  81
<211>  30
<212>  DNA
<213>  Artificial

<220>
<223>  synthetic oligonucleotide

<400>  81
atggcccaca agcagatcta ctactcggac                                        30

<210>  82
<211>  30
<212>  DNA
<213>  Artificial

<220>
<223>  synthetic oligonucleotide

<400>  82
cctgtccatc ctagcccatc ccagtttatc                                        30

<210>  83
<211>  30
<212>  DNA
<213>  Artificial

<220>
<223>  synthetic oligonucleotide

<400>  83
ttgactcctc ttcatcttag cccccctagc                                        30

<210>  84
<211>  30
<212>  DNA
<213>  Artificial

<220>
<223>  synthetic oligonucleotide

<400>  84
ggaagcctca ccatttctca aggtccataa                                        30

<210>  85
<211>  30
<212>  DNA
<213>  Artificial

<220>
<223>  synthetic oligonucleotide

<400>  85
cattcctgag gtttcttcca gctctgcatc                                        30

<210>  86
<211>  30
<212>  DNA
<213>  Artificial

<220>
<223>  synthetic oligonucleotide

<400>  86
gatgacccag gttgcttggc tgattatgtt                                        30

<210>  87
<211>  30
<212>  DNA
<213>  Artificial

<220>
<223>  synthetic oligonucleotide

<400>  87
tctacacttg aagatgtgct ccctccacga                                        30

<210>  88
<211>  30
<212>  DNA
<213>  Artificial

<220>
<223>  synthetic oligonucleotide

<400>  88
ctgtcacgtc gcatactgcc tattggtcat                                        30

<210>  89
<211>  30
<212>  DNA
<213>  Artificial

<220>
<223>  synthetic oligonucleotide

<400>  89
ggcatccctc caacctctgt gactacttta                                        30

<210>  90
<211>  30

<212>  DNA
<213>  Artificial

<220>
<223>  synthetic oligonucleotide

<400>  90
ttaccatccc caaccattca tcagactcag                                        30



<210>  91
<211>  30
<212>  DNA
<213>  Artificial

<220>
<223>  synthetic oligonucleotide

<400>  91
attggtgtca cttcttttct gctatccccc                                        30



<210>  92
<211>  30
<212>  DNA
<213>  Artificial

<220>
<223>  synthetic oligonucleotide

<400>  92
cgttgaaccc gatctcctaa gaagtgtgct                                        30

## Description

### FIELD OF THE INVENTION

The invention relates to an in vitro method for diagnosing and/or characterizing a pathological condition associated with male infertility. The invention also relates to an in vitro method for evaluating the prospect of success of a testicular sperm extraction procedure. In a further aspect, the invention provides a microarray for diagnosing and/or characterizing a pathological condition associated with male infertility and/or for evaluating the prospect of success of a testicular sperm extraction procedure. Kits for conducting the methods of the invention are provided. Finally, a method of screening an active agent for treating a pathological condition associated with male infertility is also provided.

### BACKGROUND OF THE INVENTION

Across the European countries, birth rates are continuously declining. It is presently unclear, whether this represents a mere social phenomenon, or whether biological fertility is also declining (Butler (2004), Nature 432, 38-39). For example, environmental and lifestyle factors may contribute to biological fertility. Furthermore, fertility declines with the age of a couple, and the current trend to start families late will increase the problem of infertility and thus enhance the impact of the proposed project in the future.

In more than 50% of infertile couples, assisted reproduction techniques (ART) are carried out because of male factor infertility or subfertility, using either single ejaculated sperm or testicular spermatozoa obtained after testicular sperm extraction (TESE) for intracytoplasmic sperm injection (ICSI; Palermo et al. (1992), Lancet 340, 17-18). Techniques of testicular sperm extraction (TESE) and cryo-TESE have been described in the art (Schulze et al. 1997, in: The Fate of the Male Germ Cell, ed. Ivell and Holstein, Plenum Press New York, 1997; Jezek and Schulze (1998), Hum Reprod 13, 1230-1234; Schulze (1998), In: Kempers, Cohen, Haney, Young (eds.), Elsevier Science, Amsterdam, 621-626). In a considerable proportion of patients, however, no sperm suitable for ICSI can be found, each futile attempt posing additional stress to a couple. Moreover, there is growing concern about passing on mutations and a complex infertility condition to future generations by ICSI. Still, research into the causes of male infertility has lagged considerably behind the success in other areas of medicine, leaving approximately 40-70% of patients with disorders described as "idiopathic" (i.e. the cause of which is unknown).

At present, a careful histological examination is the only reliable method for diagnosing several pathological conditions associated with male infertility (Johnsen (1970), Hormones, 1, 2-25; De Kretser and Holstein (1976), In: Human Semen and Fertility Regulation in Men, Ed. Hafez ESE, Mosby, St. Louis, 332-343; Schulze et al. (1999), Hum Reprod, 14, 82-96). This procedure, however, has major drawbacks: the method is descriptive and largely subjective, and the results of the diagnosis will depend very much upon the personal experience of the pathologist. Apart from this, only a small area of the biopsy is regularly assessed, and residual sperm may be simply overlooked. Furthermore, functional/genetic deficits of spermatozoa cannot be properly addressed, and a prognosis for the outcome of TESE is difficult to obtain.

The above problems have now been overcome by identifying marker genes with expression profiles that can be used in a reliable manner to identify distinct pathological conditions of the testes which contribute to male infertility. In the course of the present invention, genome-wide expression profiling on testicular biopsies was used to identify genes that play an essential role in human spermatogenesis. A panel of validated marker genes was extracted which can provide a highly reliable characterization of different subtypes of male infertility and allows for predicting the outcome of a TESE procedure. Distinct molecular phenotypes were identified that could be correlated to particular pathological conditions defined by the presence or absence of specific germ cell stages. Based on the marker genes disclosed herein, a molecular test system, such as a microarray, can be designed which is useful in diagnosising subtypes of male infertility and providing a prognosis of TESE success. By use of such techniques, fruitless attempts of sperm extraction and repeated surgical intervention can be avoided.

### BRIEF DESCRIPTION OF THE FIGURES

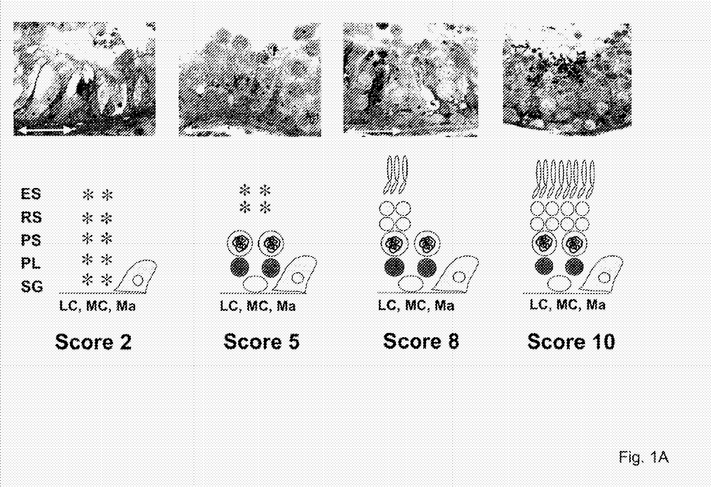

Figure 1 A shows in the upper panel semi-thin section micrographs of representative testis samples that underwent modified Johnsen scoring and were subsequently used for microarray analysis. The lower panel shows the diagrammatic and non-quantitative representation of cell types found in testicular biopsies as classified by morphological criteria. Score 2: Sertoli-cell-only (SCO); Score 5: premeiotic arrest; Score 8: postmeiotic arrest; Score 10: full spermatogenesis. Abbreviations: LC, Leydig cells; MC, myoid cells; Ma, macrophages; SG, spermatogonia; PL; preleptotane spermatocytes; PS, pachytene spermatocytes; RS, round spermatids, ES, elongated spermatids/testicular spermatozoa. White arrows indicates 50 µm scale.

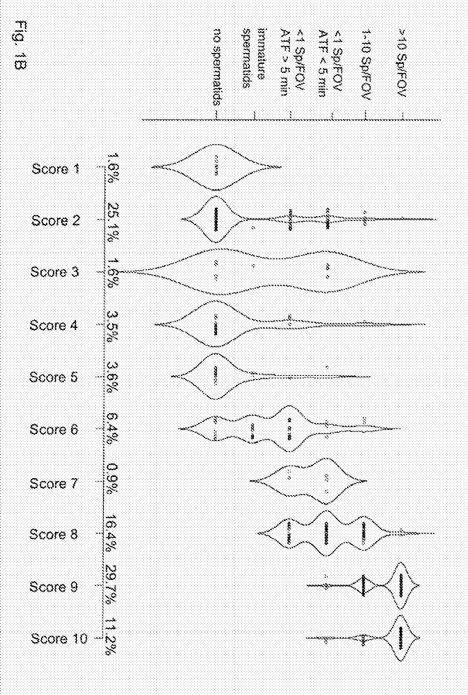

Figure 1 B shows the Kernel density box-plot ("violin plot") from 578 testicular biopsies correlating Johnsen score (modified according to de Kretser and Holstein, 1976) from one biopsy with number and search time of testicular spermatozoa found in an independent second biopsy used for TESE from the same testis. The area of the kernel density curves correlate with the proportion of samples within the category. Red dots depict the biopsy samples, red numbers are the number vs. total number of samples used for the microarray analysis within the category, numbers (in %) on the x-axis represent the percentage of Johnsen scored samples in relation to the complete clinical population. Abbreviations: Sp/FOV, testicular spermatozoa per microscopic field-of-view; ATF, "average time to find". Both criteria (FOV, ATF) are parameters derived from microscopical extraction of spermatozoa during the TESE process.

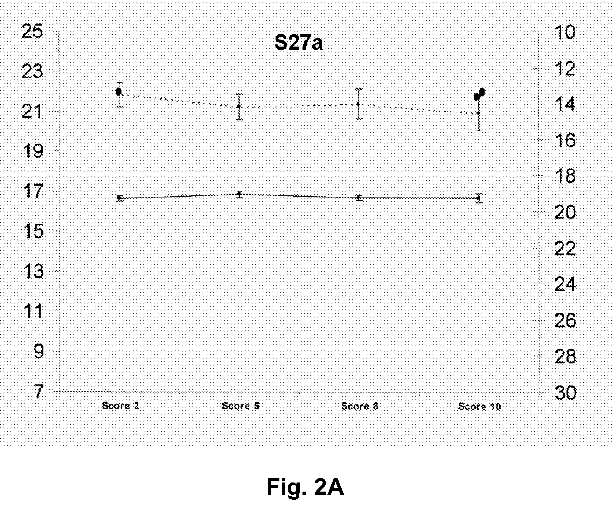

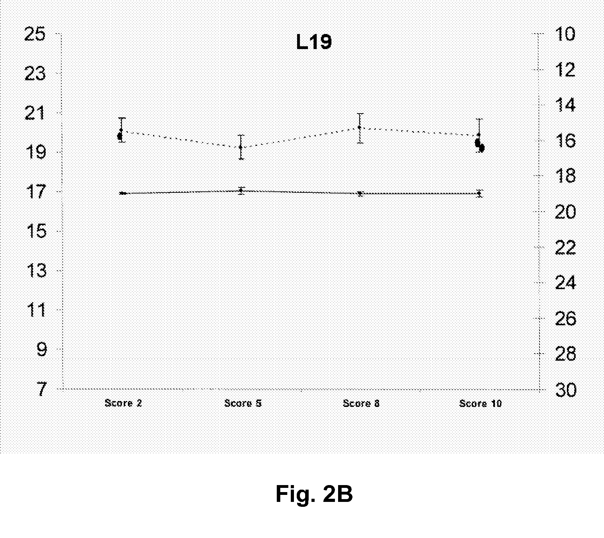

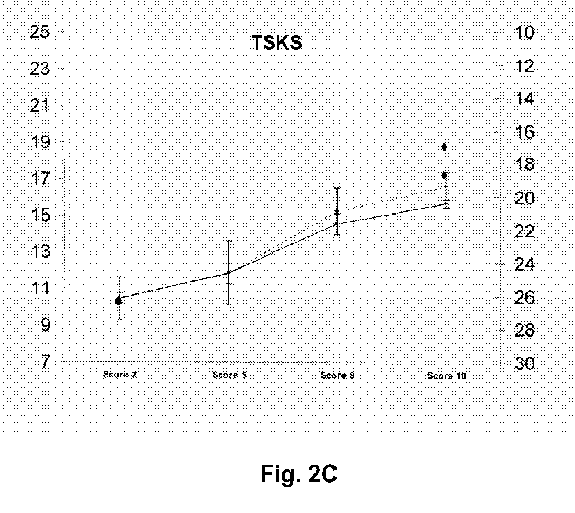

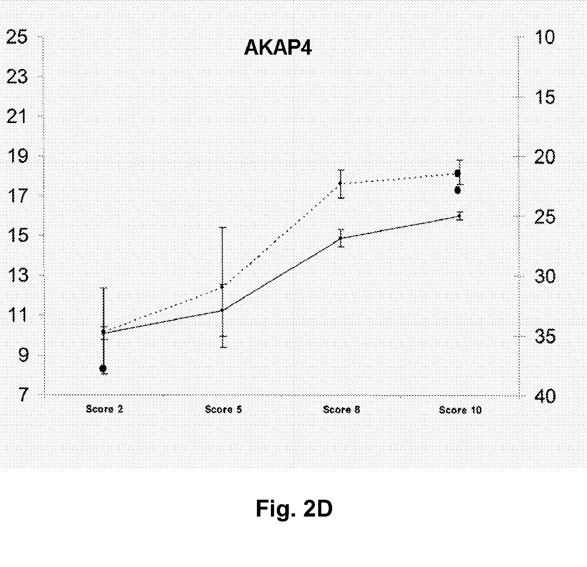

Figures 2 A-D show the validation of microarray data by quantitative real-time PCR (qRT-PCR). Two housekeeping genes S27a (Fig. 2A) and L19 (Fig. 2B), and two of the differentially transcribed marker genes identified by microarray analysis, namely TSKS (SEQ ID NO:41; Fig, 2C), and AKAP4/AKAP82 (SEQ ID NO:37; Fig. 2D) were validated by qRT-PCR on the same samples as used for the microarray experiments. Black line: expression level from microarray data; dashed line: expression level from qRT-PCR data. Error bars indicate standard deviation from the replicates of the four groups.

### DETAILED DESCRIPTION OF THE INVENTION

In the course of the present invention, it has been found that the expression levels of a group of marker genes in the testes of an individual can be directly correlated to different conditions of spermatogenetic failure and to the presence or absence of sperm. It was shown that the expression profiles of the genes depicted in SEQ ID NO: 1 to SEQ ID NO:46, i.e. the transcript levels found with these genes, considerably differ in individuals suffering from different stages of spermatogenetic failure. The varying transcript levels directly correlate with the presence or absence of distinct testicular germ cell types and might therefore be involved in a specific series of unique germ cell-associated processes including genetic recombination, meiosis, haploid gene expression, formation of the acrosome and flagellum, as well as remodeling and condensation of the sperm chromatin, each of these processes requiring novel gene products to occur in a timely precise and well-coordinated manner (for review see Schlecht and Primig (2003), Reproduction 125, 447-456). It is known in the art that diagnosis of distinct states of a disease on the basis of expression profiles require analysing a high number of genes in order to obtain a reliable result which can be correlated to the state of the patient. In course of the present invention, it was found that a combined assessment of the expression level of only two genes selected from a group of 46 marker genes allows for reliable diagnosis or characterization of different subtypes of male infertility. The use of the expression data from any pair of marker genes provided by the invention allows the clear allocation of the patient to different types of spaermatogenic failure. In other words, the marker genes provided in SEQ ID NO:1 to SEQ ID NO:46 constitute a homogeneous group of markers which (when used in any combination comprising at least two genes) allow for improved diagnosis with a minimal rate of patient misclassification.

The pathological conditions associated with male infertility that can be diagnosed by the methods of the present invention are conditions of spermatogenetic failure which have been extensively described in the art and which have been classified histologically by the so-called Johnsen scoring system (Johnsen (1970), Hormones, 1, 2-25). This scoring system is also described in detail in standard clinical textbooks, for example, in de Kretser, D. and Holstein, A.F. Testicular biopsy and abnormal germ cells. In: Hafez, E.S.E. (ed.) Human semen and fertility regulation in men (1976). The Johnsen score counts imply that the seminiferous tubules progressively lose their cell contents in a specific order, i.e. elongated spermatids disappear first, then round spermatids, then spermatocytes, then spermatogonia, and finally the Sertoli cells.

For the purposes of the present invention, all histological evaluations were performed according to the modified Johnsen scoring system described by De Kretser and Holstein (1976), In: Human Semen and Fertility Regulation in Men, ed. Hafez ESE, Mosby, St. Louis, pp.332-343; and Schulze et al. (1997), In: The Fate of the Male Germ Cell, eds. Ivell and Holstein, Plenum Press New York, 1997. This method is also referred to as the "modified Johnsen scoring". According to the invention, the complete range of histological stages characterized by a Johnsen score of 1 to 10 can be readily correlated to a corresponding molecular expression pattern of the marker genes provided in SEQ ID NO:1 to SEQ ID NO:46. Only for the purpose of illustration, the present invention is exemplified herein by pathological conditions characterized by a Johnsen scores of 2, 5, 8 and 10, respectively.

Accordingly, pathological conditions which are associated with male infertility and can be diagnosed by the methods of the present invention preferably comprise:
1.) conditions of post-testicular obstructions associated with complete spermatogenesis, i.e. >20 0 late spermatids (also referred to as 'testicular spermatozoa') per seminiferous tubule cross-section and repeated occurrence of residual bodies, i.e. Johnsen score count 10;
2.) conditions associated with severe hypospermatogenesis in all areas of the testis parenchyma with only few late spermatids, i.e. Johnsen score count 8;
3.) conditions associated with meiotic arrest of germ cells showing many spermatocytes and no spermatids, i.e. Johnsen score count 5;
4.) conditions associated with Sertoli-cell-only syndrome (SCOS) and no germ cells, i.e. Johnsen score count 2.

The marker genes depicted in SEQ ID NO:1 to SEQ ID NO:46 are generally underexpressed in patients suffering from the above conditions. The results of the expression analysis have been shown to be congruent with the histological assessment of biopsies. This means, the lower the expression level of the above-referenced marker genes, the lower the Johnsen score of the histologically examined sample. This is confirmed by Figure 3 which shows the results of expression analysis by RT-PCR and microarray analysis plotted against the histological Johnsen score.

Thus, according to a first aspect the present invention provides an in vitro method of diagnosing and/or characterizing a pathological condition associated with male infertility in an individual, comprising
(a) providing mRNA from a testis tissue sample of said individual;
(b) determining the expression levels of two or more genes selected from the group of genes shown in SEQ ID NO:1 to SEQ ID NO:46 on the basis of said mRNA; and
(c) comparing the expression levels of the two or more genes determined in step (b) to the expression levels of the same genes obtained from a reference testis tissue sample;
wherein the comparison of the gene expression levels reveals the presence and/or the degree of a pathological condition associated with male infertility.

The reference testis tissue sample may be derived from a subject which is not affected from any pathological condition associated with male infertility, i.e., from a healthy subject. In this case, the expression level for each of the two or more marker genes will be higher in the tissue sample from the healthy subject when compared to the expression levels of the corresponding genes in a subject afflicted with spermatogenetic failure. Hence, if a test patient exhibits substantially decreased expression levels for each of the two or more marker genes compared to the tissue sample from the healthy subject, this indicates the presence and/or the severity of a pathological condition associated with male infertility. According to the invention, the presence of a pathological condition associated with male infertility is assumed if each of the two or more genes selected from the group of genes shown in SEQ ID NO:1 to SEQ ID NO:46 independently exhibits an expression level that is decreased by at least by 50%, preferably 100%, more preferably 200%, even more preferably 300% and most preferably by 500% or more. With some of the marker genes, expression differences of 1000%, 5000%, 10.000%, 20.000% or even more can be detected, relative to the expression of the same gene in the tissue sample of the healthy subject.

The reference testis tissue sample may alternatively be derived from a subject which is affected from a pathological condition associated with male infertility. This means that the reference testis tissue sample may be taken from a patient showing the histological features of a pathological condition that is characterized by a Johnsen Score of between 1 and 10. In such a method, one could readily prepare a calibration curve by assessing the expression levels of the marker genes from a number of patients with a known Johnsen score and establish threshold values for these expression levels by the use of which a given patient can be allocated to a specific Johnsen Score based on the results from the expression analysis. In this manner, it will be possible to obtain insight into the status of spermatogenesis in the patient's testes without the need for histological examination of tissue samples.

In a first step of the diagnostic method provided by the invention, mRNA from a testis tissue sample of the individual to be tested is provided. As used herein, "testis tissue sample" means a sample obtained from an individual containing cells of one or both of the individual's testes. The sample may be derived from a human individual or from a related non-human mammal, such as a primate. Preferably, the individual to be diagnosed is a human.

The tissue sample can be collected in any clinically acceptable manner, but must be collected such that the mRNA contained in the cells is preserved for the subsequent method steps. For example, the tissue sample can be derived from a testis biopsy. As used herein, a testis biopsy refers to the excision of a small piece of testicular tissue for subsequent microscopic and/or molecular evaluation. The biopsy can be performed according to any suitable technique known in the art, for example as an open testis biopsy, a percutaneous testis biopsy, or according to the technique of percutaneous testicular fine needle aspiration (TFNA). An overview on the techniques of testis biopsy can be found in Schulze et al. (1997), In: The Fate of the Male Germ Cell, ed. Ivell and Holstein, Plenum Press New York.

In a next step, mRNA is provided from the testis tissue sample. Typically, the complete RNA is isolated from the testis tissue sample in a first step. Methods for isolating RNA are well known in the art and are extensively discussed in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Methods for isolating RNA regularly involve the lysis of the cells of the respective tissue samples. Cell lysis may be performed by use of detergents which are capable of disrupting the plasma membrane of the cells. For example, buffers containing guanidine thiocyanate and/or SDS may be used for cell lysis. The methods may also comprise steps in which the DNA of the cells is enzymatically digested in order to obtain pure RNA without traces of DNA which might interfere with the further downstream applications. Inhibitors of enzymes which lead to the degradation of the RNA may also be added to the lysis buffer.

Kits for the isolation of highly purified RNA are available from several manufactures. For example, the EZ1 RNA Cell Kits of Qiagen (Hilden, Germany) can be readily used for RNA isolation and purification. Similar kits are available from manufacturers such as Ambion, Stratagene, Clontech, Invitrogen, Promega, and others.

The RNA isolated from a testis tissue sample will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex^{™} matrix can be performed, as most mRNAs contain a poly(A) tail at their 3' terminus (see, for example, Ausubel et al. (1994), Current Protocols in Molecular Biology, Current Protocols Publishing, New York). Poly (A) + mRNA which has bound to the affinity matrix can be eluted using 2 mM EDTA/0.1% SDS.

The mRNA is subsequently used for the determination of the expression level of two or more genes selected from the group of the genes shown in SEQ ID NO:1 to SEQ ID NO:46. According to a preferred embodiment, step (b) of the method according to the invention comprises determining the expression level of more than two genes, for example 5, 6, 7, 8, 9, 10, 20, 25, 30, 35, 40 or 45 genes selected from the group of the genes shown in SEQ ID NO:1 to SEQ ID NO:46. According to a particularly preferred embodiment, step (b) of the method according to the invention comprises determining the expression level of all genes from the group of the genes shown in SEQ ID NO:1 to SEQ ID NO:46. Preferably, the determination of the expression level of the respective genes is performed simultaneously, for example by use of an microarray.

Several methods for determining the expression level of genes are known in the art. In a preferred embodiment of the invention, the expression level of the selected genes is determined by use of a microarray system. The terms "microarray system" or "bioarray system", which are interchangeably used herein, refer to the orderly arrangement of spatially resolved probes, for example nucleic acid probes, on a substrate. The substrate is preferably a solid substrate having a surface with a plurality of probes attached in different known locations (spots). The spots on a microarray are normally either printed on the microarrays or synthesized by photo-lithography or by ink-jet printing. On a common microarray, there may be several thousands spots, wherein each of the spots may contain a high number of identical probes , such as nucleic acid fragments or oligonucleotides. Such microarrays typically have a density of at least 100 oligonucleotides or fragments per cm². In certain embodiments the arrays can have a density of about at least 500, at least 1000, at least 10000, at least 10⁵, at least 10⁶, at least 10⁷ oligonucleotides or fragments per cm². The support can be a glass slide or membrane on the surface of which the probes are attached at fixed locations or spots.

Preferably, the probes of the microarray to be used in the diagnostic method of the invention are nucleic acid probes, i.e. oligonucleotides of fragments of DNA or RNA. The probes may be native DNA or RNA molecules or analogues of DNA or RNA or portions thereof. The length of the nucleic acid fragments or oligonucleotides may vary from 10-500 bp and is preferably in the range of 15, 20, 25, 30, 35, 40, 45 or 50 bp. The probe for a particular gene may even correspond to the full length coding sequence of said gene. Oligonucleotide probes may be conveniently synthesized by DNA synthesizer (for example, the MWG Oligo-2000 synthesizer) according to the well-known phosphoramidite method. The probes may be modified, for example by synthesizing oligonucleotides which have a modified backbone. Such modifications are known in the art and comprise, for example, phosphorothioate or phosphoramidate modifications of the backbone. The probe may also comprise one or more modified sugar moieties (e.g. *Locked Nucleic Acids^{™}"* (LNA, European Patent 1,013,661) or pepide bonds (Peptide *Nucleic Acids,* PNA, US Patent 6,841,675)).

For the purposes of the present invention, the nucleic acid probes of the microarray used in the methods for diagnosing and/or characterizing a pathological condition associated with male infertility are derived from the sequences provided in SEQ ID NO:1 to SEQ ID NO:46. The probes are complementary and hybridizable to cRNA derived from the expression of two or more of the genes shown in SEQ ID NO:1 to SEQ ID NO:46. Preferably, the nucleic acid probes are selected from the nucleic acid probes provided in SEQ ID NO:47 to SEQ ID NO:92. However, any other sequence which is derived from one of the genes of SEQ ID NO: 1 to SEQ ID NO:46 and which is suitable for being used as a probe on a microarray can be used as well. As used herein, a probe is "derived" from one of the genes shown in SEQ ID NO:1 to SEQ ID NO:46, if the nucleotide sequence of the probe is comprised by said gene sequence, whereas substitutions, insertions or deletions of the probe sequence are possible, as long as the probe remains hybridizable to the cRNA of the respective gene. Thus, the probe sequence will usually share a high degree of identity to a sequence stretch of the respective gene. Preferably, the probe will exhibit an identity of 80%, mor preferably 85%, 90%, 95% or 99% or more over a stretch of at least 15 nucleotides. Methods and criteria for selecting probe sequences are well known in the art and do not require further discussion herein. Methods for preparing microarrays are also known in the art and discussed in several publications, for example, in Petersen and Kawasaki, Manufacturing of microarrays. Adv. Exp. Med. Biol. 2007; 593:1-11.

The present invention further provides a microarray for diagnosing and/or characterizing a pathological condition associated with male infertility which comprises the above-described nucleic acid probes. The microarray consists of nucleic acid probes from not more than 1500 different genes, preferably human genes. It is preferred that the microarray of the invention consists of nucleic acid probes from not more than 1000 different genes, preferably not more than 900, 800, 700, 600, 500, 400, 300, 200, 100 or 50 different genes. Further, the microarray of the invention comprises nucleic acid probes derived from at least two or more of the nucleotide sequences provided in SEQ ID NO:1 to SEQ ID NO:46. Preferably, the nucleic acid probes are selected from the nucleic acid probes provided in SEQ ID NO:47 to SEQ ID NO:92. The microarray may also comprise nucleic acid probes suitable for the normalization of gene expression. Such probes are well known in the field and comprise, for example, probes derived from housekeeping genes such as S27a or L19. Different methods may be used for normalization of gene expression. After elimination of genes below background levels (Codelink^{™} system: estimated from 300 negative bacterial control spots) and imputation of missing values by a k-nearest neighbour approach (Troyanskaya et al., 2001), datasets can be normalized by cyclic Loess normalization using R statistical package (Affymetrix).

According to a further aspect, the invention relates to the use of such a microarray for diagnosing and/or characterizing a pathological condition associated with male infertility. According to a yet further aspect, the invention relates to the use of such a microarray for predicting the prospect of success of a testicular sperm extraction procedure.

Alternatively, commercially available bioarrays which comprise probes for measuring the expression of two or more of the genes referred to SEQ ID NO:1 to SEQ ID NO:46 may also be used for the methods of the invention. Several different bioarray systems for monitoring of mRNA levels of multiple genes are known in the art. For example, the Codelink^{™} Human 20K Bioarray (GE Healthcare) may be used for performing the methods of the present invention. This array system contains 19881 gene specific probes which are optimized for hybridization reaction with complementary RNA molecules. Other microarray systems (e.g. the Affymetrix GeneChip® U133A 2.0 array) as well as suitable imaging equipment for reading out the results of the hybridization reaction are commercially available.

Microarray analysis typically involves contacting at least one fluorescently labeled nucleic acid where it can hybridize with a corresponding probe with a complementary sequence that is immobilized on the support. The hybridization duplexes are subsequently excited by a laser and the fluorescence emission is measured and quantified (Lipshutz, RJ et al. 1999, Nature Genetics, Supplement, 21:20-23). The data may then be entered into a database and analyzed by statistical methods.

In the microarrays frequently used for determining the expression status of one or several genes, labeled is obtained from mRNA as starting material. In a first step, single stranded cDNA is synthesized by reverse transcription. The resulting single stranded cDNA is then converted into double stranded cDNA which is extracted for in vitro transcription. During in vitro transcription a detectable label, such as biotin, is incorporated into the cRNA. Prior to contact with the microarray, the cRNA is usually fragmented. By this procedure, the mRNA is amplified by factor 20 to 20000 compared to the starting sample. The hybridization complexes formed between the cRNA and the oligonucleotides/fragments of the microarray can be read out by suitable means, for example, by laser scanning imaging. These procedures are known in the art and reviewed, for example, in Hegde P, et al., 2000, A concise Guide to cDNA microarray analysis, BioTechniques 3, 548-554. Photolithographic techniques for in situ oligonucleotide synthesis are discussed in Fodor et al. U.S. 5,445,934. Electric field attraction/repulsion microarrays are disclosed in Hollis et al. U.S. Pat. No. 5,653,939 and Heller et al. U.S. Pat. No. 5,929,208. An electrode microarray for in situ oligonucleotide synthesis is disclosed in Montgomery, U.S. 6,093,302; 6,280,595 and 6,444,111.

When analyzing the expression of the genes depicted in SEQ ID NO:1 to SEQ ID NO:46 using a Codelink^{™} Human 20K Bioarray, the following expression patterns were obtained. Briefly, the expression of each of the genes of SEQ ID NO:1 to SEQ ID NO:46 was examined by microarray analysis in patients with Johnsen score 10 and 2, respectively. Subsequently, the ratio of the signal intensities (2 ^{relative Fluoresc. score 10}/2 ^{relative Fluoresc. score 2}) obtained with different types of microarrays was calculated as a measure for differential expression of said gene. The following values were obtained for the Codelink^{™} Human 20K Bioarray (values in brackets refer to the same examination using the Affymetrix GeneChip): SEQ ID NO:1, ratio 236 (7,9); SEQ ID NO:2, ratio 48,6 (0,84); SEQ ID NO:3, ratio 17,7 (2,4); SEQ ID NO:4, ratio 13,6 (6,8); SEQ ID NO:5, ratio 93,9 (58,4); SEQ ID NO:6, ratio 136,1 (16,8); SEQ ID NO:7, ratio 15,0 (11,1); SEQ ID NO:8, ratio 297,2 (156,9); SEQ ID NO:9, ratio 35,4 (3,4); SEQ ID NO:10, ratio 78,4 (18,1); SEQ ID NO:11, ratio 38,7 (2,5); SEQ ID NO:12, ratio 85,3 (7,3); SEQ ID NO:13, ratio 194,3 (167,7); SEQ ID NO:14, ratio 11,5 (22,4); SEQ ID NO:15, ratio 73,9 (-); SEQ ID NO:16, ratio 178,8 (34,2); SEQ ID NO:17, ratio 15,1 (7,2); SEQ ID NO:18, ratio 48,8 (6,4); SEQ ID NO:19, ratio 22,8 (23,8); SEQ ID NO:20, ratio 12,1 (7,6); SEQ ID NO:21, ratio 28,5 (4,7); SEQ ID NO:22, ratio 31,8 (26,7); SEQ ID NO:23, ratio 253,2 (210,4); SEQ ID NO:24, ratio 13,4 (13,0); SEQ ID NO:25, ratio 26,5 (-); SEQ ID NO:26, ratio 34,7 (4,6); SEQ ID NO:27, ratio 46,2 (139,3); SEQ ID NO:28, ratio 31,3 (13,2); SEQ ID NO:29, ratio 17,6 (4,5); SEQ ID NO:30, ratio 10, 1 (16,2); SEQ ID NO:31, ratio 39, 6 (34, 1) ; SEQ ID NO:32, ratio 18, (13, 7) ; SEQ ID NO:33, ratio 8, 7 (21,0); SEQ ID NO:34, ratio 27, 6 (25,6); SEQ ID NO:35, ratio 6, 3 (8, 5) ; SEQ ID NO:36, ratio 54,1 (8,8); SEQ ID NO:37, ratio 47,6 (74,7); SEQ ID NO:38, ratio 50,5 (20, 8); SEQ ID NO:39, ratio 13,6 (-); SEQ ID NO:40, ratio 26,0 (11,3); SEQ ID NO:41, ratio 27,2 (12,6); SEQ ID NO:42, ratio 16,0 (2,8); SEQ ID NO:43, ratio 22,2 (12,2); SEQ ID NO:44, ratio 24,5 (5,3); SEQ ID NO:45, ratio 7,8 (1,6); SEQ ID NO:46, ratio 14,3 (0,9).

It might be possible that different expression levels will be observed when other microarray systems are used for profiling the expression of the genes depicted in SEQ ID NO:1 to SEQ ID NO:46. However, in view of the insight that these genes are differentially expressed in distinct pathological conditions associated with male infertility, the skilled person will readily be able to assess the threshold levels for allocating the tested individual to a specific pathological condition associated with male infertility.

In a further preferred embodiment of the invention, the expression level of the selected genes is determined by use of a reverse transcription PCR analysis. For example, quantitative RT-PCR (qRT-PCR) can be used to determine the expression level of one or more marker gene. In this approach, the mRNA obtained from the tissue sample will subjected to reverse transcription, and the resulting cDNA will be exponentially amplified in a PCR reaction. The enzyme used for reverse transcription may be any of the transcriptases known in the art, for example, reverse transcriptase from avian myeloblastosis virus (AMV-RT) or Moloney murine leukemia virus (MLV-RT). Primers which are specific for a particular sequence, random hexamers, or oligo-dT primers may be used for priming in the reverse transcription step. The derived cDNA can then be used as a template in the subsequent PCR reaction. PCR can be conducted by use of a variety of DNA-dependent polymerases, such as Taq or Tth DNA polymerase.

In a preferred embodiment of the invention, quantitative RT-PCR may be performed as quantitative real time RT-PCR. As will be understood by those skilled in the art, real time PCR methods provide for the quantification of the target DNA after each round of amplification. Quantification is typically achieved by use of fluorescent dyes or oligonucleotide probes which comprise fluorescently labeled moieties. The methods are designed such that fluorescence increases proportionally with the amount of PCR product obtained from the amplification reaction.

In a most simple procedure of RT-PCR, quantitative real time RT-PCR may be performed by use of DNA intercalating dyes, such as ethidiumbromide or SYBR^{®} Green I. The fluorescent signal of the dye which has intercalated into the DNA produced in the amplification reaction can be detected. The use of DNA intercalating dyes, however, can only detect DNA unspecifically, so that it does not allow to draw any conclusion as to whether the amplified DNA in fact corresponds to the target sequence. Thus, according to a more preferred embodiment, the methods for quantitative real time RT-PCR allow for the detection of the specific product obtained by the amplification reaction. For this purpose, sequence specific probes can be used which are labeled with groups or moieties which can be quantitatively detected via Förster resonance energy transfer (FRET) (e. g. 5'→3' exonuclase assay known as *TaqMan*^{®}*Probe, Molecular Beacon* Probe, *Molecular Scorpion* probe, LightCycler Probes and others). FRET describes an energy transfer mechanism between two fluorescent molecules, such as two fluorescently labeled probes. A fluorescent donor is excited at its specific fluorescence excitation wavelength. By a long-range dipole-dipole coupling mechanism, this excited state is then non-radiatively transferred to a acceptor molecule. The change in the fluorescent signal of the acceptor molecule can be measured and is correlated to the distance of the donor and the acceptor molecule.

This principle is exploited in several quantitative real time RT-PCR approaches, for example in those which make use of TaqMan^{®} probes, hybridization probes or molecular beacons. These approaches are known to the skilled person and are moreover extensively discussed in standard laboratory handbooks (e.g. Molecular cloning - a laboratory manual. Sambrook J, Russel DW. 3rd ed. 2001). Kits for performing quantitative real time RT-PCR in accordance with these approaches are moreover available from different manufacturers. For example, TaqMan® RT-PCR can be performed by use of commercially available equipment, for example, the Lightcycler System (Roche, Mannheim, Germany) or the ABI PRISM 7700^{™} Sequence Detection System^{™} (Perkin-Elmer-Applied Biosystems, Foster City, USA).

RT-PCR is normally performed using an internal standard in order to minimize errors and sample-to-sample variations. The ideal internal standard is expressed at a constant level among different tissues and is moreover unaffected by the experimental treatment. Several genes are known in the art which fulfill these requirements. For example, the genes encoding glyceraldehyde-3-phosphate-dehydrogenase (GAPDH), β-actin and/or ribosomal protein S27a may be used to normalize patterns of gene expression according to the invention. Other examples of suitable internal standards will be known to the person of skill in the art.

In a further embodiment of the invention, the expression level of the selected genes is determined after performing a multiplex RT-PCR by use of a sequencing automate based on capillary electrophoresis and fluorescent detection. In this case the term multiplex means that two or more mRNA loci from SEQ ID NO:1 to SEQ ID NO:56 together with a reference mRNA are amplified by RT-PCR simultanously in one single reaction tube. In addition, one oligonucleotide primer per locus is covalently labeled at its 5'-end by a fuorophore, e.g. 6-Carboxyfluoresceine (6FAM), 6-Carboxy-4',5'-dichloro-2',7'-dimethoxy-fluoresceine (JOE), 4,7,2',4',5',7'-Hexachloro-6-carboxy-fluoresceine (HEX), 5- and 6-Carboxy-X-rhodamine (ROX), 6-Carboxytetramethyl-rhodamine (TAMRA), 6-Carboxy-4,7,2',7'-tetrachloro-fluoresceine (TET), 2'-Chloro-5'-fluoro-7',8'-benzo-1,4-dichloro-6-carboxyfluoresceine (NED). The amplificates of the multiplex RT-PCR are separated by capillary electrophoresis using DNA sequencing automates like the ABI Prism^{®} 310 Genetic Analyzer (Applied Biosystems, Foster City, us) and the locus specific signals are quantified (Scharf SJ, Smith AG, Hansen JA, McFarland C, Erlich HA. Quantitative determination of bone marrow transplant engraftment using fluorescent polymerase chain reaction primers for human identity markers. Blood. 1995, 85:1954-1963).

According to the present invention, it is preferred to use defined combinations of the described marker genes for diagnosing and/or characterizing the pathological conditions associated with male infertility. It was found that particular reliable results were obtained when using combinations of two or more genes from the following group of marker genes: SEQ ID NO:1, SEQ ID NO:2, SEQ ID NO:4, SEQ ID NO:6, SEQ ID NO:7, SEQ ID NO:10, SEQ ID NO:11, SEQ ID NO:15, SEQ ID NO:16, SEQ ID NO:17, SEQ ID NO:24, SEQ ID NO:36, SEQ ID NO:37, SEQ ID NO:38, SEQ ID NO:39, SEQ ID NO:40, SEQ ID NO:43 and SEQ ID NO:45. For example, the methods of the invention can be performed with combinations comprising SEQ ID NO:1 and SEQ ID NO:2; SEQ ID NO:1 and SEQ ID NO:4; SEQ ID NO:1 and SEQ ID NO:6; SEQ ID NO:1 and SEQ ID NO:7; SEQ ID NO:2 and SEQ ID NO:4; SEQ ID NO:2 and SEQ ID NO:6; SEQ ID NO:2 and SEQ ID NO:7; SEQ ID NO:4 and SEQ ID NO:6; SEQ ID NO:10 and SEQ ID NO:11; SEQ ID NO:15 and SEQ ID NO:24 or any other combination of two or more genes from this group. According to a particular embodiment, the methods of the invention include the determination of the expression levels of all of the above marker genes.

The invention further provides kits for performing the methods contemplated by the invention. According to one embodiment, a kit for diagnosing and/or characterizing a pathological condition associated with male infertility by microarray technique is provided which comprises a microarray as described herein. The kit may further comprise reagents and other means for microarray hybridization and detection. For example, the kit may comprise buffers and enzymes for first strand synthesis, cDNA purification and in vitro transcription and labeling. Further it may comprise buffers for hybridization, washing and cRNA fragmentation. It may also contain instructions for using the microarray.

According to a further embodiment, a kit for diagnosing and/or characterizing a pathological condition associated with male infertility by RT-PCR is provided. The RT-PCR may comprise primers for amplifying two or more genes selected from the group of genes shown in SEQ ID NO:1 to SEQ ID NO:46. It may also comprise buffers, polymerase enzymes for reverse transcription and amplification and probes, such as probes which are labeled with fluorescent groups suitable to be used in FRET-based techniques. Of course, the kit may also comprise instructions as to the use of the components in quantitative RT-PCR.

The present invention allows an assessment on the basis of a testis tissue sample whether or not and to which degree the fertility of a male individual is affected. The invention further provides for means and methods to determine in more detail the nature of the particular pathological condition which is associated with the observed infertility. Accordingly, the present invention provides valuable information to the practitioner. The invention also provides a basis for evaluating the prospect of success when performing testicular sperm extraction (TESE) methods. The above described pathological conditions to be diagnosed are characterized by, the distinct quantitative expression levels of the marker genes identified herein. The particular expression patterns predict the presence or absence in a biopsy of testicular spermatozoa suitable ICSI.

Thus, according to a further aspect, a method for predicting the prospect of success of a testicular sperm extraction procedure is provided, wherein the in vitro method of diagnosing and/or characterizing a pathological condition associated with male infertility discussed above is performed, and wherein the allocation of an individual to a pathological condition associated with male infertility is indicative for the prospect of success of the testicular sperm extraction procedure. This means, a patient which shows molcular expression patterns of the marker genes that correspond to a Johnsen score of 2 will most probably only posess few tubules with functional spermatids. Therefore, the chances of performing a successful TESE procedure are considerably low. In contrast, a molecular expression pattern which corresponds to a Johnsen score of 8 indicates that the chances for obtaining functional spermatids from the patient are high. These patients usually exhibit tubules which are associated with a sufficiently high number of functional elongated spermatids.

In a further preferred embodiment, the marker genes described herein are used in drug screening assays for identifying an active agent which may be used as a medicament for treating male infertility. In particular, the effect of drug candidates on the gene expression profile in the testis is determined. Such approach is based on the capability of the desired active agent to increase the expression level of several or all of the marker genes shown in SEQ ID NO:1 to SEQ ID NO:46.

Accordingly, a method of screening an active agent for treating a pathological condition associated with male infertility is provided, comprising:
(a) contacting a testis tissue from an individual affected with a pathological condition associated with male infertility with a drug candidate;
(b) providing mRNA from said testis tissue;
(c) determining the expression levels of two or more genes selected from the group of genes shown in SEQ ID NO:1 to SEQ ID NO:46 on the basis of said mRNA; and
(d) comparing the expression levels of the two or more genes determined in step (c) to the expression levels of the same genes in the absence of said drug candidate;
wherein increased expression levels of the two or more genes in the testis tissue that was contacted with said drug candidate indicate that the drug candidate is an active agent for treating a pathological condition associated with male infertility.

Preferably, the screening assays of the invention are performed as high throughput screening assays which allow the simultaneous evaluation of a high number of drug candidates (see, for example, Zlokamik, et al. (1998), Science 279, 84-88). Upon contact with a testis tissue from an individual suffering from a pathological condition associated with male infertility, for example a condition that is characterized by a Johnsen score of 10, 8, 5 or 2, suitable active agents should increase expression levels of the marker genes depicted in SEQ ID NO:1 to SEQ ID NO:46. The increase in expression will be dependent from the particular marker gene and the severity of the pathological condition of the individual from which the tissue is derived. Significant changes in the expression levels which indicate that a given drug candidate may be used as an active agent will include an increase in two or more of the marker genes used in the assay by at least 25%, normally by at least 50%, 75%, 100%, 200%, 500% and in some embodiments 750-1000%. The screening methods of the invention may be performed in vitro by use of a testis tissue sample.

As used herein, the term "candidate drug" refers to any molecule which is suspected to have the capability to enhance expression of the marker genes. Suitable candidate drugs usually comprise proteins, peptides, polysaccharides, polynucleotides, small organic or inorganic molecules and the like. Candidate drugs are often organic or inorganic molecules, preferably small organic compounds having a molecular weight of more than 100 and less than about 2500 daltons, more preferably less than 2000, 1000 or 500 daltons. Other typical candidate drugs are proteins, peptides or fragments thereof. In a preferred embodiment, the candidate drugs are peptides having a length of about 8 to about 40 amino acids, preferably about 15 to about 20 amino acids. The peptides may be, for example, digests of naturally occurring proteins or chemically synthesized random peptides.

Candidate drugs may be readily obtained from well-known screening libraries comprising synthetic or natural compounds. These libraries can be of natural or synthetic origin. For example, libraries of natural origin are available in the form of extracts from bacterial, fungal, viral, plant and animal cells. Alternatively, numerous methods are known for the synthesis of a wide variety of organic compounds and bio-molecules, for example expression of randomized oligonucleotides.

In the screening methods according to the invention, the candidate drugs are added to a testis tissue from an individual affected with a pathological condition associated with male infertility and allowed to incubate for a defined period of time, typically several minutes. Subsequently, mRNA is isolated from the testis tissue and the expression levels of two or more of the marker genes shown in SEQ ID NO:1 to SEQ ID NO:46 is determined on the basis of the mRNA, e.g. by microarray or RT-PCR. The expression levels determined in the tissue from the affected individual are then compared to the expression levels of the identical genes as obtained in the absence of said drug candidate. This can be achieved, for example, by using a testis tissue sample which is divided into two samples, one of which is subjected directly to determination of expression levels, whereas the other is first contacted with the drug candidate and then subjected to determination of expression levels. The detection of increased expression levels of the two or more genes in the testis tissue that was contacted with said drug candidate relative to the testis tissue that was not contacted with said drug candidate indicate that the drug candidate is an active agent for treating a pathological condition associated with male infertility.

Preferably, the pathological condition associated with male infertility is one that is characterized by a Johnsen score of 10, 8, 5 or 2. The method may comprise determining the expression levels of more than two genes, for example 5, 6, 7, 8, 9, 10, 20, 25, 30, 35, 40 or 45 genes selected from the group of the genes shown in SEQ ID NO:1 to SEQ ID NO:46. Preferably, the screening method according to the invention comprises determining the expression level of all genes from the group of the genes shown in SEQ ID NO:1 to SEQ ID NO:46. Even more preferably, the determination of the expression level of the respective genes is performed simultaneously, for example by use of an microarray or by amplification-based methods. For example, a microarray of the present invention as described elsewhere herein may be used for screening the active agent. Thus, the present invention also relates to the use of such microarray in screening an active agent for treating a pathological condition associated with male infertility.

### EXAMPLES

### Patients

The study population comprised patients with azoospermia due to either obstruction of the sperm transporting ducts or defects in the spermatogenic tissue who underwent an attempt for testicular sperm extraction (TESE) in the Department of Andrology, University Hospital Hamburg-Eppendorf, Germany, between June 2004 and December 2005. Informed consent and Ethic committee approval (OB/(X/2000) was obtained, and the studies conducted in accordance with the guidelines of the *Helsinki* Declaration regarding the use of human tissues. Morning baseline serum hormone concentrations were measured prior to testicular biopsy. No constitutive chromosomal abnormalities and two microdeletions of the Y chromosome had been documented for the patients.

### Testicular biopsy and TESE

Testis tissue was obtained at the same time for therapeutic TESE and diagnostic purposes. Tissue samples were taken according to a protocol previously described as the "sandwich principle" (Jezek and Schulze (1998), Hum Reprod 13, 1230-1234; Schulze (1998), In: Kempers, Cohen, Haney, Young (eds.), Elsevier Science, Amsterdam, pp. 621-626). Briefly, at least four fragments per testis were processed for histological analysis, post-surgical trial-TESE, and cryopreservation. An additional sample of rice grain size (approx. 30 mg) was submerged in 2 ml RNAlater® (Ambion, Austin, TX) for RNA extraction and subsequent gene expression profiling.

### Histology

Tissue samples were processed as described by Jezek and Schulze (1998), Hum Reprod 13, 1230-1234; Schulze (1998), In: Kempers, Cohen, Haney, Young (eds.), Elsevier Science, Amsterdam, pp. 621-626). Briefly, fragments were immersed in 5.5% glutaraldehyde at 4°C for 2h and post-fixed in 1% OsO₄ for another 2h. Specimens were dehydrated and embedded in Epon employing standard procedures. 1 µm semi-thin sections were obtained and stained with toluidine blue/pyronine for bright field microscopic evaluation. The following parameters were assessed: number of germ cell-containing tubules, score count of germ cell-containing tubules (Johnsen (1970), Hormones, 1, 2-25) and spermatozoa obtained at trial-TESE. Cases of maldescensus testis or hypospermatogenesis with so-called "mixed atrophy" where score count differences between individual tubules were obvious were excluded from subsequent microarray analysis.

### Hybridization, post-hybridization and scanning of arrays

RNAlater®-fixed tissue was homogenized in 3 ml RNApure^{™} (Peqlab, Germany) using an Ultra-Turrax^{™}, and total RNA was extracted according to the manufacturer's protocols. Extracts were post-purified on RNeasy^{™} columns (Qiagen, Germany). RNA integrity (28S/18S ratio) and purity was assessed on an Agilent Bioanalyzer (Model 2100; Agilent Technologies, Palo Alto, CA); only samples with an RNA integrity number higher than 7.5 (RIN, Agilent software) were employed in the microarray analysis. Amplification using the well-known T7-primer and labeling of cRNA was done according to the manufacturer's protocol (GE Healthcare, Piscataway, NJ) using time-optimized amplification parameters as previously described (Spiess et al., 2003). 10 µg of cRNA were hybridized to a Codelink^{™} Human 20K Bioarray (GE Healthcare) containing 19881 gene specific probes. Arrays were stringency washed, stained with Cy5TM-Streptavidin (GE Healthcare) and washed again according to the manufacturer's instructions. Slides were dried by centrifugation and immediately scanned on a 428TM Array Scanner (Affymetrix) using Jaguar 2.0 Software. Images were analyzed and quantified using the CodeLink^{™} Expression Analysis Software v4.1 (GE Healthcare) and rigorously quality controlled for hybridization artefacts.

### Quantitative real-time PCR (qRT-PCR)

qRT-PCR was performed using the LightCycler^{™} (Roche, Basel, Switzerland) technology. cDNAs were synthesized with Superscript^{™} II reverse transcriptase (Invitrogen, Carlsbad, CA) and PCR reactions were performed using 10 pmol of each gene specific primers, 2 µl dNTP mix (25 mM each, Takara Bio, Shiga, Japan), 0.5 µl SybrGreen I (1:1000 in DMSO; Molecular Probes, Leiden, Netherlands), 0.25 µl BSA (20 mg/ml; Sigma, Germany) and 0.2 µl Ex-Taq HS (5 U/µl; Takara Bio, Shiga, Japan) in a total volume of 20 µl. Cycling conditions were as follows: denaturation (95°C for 5 min), amplification and quantification (95°C for 10 s, primer-specific annealing temperature for 10 s, 72°C for 30 s with a single fluorescence measurement at the end of the segment) repeated 40 times, a melting curve program (60-95°C with a heating rate of 0.1° C/s and continuous fluorescence measurement) and a cooling step to 40°C. The threshold cycle (crossing point) was determined by a second derivate maximum method (LightCycler^{™} Quantification Software).

Expression levels were normalized to ribosomal protein S27a expression showing minimum variation between individual human testis samples of differing pathologies (Figure 7). Fold-differences were calculated by use of the REST© software (Pfaffl et al., 2002). The PCR efficiency was calculated to be in the range between 1.65 and 1.81 by an R script which uses a 'sliding window' method to estimate the slope of the amplification curve at the point of highest linearity (Ramakers et al., 2003). PCR products were electrophoretically separated on 1.3% agarose/TAE gels and verified by sequence analysis.

It was found from both the PCR analysis and the microarray data that expression of the genes provided in SEQ ID NO:1 to SEQ ID NO:46 are clearly correlated to the severity of the spermatogenic failure (see Figure 3). Low expression levels indicate a low Johnsen score.

## Claims

1. In vitro method of diagnosing and/or characterizing a pathological condition associated with male infertility in an individual, comprising
(a) providing mRNA from a testis tissue sample of said individual;
(b) determining the expression levels of two or more genes selected from the group of genes shown in SEQ ID NO:1 to SEQ ID NO: 46 on the basis of said mRNA; and
(c) comparing the expression levels of the two or more genes determined in step (b) to the expression levels of the same genes in a reference testis tissue sample;
wherein the comparison of the gene expression levels reveals the presence and/or the degree of a pathological condition associated with male infertility.

2. Method according to claim 1, wherein said reference sample is from a subject which is not affected from a pathological condition associated with male infertility.

3. Method according to claim 1, wherein said reference sample is from a subject which is affected from a pathological condition associated with male infertility.

4. Method according to any of claims 1 to 3, wherein step (b) comprises determining the expression level of five or more genes selected from the group of the genes shown in SEQ ID NO:1 to SEQ ID NO:46.

5. Method according to claim 4, wherein step (b) comprises determining the expression level of ten or more genes selected from the group of the genes shown in SEQ ID NO:1 to SEQ ID NO:46.

6. Method according to claim 5, wherein step (b) comprises determining the expression level of twenty or more genes selected from the group of the genes shown in SEQ ID NO:1 to SEQ ID NO:46.

7. Method according to claims 1 to 6, wherein step (b) comprises determining the expression level of all genes shown in SEQ ID NO:1 to SEQ ID NO:46.

8. Method according to claims 1 to 7, wherein the pathological condition associated with male infertility is a condition that is **characterized by** a Johnsen score of 10, 8, 5 or 2.

9. Method according to claims 1 to 8, wherein the expression levels of the two or more genes are determined by use of a microarray comprising two or more genes selected from the group of genes shown in SEQ ID NO:1 to SEQ ID NO:46.

10. Method according to claims 1 to 8, wherein the expression levels of the two or more genes are determined by use of RT-PCR analysis.

11. Method according to claim 10, wherein the RT-PCR analysis is a quantitative real time RT-PCR analysis.

12. Method for predicting the prospect of success of a testicular sperm extraction procedure, comprising performing the method according to any of claims 1 to 11, wherein the allocation of an individual to a pathological condition associated with male infertility is indicative for the prospect of success of the testicular sperm extraction procedure.

13. Microarray for diagnosing and/or characterizing a pathological condition associated with male infertility consisting of nucleic acid probes from not more than 1500 different genes and comprising nucleic acid probes derived from two or more of the nucleotide sequences provided in SEQ ID NO:1 to SEQ ID NO:46.

14. Microarray according to claim 13, comprising two or more of the nucleic acid probes provided in SEQ ID NO:47 to SEQ ID NO:92.

15. Microarray according to claims 13 or 14, further comprising nucleic acid probes suitable for the normalization of gene expression.

16. Microarray according to claims 13 to 15, consisting of nucleic acid probes from not more than 1000 different genes.

17. Microarray according to claim 16, consisting of nucleic acid probes from not more than 500 different genes.

18. Kit for diagnosing and/or characterizing a pathological condition associated with male infertility by microarray analysis comprising a microarray according to any of claims 13 to 17.

19. Kit for diagnosing and/or characterizing a pathological condition associated with male infertility by RT-PCR comprising primers for amplifying two or more genes selected from the group of genes shown in SEQ ID NO:1 to SEQ ID NO:46.

20. Method of screening an active agent for treating a pathological condition associated with male infertility comprising:
(a) contacting a testis tissue from an individual affected with a pathological condition associated with male infertility with a drug candidate;
(b) providing mRNA from said testis tissue;
(c) determining the expression levels of two or more genes selected from the group of genes shown in SEQ ID NO:1 to SEQ ID NO: 46 on the basis of said mRNA; and
(d) comparing the expression levels of the two or more genes determined in step (c) to the expression levels of the same genes in the absence of said drug candidate;
wherein increased expression levels of the two or more genes in the testis tissue that was contacted with said drug candidate indicate that the drug candidate is an active agent for treating a pathological condition associated with male infertility.

21. Method according to claim 20, wherein the pathological condition associated with male infertility is a condition that is **characterized by** a Johnsen score of 10, 8, 5 or 2.

22. Use of a microarray according to claims 13 to 17 for diagnosing and/or characterizing a pathological condition associated with male infertility.

23. Use of a microarray according to claims 13 to 17 for predicting the prospect of success of a testicular sperm extraction procedure.

24. Use of a microarray according to claims 13 to 17 for screening an active agent for treating a pathological condition associated with male infertility.
